# EUROPEAN PATENT SPECIFICATION

(11) **EP 1 787 982 B1**
(45) Date of publication and mention of the grant of the patent: **12.05.2010**
(21) Application number: 07102177.8
(22) Date of filing: 06.04.2004
(51) Int. Cl.: C07D 209/02, C07D 401/12, C07D 401/06, C07D 403/12, C07D 487/08, A61K 31/403, A61K 31/55, A61P 3/10

(54) **11Beta-Hydroxysteroid dehydrogenase type 1 active compounds**
Verbindungen mit Aktivität an der 11Beta-Hydroxasteroiddehydrogenase
Composés ayant une activité sur la 11beta-hydroxysteroid dehydrogenase

(30) Priority: 11.04.2003 DK 200300565; 27.06.2003 DK 200300972; 30.06.2003 DK 200300990; 30.06.2003 DK 200300989; 30.06.2003 DK 200300988; 02.07.2003 DK 200300998; 22.12.2003 DK 200301910; 06.01.2004 DK 200400009
(43) Date of publication of application: 23.05.2007
(62) Divisional of application: 04725888.4
(73) Proprietor: High Point Pharmaceuticals, LLC, High Point, NC 27265 (US)
(72) Inventor: Gundertofte, Anette, DK-2000, Frederiksberg (DK); Jørgensen, Anker Steen, 2100, Copenhagen Ø (DK); Kampen, Gita Camilla Tejlgaard, 2850, Nærum (DK); Andersen, Henrik Sune, 2800, Lyngby (DK); Christensen, Inge Thøger, DK-2800, Kgs. Lyngby (DK); Kilburn, John Paul, DK-4690, Haslev (DK)
(74) Representative: Russell, Lindsey

(56) References cited:
- WO-A-01/90090
- SOHAR, P. ET AL: "Conformational analysis of N-acylazabicyclooctanes" MAGNETIC RESONANCE IN CHEMISTRY , 23(7), 506-13 CODEN: MRCHEG; ISSN: 0749-1581, 1985, XP009033435
- MARIANI, E. ET AL: "Derivatives of 1,3,3-trimethyl-6-azabicyclo[3.2.1]octane with hypotensive activity. I" FARMACO, EDIZIONE SCIENTIFICA , 38(9), 653-63 CODEN: FRPSAX; ISSN: 0430-0920, 1983, XP001194477

## Description

### FIELD OF INVENTION

The present invention relates to novel substituted bi- or tricyclic amides, to their use in therapy, to pharmaceutical compositions comprising the compounds, and to the use of said compounds in the manufacture of medicaments.

The present compounds modulate the activity of 11β-hydroxysteroid dehydrogenase type 1 (11βHSD1) and are accordingly useful in the treatment of diseases in which such a modulation is beneficial, such as the metabolic syndrome.

### BACKGROUND OF THE INVENTION

The metabolic syndrome is a major global health problem. In the US, the prevalence in the adult population is currently estimated to be approximately 25%, and it continues to increase both in the US and worldwide. The metabolic syndrome is characterized by a combination of insulin resistance, dyslipidemia, obesity and hypertension leading to increased morbidity and mortality of cardiovascular diseases. People with the metabolic syndrome are at increased risk of developing frank type 2 diabetes, the prevalence of which is equally escalating.

In type 2 diabetes, obesity and dyslipidemia are also highly prevalent and around 70% of people with type 2 diabetes additionally have hypertension once again leading to increased mortality of cardiovascular diseases.

In the clinical setting, it has long been known that glucocorticoids are able to induce all of the cardinal features of the metabolic syndrome and type 2 diabetes.

11β-hydroxysteroid dehydrogenase type 1 (11βHSD1) catalyses the local generation of active glucocorticoid in several tissues and organs including predominantly the liver and adipose tissue, but also e.g. skeletal muscle, bone, pancreas, endothelium, ocular tissue and certain parts of the central nervous system. Thus, 11βHSD1 serves as a local regulator of glucocorticoid actions in the tissues and organs where it is expressed (Tannin et al., J. Biol. Chem., 266, 16653 (1991); Bujalska et al., Endocrinology, 140, 3188 (1999); Whorwood et al., J. Clin. Endocrinol. Metab., 86, 2296 (2001); Cooper et al., Bone, 27, 375 (2000); Davani et al., J. Biol. Chem., 275, 34841 (2000); Brem et al., Hypertension, 31, 459 (1998); Rauz et al., Invest. Ophthalmol. Vis. Sci., 42, 2037 (2001); Moisan et al., Endocrinology, 127, 1450 (1990)).

The role of 11βHSD1 in the metabolic syndrome and type 2 diabetes is supported by several lines of evidence. In humans, treatment with the non-specific 11βHSD1 inhibitor carbenoxolone improves insulin sensitivity in lean healthy volunteers and people with type 2 diabetes. Likewise, 11βHSD1 knock-out mice are resistant to insulin resistance induced by obesity and stress. Additionally, the knock-out mice present with an anti-atherogenic lipid profile of decreased VLDL triglycerides and increased HDL-cholesterol. Conversely, mice that overexpress 11βHSD1 in adipocytes develop insulin resistance, hyperlipidemia and visceral obesity, a phenotype that resembles the human metabolic syndrome (Andrews et al., J. Clin. Endocrinol. Metab., 88, 285 (2003); Walker et al., J. Clin. Endocrinol. Metab., 80, 3155 (1995); Morton et al., J. Biol. Chem. 276, 41293 (2001); Kotelevtsev et al., Proc. Natl. Acad. Sci. USA, 94, 14924 (1997); Masuzaki et al., Science, 294, 2166 (2001)).

The more mechanistic aspects of 11βHSD1 modulation and thereby modulation of intracellular levels of active glucocorticoid have been investigated in several rodent models and different cellular systems. 11βHSD1 promotes the features of the metabolic syndrome by increasing hepatic expression of the rate-limiting enzymes in gluconeogenesis, namely phosphoenolpyuvate carboxykinase and glucose-6-phosphatase, promoting the differentiation of preadipocytes into adipocytes thus facilitating obesity, directly and indirectly stimulating hepatic VLDL secretion, decreasing hepatic LDL uptake and increasing vessel contractility (Kotelevtsev et al., Proc. Natl. Acad. Sci. USA, 94, 14924 (1997); Morton et al., J. Biol. Chem. 276, 41293 (2001); Bujalska et al., Endocrinology, 140, 3188 (1999); Souness et al., Steroids, 67, 195 (2002); Brindley & Salter, Prog. Lipid Res., 30, 349 (1991)).

WO 01/90090, WO 01/90091, WO 01/90092, WO 01/90093 and WO 01/90094 discloses various thiazol-sulfonamides as inhibitors of the human 11β-hydroxysteroid dehydrogenase type 1 enzyme, and further states that said compounds may be useful in treating diabetes, obesity, glaucoma, osteoporosis, cognitive disorders, immune disorders and depression.

We have now found novel substituted bi- or tricyclic amides that modulate the activity of 11βHSD1 leading to altered intracellular concentrations of active glucocorticoid. More specifically, the present compounds inhibit the activity of 11βHSD1 leading to decreased intracellular concentrations of active glucocorticoid. Thus, the present compounds can be used to treat disorders where a decreased level of active intracellular glucocorticoid is desirable, such as e.g. the metabolic syndrome, type 2 diabetes, impaired glucose tolerance (IGT), impaired fasting glucose (IFG), dyslipidemia, obesity, hypertension, diabetic late complications, cardiovascular diseases, arteriosclerosis, atherosclerosis, myopathy, muscle wasting, osteoporosis, neurodegenerative and psychiatric disorders, and adverse effects of treatment or therapy with glucocorticoid receptor agonists.

Objects of the present invention are to provide compounds, pharmaceutical compositions and use of said compounds that modulate the activity of 11βHSD1.

### DEFINITIONS

In the following structural formulas and throughout the present specification, the following terms have the indicated meaning:

The term "halo" includes fluorine, chlorine, bromine, and iodine.

The term "trihalomethyl" includes trifluoromethyl, trichloromethyl, tribromomethyl, and triiodomethy).

The term "trihalomethoxy" includes trifluorometoxy, trichlorometoxy, tribromometoxy, and triiodometoxy.

The term "alkyl" includes C₁-C₈, preferred C₁-C₆ straight chain saturated and methylene aliphatic hydrocarbon groups, C₃-C₈ branched saturated hydrocarbon groups having the specified number of carbon atoms. For example, this definition shall include but is not limited to methyl (Me), ethyl (Et), propyl (Pr), butyl (Bu), pentyl, hexyl, isopropyl (i-Pr), isobutyl (i-Bu), *tert-*butyl (*t*-Bu), sec-butyl (s-Bu), isopentyl, neopentyl, and the like.

The term "alkenyl" includes C₂-C₆ straight chain unsaturated aliphatic hydrocarbon groups and branched C₃-C₆ unsaturated aliphatic hydrocarbon groups having the specified number of carbon atoms. For example, this definition shall include but is not limited to ethenyl, propenyl, butenyl, pentenyl, hexenyl, methylpropenyl, methylbutenyl and the like.

The term "alkynyl" includes C₂-C₆ straight chain unsaturated aliphatic hydrocarbon groups and C₄-C₆ branched unsaturated aliphatic hydrocarbon groups having the specified number of carbon atoms. For example, this definition shall include but is not limited to ethynyl, propynyl, butynyl, pentynyl, hexynyl, methylbutynyl, and the like.

The term "saturated or partially saturated cyclic, bicyclic or tricyclic ring system" represents but are not limited to azepanyl, azocanyl, 1,2,3,4-tetrahydro-quinolinyl, 1,2,3,4-tetrahydro-isoquinolinyl, 1,2,3,4-tetrahydro-quinoxalinyl, indolinyl, 6-aza-bicyclo[3.2.1]octane, 2-aza-bicyclo[4.1.1]octane, 2-aza-bicyclo[3.2.1]octanyl, 7-aza-bicyclo[4.1.1]octanyl, 9-aza-bicyclo[3.3.2]decanyl, 4-aza-tricyclo[4.3 .1.1^{3,8}]undecanyl, 9-aza-tricyclo[3.3.2.0^{3,7}]decanyl, 8-aza-spiro[4.5]decane.

The term "cycloalkyl" (e.g. cyclopropyl, cyclobutyl, cyclopentyl, cyclohexyl, cycloheptyl, cyclooctyl, cyclononyl, cyclodecyl, bicyclo[3.2.1]octyl, spiro[4.5]decyl, norpinyl, norbonyl, norcaryl, adamantyl and the like) represents a saturated, mono-, bi-, tri- or spirocarbocyclic group having the specified number of carbon atoms.

The term "cycloalkylalkyl" (e.g. cyclopropylmethyl, cyclobutylethyl, adamantylmethyl and the like) represents a cycloalkyl group as defined above attached through an alkyl group having the indicated number of carbon atoms or substituted alkyl group as defined above.

The term "cycloalkenyl" (e.g. cyclobutenyl, cyclopentenyl, cyclohexenyl, cycloheptenyl, cyclooctenyl, cyclononenyl, cyclodecenyl and the like) represents a partially saturated, mono-, bi-, tri- or spirocarbocyclic group having the specified number of carbon atoms.

The term "cycloalkylcarbonyl" (e.g. cyclopropylcarbonyl, cyclohexylcarbonyl) represents an cycloalkyl group as defined above having the indicated number of carbon atoms attached through a carbonyl group.

The term "hetcycloalkylcarbonyl" (e.g. 1-piperidin-4-yl-carbonyl, 1-(1,2,3,4-tetrahydro-isoquinolin-6-yl)carbonyl) represents an hetcycloalkyl group as defined above having the indicated number of carbon atoms attached through a carbonyl group.

The term "hetcycloalkyl" (e.g. tetrahydrofuranyl, tetrahydropyranyl, tertahydrothiopyranyl, piperidine, pyridazine and the like) represents a saturated mono-, bi-, tri- or spirocarbocyclic group having the specified number of carbon atoms and one or two additional heteroatoms or groups selected from nitrogen, oxygen, sulphur, SO or SO₂.

The term "hetcycloalkylalkyl" (e.g. tetrahydrofuranylmethyl, tetrahydropyranylethyl, tertahydrothiopyranylmethyl, and the like) represents a hetcycloalkyl group as defined above attached through an alkyl group having the indicated number of carbon atoms or substituted alkyl group as defined above.

The term "alkyloxy" (e.g. methoxy, ethoxy, propyloxy, allyloxy, cyclohexyloxy) represents an alkyl group as defined above having the indicated number of carbon atoms attached through an oxygen bridge.

The term "alkyloxyalkyl" (e.g. methyloxymethyl and the like) represents an alkyloxy group as defined above attached through an "alkyl" group.

The term "aryloxy" (e.g. phenoxy, naphthyloxy and the like) represents an aryl group as defined below attached through an oxygen bridge.

The term "hetaryloxy" (e.g. 2-pyridyloxy and the like) represents a hetaryl group as defined below attached through an oxygen bridge.

The term "aryloxyalkyl" (e.g. phenoxymethyl, naphthyloxyethyl and the like) represents an aryloxy group as defined above attached through an "alkyl" group having the indicated number of carbon atoms.

The term "arylalkyloxy" (e.g. phenethyloxy, naphthylmethyloxy and the like) represents an arylalkyl group as defined below attached through an oxygen bridge.

The term "hetarylalkyloxy" (e.g. 2-pyridylmethyloxy and the like) represents a hetarylalkyl group as defined below attached through an oxygen bridge.

The term "hetaryloxyalkyl" (e.g. 2-pyridyloxymethyl, 2-quinolyloxyethyl and the like) represents a hetaryloxy group as defined above attached through an "alkyl" group having the indicated number of carbon atoms.

The term "hetarylalkyloxyalkyl" (e.g. 4-methoxymethyl-pyrimidine, 2-methoxymethylquinoline and the like) represents a hetarylalkyloxy group as defined above attached through an "alkyl" group having the indicated number of carbon atoms.

The term "arylalkyloxyalkyl" (e.g. ethoxymethyl-benzene, 2-methoxymethylnaphthalene and the like) represents an arylalkyloxy group as defined above attached through an "alkyl" group having the indicated number of carbon atoms.

The term "alkylthio" (e.g. methylthio, ethylthio and the like) represents an alkyl group as defined above attached through a sulphur bridge.

The term "alkyloxycarbonyl" (e.g. methylformiat, ethylformiat and the like) represents an alkyloxy group as defined above attached through a carbonyl group.

The term "aryloxycarbonyl" (e.g. phenylformiat, 2-thiazolylformiat and the like) represents an aryloxy group as defined above attached through a carbonyl group.

The term "arylalkyloxycarbonyl" (e.g. benzylformiat, phenyletylformiat and the like) represents an "arylalkyloxy" group as defined above attached through a carbonyl group.

The term "arylalkyl" (e.g. benzyl, phenylethyl, 3-phenylpropyl, 1-naphtylmethyl, 2-(1-naphtyl)ethyl and the like) represents an aryl group as defined below attached through an alkyl having the indicated number of carbon atoms or substituted alkyl group as defined above.

The term "hetarylalkyl" (e.g. (2-furyl)methyl, (3-furyl)methyl, (2-thienyl)methyl, (3-thienyl)methyl, (2-pyridyl)methyl, 1-methyl-1-(2-pyrimidyl)ethyl and the like) represents a hetaryl group as defined below attached through an alkyl having the indicated number of carbon atoms or substituted alkyl group as defined above.

The term "alkylcarbonyl" (e.g. octylcarbonyl, pentylcarbonyl, 3-hexenylcarbonyl) represents an alkyl group as defined above having the indicated number of carbon atoms attached through a carbonyl group.

The term "arylcarbonyl" (e.g. benzoyl) represents an aryl group as defined below attached through a carbonyl group.

The term "hetarylcarbonyl" (e.g. 2-thiophenylcarbonyl, 3-methoxy-anthrylcarbonyl, oxazolylcarbonyl and the like) represents a hetaryl group as defined below attached through a carbonyl group.

The term "alkylcarbonylalkyl" (e.g. propan-2-one, 4,4-dimethyl-pentan-2-one and the like) represents an alkylcarbonyl group as defined above attached through an alkyl group as defined above having the indicated number of carbon atoms.

The term "hetarylcarbonylalkyl" (e.g. 1-pyridin-2-yl-propan-1-one, 1-(1-*H*-imidazol-2-yl)-propan-1-one and the like) represents a hetarylcarbonyl group as defined above attached through an alkyl group as defined above having the indicated number of carbon atoms.

The term "arylalkylcarbonyl" (e.g. phenylpropylcarbonyl, phenylethylcarbonyl and the like) represents an arylalkyl group as defined above having the indicated number of carbon atoms attached through a carbonyl group.

The term "hetarylalkylcarbonyl" (e.g. imidazolylpentylcarbonyl and the like) represents a hetarylalkyl group as defined above wherein the alkyl group is in turn attached through a carbonyl.

The term "alkylcarboxy" (e.g. heptylcarboxy, cyclopropylcarboxy, 3-pentenylcarboxy) represents an alkylcarbonyl group as defined above wherein the carbonyl is in turn attached through an oxygen bridge.

The term "arylcarboxy" (e.g. benzoic acid and the like) represents an arylcarbonyl group as defined above wherein the carbonyl is in turn attached through an oxygen bridge.

The term "alkylcarboxyalkyl" (e.g. heptylcarboxymethyl, propylcarboxy *tert*-butyl, 3-pentylcarboxyethyl) represents an alkylcarboxy group as defined above wherein the carboxy group is in turn attached through an alkyl group as defined above having the indicated number of carbon atoms.

The term "arylalkylcarboxy" (e.g. benzylcarboxy, phenylpropylcarboxy and the like) represents an arylalkylcarbonyl group as defined above wherein the carbonyl is in turn attached through an oxygen bridge.

The term "hetarylalkylcarboxy" (e.g. (1-*H*-imidazol-2-yl)-acetic acid, 3-pyrimidin-2-yl-propionic acid and the like) represents a hetarylalkylcarbonyl group as defined above wherein the carbonyl is in turn attached through an oxygen bridge.

The term "alkylS(O)ₙ" (e.g. ethylsulfonyl, ethylsulfinyl and the like) represents an alkyl group as defined above, wherein the alkyl group is in turn attached through a sulphur bridge wherein the sulphur is substituted with n oxygen atoms.

The term "arylS(O)ₙ" (e.g. phenylsulfinyl, naphthyl-2-sulfonyl and the like) represents an aryl group as defined above, wherein the aryl group is in turn attached through a sulphur bridge wherein the sulphur is substituted with n oxygen atoms.

The term "arylalkylS(O)ₙ" (e.g. benzylsulfinyl, phenetyl-2-sulfonyl and the like) represents an arylalkyl group as defined above, wherein the arylalkyl group is in turn attached through a sulphur bridge wherein the sulphur is substituted with n oxygen atoms.

The term "aryl" is a carbocyclic aromatic ring system being either monocyclic, bicyclic, or polycyclic, such as phenyl, biphenyl, naphthyl, anthracenyl, phenanthrenyl, fluorenyl, indenyl, pentalenyl, azulenyl, biphenylenyl and the like. Aryl is also intended to include the partially hydrogenated derivatives of the carbocyclic aromatic systems enumerated above. Non-limiting examples of such partially hydrogenated derivatives are 1,2,3,4-tetrahydronaphthyl, 1,4-dihydronaphthyl and the like.

The term "hetaryl" includes but is not limited to pyrrolyl (2-pyrrolyl), pyrazolyl (3-pyrazolyl), imidazolyl (1-imidazolyl, 2-imidazolyl, 4-imidazolyl, 5-imidazolyl), triazolyl (1,2,3-triazol-1-yl, 1,2,3-triazol-2-yl 1,2,3-triazol-4-yl, 1,2,4-triazol-3-yl), oxazolyl (2-oxazolyl, 4-oxazolyl, 5-oxazolyl), isoxazolyl (3-isoxazolyl, 4-isoxazolyl, 5-isoxazolyl), thiazolyl (2-thiazolyl, 4-thiazolyl, 5-thiazolyl), thiophenyl (2-thiophenyl, 3-thiophenyl, 4-thiophenyl, 5-thiophenyl), furanyl (2-furanyl, 3-furanyl, 4-furanyl, 5-furanyl), pyridyl (2-pyridyl, 3-pyridyl, 4-pyridyl, 5-pyridyl), 5-tetrazolyl, pyrimidinyl (2-pyrimidinyl, 4-pyrimidinyl, 5-pyrimidinyl, 6-pyrimidinyl), pyrazinyl, pyridazinyl (3-pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydro-benzo[b]furanyl), 3-(2,3-dihydro-benzo[b]furanyl), 4-(2,3-dihydro-benzo[b]furanyl), 5-(2,3-dihydrobenzo[b]furanyl), 6-(2,3-dihydro-benzo[b]furanyl), 7-(2,3-dihydro-benzo[b]furanyl)), benzo[b]thiophenyl (2-benzo[b]thiophenyl, 3-benzo[b]thiophenyl, 4-benzo[b]thiophenyl, 5-benzo[b]thiophenyl, 6-benzo[b]thiophenyl, 7-benzo[b]thiophenyl), 2,3-dihydro-benzo[b]thiophenyl (2-(2,3-dihydro-benzo[b]thiophenyl), 3-(2,3-dihydro-benzo[b]thiophenyl), 4-(2,3-dihydro-benzo[b]thiophenyl), 5-(2,3-dihydro-benzo[b]thiophenyl), 6-(2,3-dihydro-benzo[b]thiophenyl), 7-(2,3-dihydro-benzo[b]thiophenyl)), 4,5,6,7-tetrahydro-benzo[b]thiophenyl (2-(4,5,6,7-tetrahydro-benzo[b]thiophenyl), 3-(4,5,6,7-tetrahydro-benzo[b]thiophenyl), 4-(4,5,6,7-tetrahydrobenzo[b]thiophenyl), 5-(4,5,6,7-tetrahydro-benzo[b]thiophenyl), 6-(4,5,6,7-tetrahydro-benzo[b]thiophenyl), 7-(4,5,6,7-tetrahydro-benzo[b]thiophenyl)), thieno[2,3-b]thiophenyl, 4,5,6,7-tetrahydro-thieno[2,3-c]pyridyl (4-(4,5,6,7-tetrahydro-thieno[2,3-c]pyridyl), 5-4,5,6,7-tetrahydro-thieno[2,3-c]pyridyl), 6-(4,5,6,7-tetrahydro-thieno[2,3-c]pyridyl), 7-(4,5,6,7-tetrahydrothieno[2,3-c]pyridyl)), indolyl (1-indolyl, 2-indolyl, 3-indolyl, 4-indolyl, 5-indolyl, 6-indolyl, 7-indolyl), isoindolyl (1-isoindolyl, 2-isoindolyl, 3-isoindolyl, 4-isoindolyl, 5-isoindolyl, 6-isoindolyl, 7-isoindolyl), 1,3-dihydro-isoindolyl (1-(1,3-dihydro-isoindolyl), 2-(1,3-dihydro-isoindolyl), 3-(1,3-dihydro-isoindolyl), 4-(1,3-dihydro-isoindolyl), 5-(1,3-dihydro-isoindolyl), 6-(1,3-dihydro-isoindolyl), 7-(1,3-dihydro-isoindolyl)), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benz-oxazolyl, 2-benzoxazolyl), benzothiazolyl (1-benzothiazolyl, 2-benzothiazolyl, 4-benzothiazolyl, 5-benzothiazolyl, 6-benzothiazolyl, 7-benzothiazolyl), benzo-[1,2,5]oxadiazolyl, (4-benzo[1,2,5]oxadiazole, 5-benzo[1,2,5]oxadiazole),carbazolyl (1-carbazolyl, 2-carbazolyl, 3-carbazolyl, 4-carbazolyl).

The term "R⁵oxy" (e.g. MeC(O)O-, phenylC(O)O-,pyridine-2-yl-C(O)O- and the like) represents an R⁵ group as defined above attached through an oxygen bridge.

The term "R¹⁴alkylcarbonyl" (e.g. 2-cyclohexyloxy-acetyl, 3-(1-methyl-piperidin-4-yloxy)-propionyl, 2-phenoxy-acetyl and the like) represents an R¹⁴ group as defined above attached through an alkylcarbonyl group as defined above.

The term "R¹⁶carbonyl" (e.g. acetyl, 3-phenyl-propionyl, phenyl-acetyl, 2-(pyridin-2-ylmethoxy)-acetyl and the like) represents an R¹⁶ group as defined above attached through a carbonyl group.

The term "R¹⁶carbonylN(R¹²)" (e.g. 3-phenyl-propionamide, phenyl-acetamide, 2-(pyridin-2-ylmethoxy)-acetamide, N-methyl-2-(pyridin-2-ylmethoxy)-acetamide, benzyl-2-(pyridin-3-ylmethoxy)-acetamide and the like) represents an R¹⁶carbonyl group as defined above attached through an amino group substituted with R¹² as defined above.

The term "NR¹²R¹³carbonylalkyl" (e.g. N,N-dimethyl-propionamide, N-isopropyl-N-methyl-propionamide and the like) represents an NR¹²R¹³ group attached through a carbonylalkyl group as defined above.

The term "NR¹²R¹³alkylcarbonyl" (e.g. N,N-dimethylamino-acetyl, (*N*-cyclohexyl-*N-*methyl-amino)-acetyl, 2-(4-acetyl-piperazin-1-yl)-acetyl and the like) represents an NR¹²R¹³ group attached through an alkylcarbonyl group as defined above.

Certain of the above defined terms may occur more than once in the structural formulae, and upon such occurrence each term shall be defined independently of the other.

The term "optionally substituted" as used herein means that the groups in question are either unsubstituted or substituted with one or more of the substituents specified. When the groups in question are substituted with more than one substituent the substituents may be the same or different.

The term "treatment" is defined as the management and care of a patient for the purpose of combating or alleviating the disease, condition or disorder, and the term includes the administration of the active compound to prevent the onset of the symptoms or complications, or alleviating the symptoms or complications, or eliminating the disease, condition, or disorder.

The term "pharmaceutically acceptable" is defined as being suitable for administration to humans without adverse events.

The term "prodrug" is defined as a chemically modified form of the active drug, said prodrug being administered to the patient and subsequently being converted to the active drug. Techniques for development of prodrugs are well known in the art.

### DETAILED DESCRIPTION OF THE INVENTION

The present invention is based on the observation that the compounds of the general formulas (I) disclosed below are able to modulate or inhibit the activity of 11βHSD1.

Accordingly, the present invention is concerned with compounds of the general formula (I) 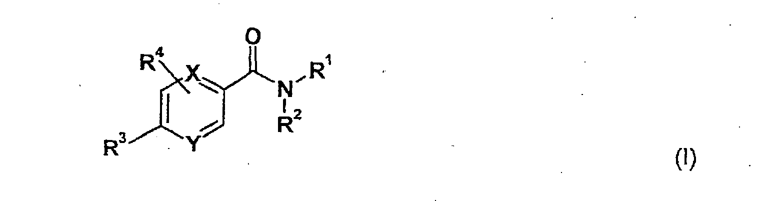 wherein
R¹ and R² together with the nitrogen to which they are attached is 6-aza-bicyclo[3.2.1]octane optionally substituted with at least one of C₁-C₆alkyl;
R³ is C₁-C₆alkyl, -NR⁸R⁹, -C(=O)NR⁸R⁹ or -OR¹⁰, wherein the alkyl group is optionally substituted with one or more of R¹¹;
R⁴ is hydrogen, halo, hydroxy, cyano, trihalomethyl or C₁-C₆alkyl;
R⁵ is C₁-C₆alkylcarbonyl-, C₃-C₁₀cycloalkylcarbonyl-, C₃-C₁₀cycloalkylC₁-C₆alkylcarbonyl-, arylcarbonyl-, arylC₁-C₆alkylcarbonyl-, hetarylcarbonyl- or hetarylC₁-C₆alkylcarbonyl-;
R⁶ is C₁-C₆alkyloxy-, aryloxy-, arylC₁-C₆alkyloxy-, hetaryloxy- or hetarylC₁-C₆alkyloxy-;
R⁷ is hydrogen, C₁-C₈alkyl, C₁-C₆alkyloxy or arylC₁-C₆alkyl;
R⁸ is hydrogen, C₁-C₆alkyl, C₃-C₁₀cycloalkyl, C₃-C₁₀cycloalkylC₁-C₆alkyl, C₁-C₆alkyloxyC₁-C₆alkyl, arylC₁-C₆alkyloxyC₁-C₆alkyl or C₂-C₆alkenyl;
R⁹ is C₁-C₆alkyl, C₃-C₁₀cycloalkyl, C₃-C₁₀cycloalkylC₁-C₆alkyl, C₃-C₁₀cycloalkylcarbonyl-, C₃-C₁₀hetcycloalkylcarbonyl-, arylcarbonyl-, hetarylcarbonyl-, C₁-C₆alkyloxyC₁-C₆alkyl, NR¹²R¹³carbonylC₁-C₆alkyl-, R¹⁴C₁-C₆alkylcarbonyl-, -COR¹⁵, C₁-C₆alkylS(O)ₙ-, arylS(O)ₙ-, arylC₁-C₆alkylS(O)ₙ-, arylC₁-C₆alkyl or hetarylC₁-C₆alkyl, wherein the alkyl, cycloalky, aryl and hetaryl groups independently are optionally substituted with one or more R¹¹;
R¹⁰ is C₁-C₆alkyl, arylC₁-C₆alkyl or NR¹²R¹³carbonylC₁-C₆alkyl, wherein the alkyl and aryl groups independently are optionally substituted with one or more R¹¹;
R¹¹ is R⁵, R⁶, halo, hydroxy, oxo, cyano, -COR¹⁵, C₁-C₈alkyl, C₁-C₈alkyloxy, C₃-C₁₀cycloalkyl, trihalomethyl, trihalomethyloxy, aryl, arylC₁-C₆alkyl, C₁-C₆alkyloxyC₁-C₆alkyl, aryloxyC₁-C₆alkyl, arylC₁-C₆alkyloxyC₁-C₆alkyl, hetaryl, hetarylC₁-C₆alkyl, hetaryloxyC₁-C₆alkyl, hetarylC₁-C₆alkyloxyC₁-C₆alkyl, -NR¹²R¹³, -SO₂NR¹²R¹³, NR¹²R¹³carbonylC₁-C₆alkyl, R¹⁶carbonylN(R¹²)-, arylS(O)ₙ-, hetarylS(O)ₙ- or R¹⁷S(O)ₙN(R¹²)-; wherein the aryl and hetaryl groups independently are optionally substituted with one or more R¹⁸;
R¹² and R¹³ independently are hydrogen, C₁-C₈alkyl, C₃-C₁₀cycloalkyl, C₃-C₁₀cycloalkylC₁-C₆-alkyl, C₃-C₁₀hetcycloalkyl, aryl, hetaryl, arylC₁-C₆alkyl or hetarylC₁-C₆alkyl wherein the alkyl, aryl and hetaryl groups independently are optionally substituted with one or more of R¹⁸; or
R¹² and R¹³ together with the nitrogen to which they are attached, are forming a saturated or partially saturated cyclic, bicyclic or tricyclic ring system containing from 4 to 12 carbon atoms and from 0 to 2 additional heteroatoms selected from nitrogen or oxygen, the ring system optionally being substituted with at least one R⁵, R⁵oxy-, R⁶, halo, cyano, hydroxy, oxo, C₁-C₈alkyl, aryl, hetaryl, arylC₁-C₆alkyl, hetarylC₁-C₆alkyl, C₁-C₆alkyloxyC₁-C₆alkyl, NR¹²R¹³carbonylC₁-C₆alkyl, NR¹²R¹³C₁-C₆alkylcarbonyl-, R¹⁴C₁-C₆alkylcarbonyl- or -COR¹⁵
R¹⁴ is C₁-C₆alkyloxy, C₃-C₁₀cycloalkyloxy-, C₃-C₁₀cycloalkylC₁-C₆alkyoxy-, C₃-C₁₀hetcycloalkyloxy-, aryl, hetaryl, arylC₁-C₆alkyloxy, hetarylC₁-C₆alkyloxy, -NR¹²R¹³, -COR¹⁵, wherein the alkyl, cycloalkyl, hetcycloalkyl, aryl and hetaryl groups independently are optionally substituted with one or more of R²⁰;
R¹⁵ is C₁-C₆alkyl, hydroxy, C₁-C₈alkyloxy, -NR¹²R¹³, aryl, aryloxy or arylC₁-C₆alkyloxy;
R¹⁶ is R⁶, C₁-C₆alkyl, C₂-C₆alkenyl, aryl, arylC₁-C₆alkyl, hetaryl, hetarylC₁-C₆alkyl, C₃-C₁₀-cycloalkyl, C₃-C₁₀hetcycloalkyl, arylC₁-C₆alkyloxyC₁-C₆alkyl-, hetarylC₁-C₆alkyloxyC₁-C₆alkyl- or R¹²R¹³NC₁-C₆alkyl- wherein the alkyl, alkenyl, cycloalkyl, hetcycloalkyl, aryl and hetaryl groups are optionally substituted with R¹⁹;
R¹⁷ is C₁-C₆alkyl, C₃-C₁₀cycloalkyl, C₃-C₁₀hetcycloalkyl, aryl, arylC₁-C₆alkyl, hetaryl, hetarylC₁-C₆alkyl;
R¹⁸ is R⁶, -NR¹²R¹³, oxo, C₁-C₆alkyl, C₃-C₁₀cycloalkyl or C₃-C₁₀hetcycloalkyl, wherein the alkyl, cycloalkyl, hetcycloalkyl, aryl and hetaryl groups independently are optionally substituted with one or more of R⁷;
R¹⁹ is hydrogen, halo, hydroxy, oxo, nitro, cyano or -COR¹⁵;
R²⁰ is hydrogen, C₁-C₈alkyl, -NR¹²R¹³, C₁-C₆alkyloxy or arylC₁-C₆alkyl;
X and Y independently are -CH- or nitrogen;
n is 1 or 2; or
a salt thereof with a pharmaceutically acceptable acid or base, or any optical isomer or mixture of optical isomers, including a racemic mixture, or any tautomeric forms,
with the proviso that the compound is not

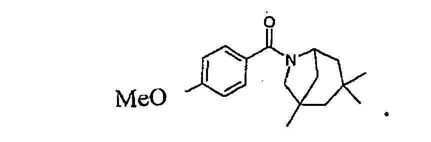

In one embodiment of the present invention, in formula (I) X is -CH-.

In another embodiment of the present invention, in formula (I) Y is -CH-.

In another embodiment of the present invention, in formula (I) X is nitrogen.

In another embodiment of the present invention, in formula (I) Y is nitrogen.

In another embodiment of the present invention, in formula (I) X and Y are -CH-.

In another embodiment of the present invention, in formula (I) R³ is -NR⁸R⁹, wherein R⁸ and R⁹ are defined as above.

In another embodiment of the present invention, in formula (I) R³ is -C(=O)NR⁸R⁹ wherein R⁸ and R⁹ are defined as above.

In another embodiment of the present invention, in formula (I) R³ is -OR¹⁰, wherein R¹⁰ is defined as above.

In another embodiment of the present invention, in formula (I) R¹ and R² together with the nitrogen to which they are attached is 1,3,3-trimethyl-6-aza-bicyclo[3.2.1]octane.

In another embodiment of the present invention, in formula (I) R⁴ is hydrogen or halo.

In another embodiment of the present invention, in formula (I) R⁴ is hydrogen.

In another embodiment of the present invention, in formula (I) R⁵ is C₃-C₁₀cycloalkylcarbonyl- or C₃-C₁₀cycloalkylC₁-C₆alkylcarbonyl-.

In another embodiment of the present invention, in formula (I) R₆ is C₁-C₆alkyloxy-.

In another embodiment of the present invention, in formula (I) R⁸ is hydrogen, C₁-C₆-alkyl, C₃-C₁₀cycloalkyl, C₃-C₁₀ycloalkylC₁-C₆alkyl.

In another embodiment of the present invention, in formula (I) R⁸ is hydrogen.

In another embodiment of the present invention, in formula (I) R⁸ is C₁-C₆alkyl.

In another embodiment of the present invention, in formula (I) R⁸ is C₃-C₁₀cycloalkyl.

In another embodiment of the present invention, in formula (I) R⁸ is C₃-C₁₀cycloalkylC₁-C₆alkyl.

In another embodiment of the present invention, in formula (I) R⁹ is C₁-C₆alkyl, C₃-C₁₀cyclo-alkyl, C₃-C₁₀cycloalkylC₁-C₆alkyl, C₃-C₁₀cycloalkylcarbonyl-, C₃-C₁₀hetcycloalkylcarbonyl-, arylcarbonyl-, hetarylcarbonyl-, C₁-C₆alkyloxyC₁-C₆alkyl, NR¹²R¹³carbonylC₁-C₆-alkyl-, R¹⁴C₁-C₆alkylcarbonyl-, -COR¹⁵, arylC₁-C₆alkylS(O)ₙ-, arylC₁-C₆alkyl or hetarylC₁-C₆alkyl, wherein the alkyl, aryl and hetaryl groups independently are optionally substituted with one or more of R¹¹; and wherein R¹², R¹³, R¹⁴ and n are defined as above.

In another embodiment of the present invention, in formula (I) R⁹ is C₁-C₆alkyl, C₃-C₁₀cyclo-alkyl, C₃-C₁₀cycloalkylC₁-C₆alkyl, C₃-C₁₀cycloalkylcarbonyl-, C₃-C₁₀hetcycloalkylcarbonyl-, wherein the alkyl and cycloalkyl groups independently are optionally substituted with one or more of R¹¹.

In another embodiment of the present invention, in formula (I) R⁹ is arylcarbonyl-, hetarylcarbonyl-, NR¹²R¹³carbonylC₁-C₆alkyl-, R¹⁴C₁-C₆alkylcarbonyl-, wherein the alkyl, aryl and hetaryl groups independently are optionally substituted with one or more of R¹¹; and wherein R¹², R¹³ R¹⁴ R¹⁵ are as defined above.

In another embodiment of the present invention, in formula (I) R⁹ is C₁-C₆alkylS(O)₂-, arylS(O)₂-, arylC₁-C₆alkylS(O)₂-, wherein the alkyl and aryl groups independently are optionally substituted with one or more R¹⁸.

In another embodiment of the present invention, in formula (I) R⁹ is NR¹²R¹³-carbonylC₁-C₆alkyl- or R¹⁴C₁-C₆alkylcarbonyl-, wherein R¹², R¹³, and R¹⁴ are defined as above.

In another embodiment of the present invention, in formula (I) R⁹ is NR¹²R¹³carbonylC₁-C₆alkyl-, wherein R¹², and R¹³ are defined as above.

In another embodiment of the present invention, in formula (I) R¹⁰ is C₁-C₆alkyl, optionally substituted with one or more R¹¹.

In another embodiment of the present invention, in formula (I) R¹⁰ is arylC₁-C₆alkyl, wherein the alkyl and aryl groups independently are optionally substituted with one or more R¹¹.

In another embodiment of the present invention, in formula (I) R¹⁰ is NR¹²R¹³carbonylC₁-C₆alkyl, wherein the alkyl groups independently are optionally substituted with one or more R¹¹_{.}

In another embodiment of the present invention, in formula (I) R¹¹ is R⁵, R⁶, halo, hydroxy, C₁-C₈alkyloxy, oxo, cyano, -COR¹⁵, C₁-C₈alkyl or trihalomethyl.

In another embodiment of the present invention, in formula (I) R¹² and R¹³ independently are hydrogen, C₁-C₈alkyl, C₃-C₁₀cycloalkyl, C₃-C₁₀hetycloalkyl, aryl, hetaryl, arylC₁-C₆alkyl or hetarylC₁-C₆alkyl, wherein the alkyl, aryl and hetaryl groups independently are optionally substituted with one or more of R¹⁸.

In another embodiment of the present invention, in formula (I) R¹² and R¹³ together with the nitrogen to which they are attached, are forming a saturated or partially saturated cyclic, bicyclic or tricyclic ring system containing from 4 to 12 carbon atoms and from 0 to 2 additional heteroatoms selected from nitrogen or oxygen, the ring system optionally being substituted with at least one R⁵, R⁵oxy-, R⁶, halo, cyano, hydroxy, oxo, C₁-C₈alkyl, aryl, hetaryl, arylC₁-C₆alkyl, hetarylC₁-C₆alkyl, C₁-C₆alkyloxyC₁-C₆alkyl, R¹⁴C₁-C₆alkylcarbonyl- or-COR¹⁵.

In another embodiment of the present invention, in formula (I) R¹⁴ is C₁-C₆alkyloxy, C₃-C₁₀cycloalkyloxy-, C₃-C₁₀cycloalkylC₁-C₆alkyloxy-, C₃-C₁₀hetcycloalkyloxy-, arylC₁-C₆alkyloxy, hetarylC₁-C₆alkyloxy, -NR¹²R¹³, or -COR¹⁵, wherein the alkyl, cycloalkyl, hetcycloalkyl, aryl and hetaryl groups independently are optionally substituted with one or more of R²⁰.

In another embodiment of the present invention, in formula (I) R¹⁵ is C₁-C₆alkyl, hydroxy or C₁-C₈alkyloxy.

In another embodiment of the present invention, in formula (I) R¹⁵ is aryl, aryloxy or arylC₁-C₆alkyloxy.

In another embodiment of the present invention, the compounds of the general formula (I) is selected from the group of compounds of examples 1 and 3 through 16, or salt thereof with a pharmaceutically acceptable acid or base, or any optical isomer or mixture of optical isomers, including a racemic mixture, or any tautomeric forms.

The compounds of the present invention have asymmetric centers and may occur as racemates, racemic mixtures, and as individual enantiomers or diastereoisomers, with all isomeric forms being included in the present invention as well as mixtures thereof.

The present invention also encompasses pharmaceutically acceptable salts of the present compounds. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci., 66, 2 (1977), which is incorporated herein by reference. Examples of metal salts include lithium, sodium, potassium, barium, calcium, magnesium, zinc, calcium salts and the like. Examples of amines and organic amines include ammonium, methylamine, dimethylamine, trimethylamine, ethylamine, diethylamine, propylamine, butylamine, tetramethylamine, ethanolamine, diethanolamine, triethanolamine, meglumine, ethylenediamine, choline, N,N'-dibenzylethylenediamine, N-benzylphenylethylamine, N-methyl-D-glucamine, guanidine and the like. Examples of cationic amino acids include lysine, arginine, histidine and the like.

Further, some of the compounds of the present invention may form solvates with water or common organic solvents. Such solvates are encompassed within the scope of the invention.

The pharmaceutically acceptable salts are prepared by reacting a compound of the present invention with 1 to 4 equivalents of a base such as sodium hydroxide, sodium methoxide, sodium hydride, potassium *tert*-butoxide, calcium hydroxide, magnesium hydroxide and the like, in solvents like ether, THF, methanol, *tert*-butanol, dioxane, isopropanol, ethanol etc. Mixtures of solvents may be used. Organic bases like lysine, arginine, diethanolamine, choline, guandine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane etc. Mixture of solvents may also be used.

The stereoisomers of the compounds forming part of this invention may be prepared by using reactants in their single enantiomeric form in the process wherever possible or by conducting the reaction in the presence of reagents or catalysts in their single enantiomer form or by resolving the mixture of stereoisomers by conventional methods. Some of the preferred methods include use of microbial resolution, enzymatic resolution, resolving the diastereomeric salts formed with chiral acids such as mandelic acid, camphorsulfonic acid, tartaric acid, lactic acid, and the like wherever applicable or chiral bases such as brucine, (R)- or (S)-phenylethylamine, cinchona alkaloids and their derivatives and the like. Commonly used methods are compiled by Jaques et al. in "Enantiomers, Racemates and Resolution" (Wiley Interscience, 1981). More specifically the compound of the present invention may be converted to a 1:1 mixture of diastereomeric amides by treating with chiral amines, aminoacids, aminoalcohols derived from aminoacids; conventional reaction conditions may be employed to convert acid into an amide; the diastereomers may be separated either by fractional crystallization or chromatography and the stereoisomers of compound of formula I may be prepared by hydrolysing the pure diastereomeric amide.

Various polymorphs of the compounds forming part of this invention may be prepared by crystallization of said compounds under different conditions; for example, using different solvents commonly used or their mixtures for recrystallization; crystallizations at different temperatures; or various modes of cooling, ranging from very fast to very slow cooling during crystallizations. Polymorphs may also be obtained by heating or melting the compound followed by gradual or fast cooling. The presence of polymorphs may be determined by solid probe nmr spectroscopy, ir spectroscopy, differential scanning calorimetry, powder X-ray diffraction or such other techniques.

The disclosure also encompasses prodrugs of the present compounds, which on administration undergo chemical conversion by metabolic processes before becoming active pharmacological substances. In general, such prodrugs will be functional derivatives of the present compounds, which are readily convertible *in vivo* into the required compound of the present invention. Conventional procedures for the selection and preparation of suitable prodrug derivatives are described, for example, in "Design of Prodrugs", ed. H. Bundgaard, Elsevier, 1985.

It is a well known problem in drug discovery that compounds, such as enzyme inhibitors, may be very potent and selective in biochemical assays, yet be inactive *in vivo.* This lack of so-called bioavailability may be ascribed to a number of different factors such as lack of or poor absorption in the gut, first pass metabolism in the liver and/or poor uptake in cells. Although the factors determining bioavailability are not completely understood, there are many examples in the scientific literature - well known to those skilled in the art - of how to modify compounds, which are potent and selective in biochemical assays but show low or no activity *in vivo,* into drugs that are biologically active.

It is within the scope of the disclosure to modify the compounds of the present invention, termed the 'original compound', by attaching chemical groups that will improve the bioavailability of said compounds in such a way that the uptake in cells or mammals is facilitated.

Examples of said modifications, which are not intended in any way to limit the scope of the invention, include changing of one or more carboxy groups to esters (for instance methyl esters, ethyl esters, *tert-*butyl, acetoxymethyl, pivaloyloxymethyl esters or other acyloxymethyl esters). Compounds of the invention, original compounds, such modified by attaching chemical groups are termed 'modified compounds'.

The disclosure also encompasses active metabolites of the present compounds.

The compounds according to the invention and the compound that is the subject of the proviso alter, and more specifically, reduce the level of active intracellular glucocorticoid and are accordingly useful for the treatment, prevention and/or prophylaxis of disorders and diseases in which such a modulation or reduction is beneficial.

Accordingly, the present compounds may be applicable for the treatment, prevention and/or prophylaxis of the metabolic syndrome, insulin resistance, dyslipidemia, hypertension, obesity, type 2 diabetes, impaired glucose tolerance (IGT), impaired fasting glucose (IFG), Latent Autoimmune Diabetes in the Adult (LADA), type 1 diabetes, diabetic late complications including cardiovascular diseases, cardiovascular disorders, disorders of lipid metabolism, neurodegenerative and psychiatric disorders, dysregulation of intraocular pressure including glaucoma, immune disorders, inappropriate immune responses, musculo-skeletal disorders, gastrointestinal disorders, polycystic ovarie syndrome (PCOS), reduced hair growth or other diseases, disorders or conditions that are influenced by intracellular glucocorticoid levels, adverse effects of increased blood levels of active endogenous or exogenous glucocorticoid, and any combination thereof, adverse effects of increased plasma levels of endogenous active glucocorticoid, Cushing's disease, Cushing's syndrome, adverse effects of glucocorticoid receptor agonist treatment of autoimmune diseases, adverse effects of glucocorticoid receptor agonist treatment of inflammatory diseases, adverse effects of glucocorticoid receptor agonist treatment of diseases with an inflammatory component, adverse effects of glucocorticoid receptor agonist treatment as a part of cancer chemotherapy, adverse effects of glucocorticoid receptor agonist treatment for surgical/post-surgical or other trauma, adverse effects of glucocorticoid receptor agonist therapy in the context of organ or tissue transplantation or adverse effects of glucocorticoid receptor agonist treatment in other diseases, disorders or conditions where glucocorticoid receptor agonists provide clinically beneficial effects.

More specifically the present compounds may be applicable for the treatment, prevention and/or prophylaxis of the metabolic syndrome, type 2 diabetes, diabetes as a consequence of obesity, insulin resistance, hyperglycemia, prandial hyperglycemia, hyperinsulinemia, inappropriately low insulin secretion, impaired glucose tolerance (IGT), impaired fasting glucose (IFG), increased hepatic glucose production, type 1 diabetes, LADA, pediatric diabetes, dyslipidemia, diabetic dyslipidemia, hyperlipidemia, hypertriglyceridemia, hyperlipoproteinemia, hypercholesterolemia, decreased HDL cholesterol, impaired LDL/HDL ratio, other disorders of lipid metabolism, obesity, visceral obesity, obesity as a consequence of diabetes, increased food intake, hypertension, diabetic late complications, micro-/macroalbuminuria, nephropathy, retinopathy, neuropathy, diabetic ulcers, cardiovascular diseases, arteriosclerosis, atherosclerosis, coronary artery disease, cardiac hypertrophy, myocardial ischemia, heart insufficiency, congestional heart failure, stroke, myocardial infarction, arrythmia, decreased blood flow, erectile dysfunction (male or female), myopathy, loss of muscle tissue, muscle wasting, muscle catabolism, osteoporosis, decreased linear growth, neurodegenerative and psychiatric disorders, Alzheimers disease, neuronal death, impaired cognitive function, depression, anxiety, eating disorders, appetite regulation, migraine, epilepsia, addiction to chemical substances, disorders of intraocular pressure, glaucoma, polycystic ovary syndrome (PCOS), inappropriate immune responses, inappropriate T helper-1/T helper-2 polarisation, bacterial infections, mycobacterial infections, fungal infections, viral infections, parasitic infestations, suboptimal responses to immunizations, immune dysfunction, partial or complete baldness, or diseases, disorders or conditions that are influenced by intracellular glucocorticoid levels and any combination thereof, adverse effects of glucocorticoid receptor agonist treatment of allergic-inflammatory diseases such as asthma and atopic dermatitis, adverse effects of glucocorticoid receptor agonist treatment of disorders of the respiratory system e.g. asthma, cystic fibrosis, emphysema, bronchitis, hypersensitivity, pneumonitis, eosinophilic pneumonias, pulmonary fibrosis, adverse effects of glucocorticoid receptor agonist treatment of inflammatory bowel disease such as Crohn's disease and ulcerative colitis; adverse effects of glucocorticoid receptor agonist treatment of disorders of the immune system, connective tissue and joints e.g. reactive arthritis, rheumatoid arthritis, Sjögren's syndrome, systemic lupus erythematosus, lupus nephritis, Henoch-Schbnlein purpura, Wegener's granulomatosis, temporal arteritis, systemic sclerosis, vasculitis, sarcoidosis, dermatomyositis-polymyositis, pemphigus vulgaris; adverse effects of glucocorticoid receptor agonist treatment of endocrinological diseases such as hyperthyroidism, hypoaldosteronism, hypopituitarism; adverse effects of glucocorticoid receptor agonist treatment of hematological diseases e.g. hemolytic anemia, thrombocytopenia, paroxysmal nocturnal hemoglobinuria; adverse effects of glucocorticoid receptor agonist treatment of cancer such as spinal cord diseases, neoplastic compression of the spinal cord, brain tumours, acute lymphoblastic leukemia, Hodgkin's disease, chemotherapy-induced nausea, adverse effects of glucocorticoid receptor agonist treatment of diseases of muscle and at the neuro-muscular joint e.g. myasthenia gravis and heriditary myopathies (e.g. Duchenne muscular dystrophy), adverse effects of glucocorticoid receptor agonist treatment in the context of surgery & transplantation e.g. trauma, post-surgical stress, surgical stress, renal transplantation, liver transplantation, lung transplantation, pancreatic islet transplantation, blood stem cell transplantation, bone marrow transplantation, heart transplantation, adrenal gland transplantation, tracheal transplantation, intestinal transplantation, corneal transplantation, skin grafting, keratoplasty, lens implantation and other procedures where immunosuppression with glucocorticoid receptor agonists is beneficial; adverse effects of glucocorticoid receptor agonist treatment of brain absess, nausea/vomiting, infections, hypercalcemia, adrenal hyperplasia, autoimmune hepatitis, spinal cord diseases, saccular aneurysms or adverse effects to glucocorticoid receptor agonist treatment in other diseases, disorders and conditions where glucocorticoid receptor agonists provide clinically beneficial effects.

Accordingly, in a further aspect the invention relates to a compound according to the invention for use as a pharmaceutical composition.

The invention also relates to pharmaceutical compositions comprising, as an active ingredient, at least one compound according to the invention including the compound of the proviso together with one or more pharmaceutically acceptable carriers or diluents.

The pharmaceutical composition is preferably in unit dosage form, comprising from about 0.05 mg/day to about 2000 mg/day, preferably from about 1mg/day to about 500 mg/day of a compound according to the invention.

In another embodiment, the patient is treated with a compound according to the invention for at least about 1 week, for at least about 2 weeks, for at least about 4 weeks, for at least about 2 months or for at least about 4 months.

In yet another embodiment, the pharmaceutical composition is for oral, nasal, transdermal, pulmonal or parenteral administration.

Furthermore, the invention relates to the use of a compound according to the invention including the compound of the proviso for the preparation of a pharmaceutical composition for the treatment, prevention and/or prophylaxis of disorders and diseases wherein a modulation or an inhibition of the activity of 11βHSD1 is beneficial.

In a preferred embodiment of the invention the present compounds are used for the preparation of a medicament for the treatment, prevention and/or prophylaxis of any diseases and conditions that are influenced by intracellular glucocorticoid levels as mentioned above.

Thus, in a preferred embodiment of the invention the present compounds are used for the preparation of a medicament for the treatment, prevention and/or prophylaxis of conditions and disorders where a decreased level of active intracellular glucocorticoid is desirable, such as the conditions and diseases mentioned above.

In yet a preferred embodiment of the invention the present compounds are used for the preparation of a medicament for the treatment, prevention and/or prophylaxis of the metabolic syndrome including insulin resistance, dyslipidemia, hypertension and obesity.

In yet another preferred embodiment of the invention the present compounds are used for the preparation of a medicament for the treatment, prevention and/or prophylaxis of type 2 diabetes, impaired glucose tolerance (IGT), impaired fasting glucose (IFG).

In yet another preferred embodiment of the invention the present compounds are used for the preparation of a pharmaceutical composition for the delaying or prevention of the progression from IGT to type 2 diabetes.

In yet another preferred embodiment of the invention the present compounds are used for the preparation of a pharmaceutical composition for the delaying or prevention of the progression of the metabolic syndrome into type 2 diabetes.

In still another preferred embodiment of the invention the present compounds are used for the preparation of a pharmaceutical composition for the treatment, prevention and/or prophylaxis of diabetic late complications including cardiovascular diseases; arteriosclerosis; atherosclerosis.

In a further preferred embodiment of the invention the present compounds are used for the preparation of a pharmaceutical composition for the treatment, prevention and/or prophylaxis of neurodegenerative and psychiatric disorders.

In yet a further preferred embodiment of the invention the present compounds are used for the preparation of a pharmaceutical composition for the treatment, prevention and/or prophylaxis of adverse effects of glucocorticoid receptor agonist treatment or therapy.

In another embodiment of the present invention, the route of administration may be any route which effectively transports a compound according to the invention to the appropriate or desired site of action, such as oral, nasal, buccal, transdermal, pulmonal, or parenteral.

In still a further aspect of the invention the present compounds are administered in combination with one or more further active substances in any suitable ratios. Such further active substances may e.g. be selected from antiobesity agents, antidiabetics, agents modifying the lipid metabolism, antihypertensive agents, glucocorticoid receptor agonists, agents for the treatment and/or prevention of complications resulting from or associated with diabetes and agents for the treatment and/or prevention of complications and disorders resulting from or associated with obesity.

Thus, in a further aspect of the invention the present compounds may be administered in combination with one or more antiobesity agents or appetite regulating agents.

Such agents may be selected from the group consisting of CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, PPAR (peroxisome proliferator-activated receptor) modulators, RXR (retinoid X receptor) modulators, TRβ agonists, AGRP (Agouti related protein) inhibitors, H3 histamine antagonists, opioid antagonists (such as naltrexone), exendin-4, GLP-1 and ciliary neurotrophic factor.

In one embodiment of the invention the antiobesity agent is leptin; dexamphetamine or amphetamine; fenfluramine or dexfenfluramine; sibutramine; orlistat; mazindol or phentermine.

Suitable antidiabetic agents include insulin, insulin analogues and derivatives such as those disclosed in EP 792 290 (Novo Nordisk A/S), e.g. N^{εB29}-tetradecanoyl des (B30) human insulin, EP 214 826 and EP 705 275 (Novo Nordisk A/S), e.g. Asp^{B28} human insulin, US 5,504,188 (Eli Lilly), e.g. Lys^{B28} Pro^{B29} human insulin, EP 368 187 (Aventis), e.g. Lantus, which are all incorporated herein by reference, GLP-1 (glucagon like peptide-1) and GLP-1 derivatives such as those disclosed in WO 98/08871 to Novo Nordisk A/S, which is incorporated herein by reference as well as orally active hypoglycaemic agents.

The orally active hypoglycaemic agents preferably comprise sulphonylureas, biguanides, meglitinides, glucosidase inhibitors, glucagon antagonists such as those disclosed in WO 99/01423 to Novo Nordisk A/S and Agouron Pharmaceuticals, Inc., GLP-1 agonists, potassium channel openers such as those disclosed in WO 97/26265 and WO 99/03861 to Novo Nordisk A/S which are incorporated herein by reference, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents and antilipidemic agents as PPARα modulators, PPARδ modulators, cholesterol absorption inhibitors, HSL (hormone-sensitive lipase) inhibitors and HMG CoA inhibitors (statins), nicotinic acid, fibrates, anion exchangers, compounds lowering food intake, bile acid resins, RXR agonists and agents acting on the ATP-dependent potassium channel of the β-cells.

In one embodiment, the present compounds are administered in combination with insulin or an insulin analogue or derivative, such as N^{εB29}-tetradecanoyl des (B30) human insulin, Asp^{B28} human insulin, Lys^{B28} Pro^{B29} human insulin, Lantus®, or a mix-preparation comprising one or more of these.

In a further embodiment the present compounds are administered in combination with a sulphonylurea e.g. tolbutamide, glibenclamide, glipizide or glicazide.

In another embodiment the present compounds are administered in combination with a biguanide e.g. metformin.

In yet another embodiment the present compounds are administered in combination with a meglitinide e.g. repaglinide or senaglinide.

In still another embodiment the present compounds are administered in combination with a thiazolidinedione e.g. troglitazone, ciglitazone, pioglitazone, rosiglitazone or compounds disclosed in WO 97/41097 such as 5-[[4-[3-Methyl-4-oxo-3,4-dihydro-2-quinazolinyl]methoxy]phenyl-methyl]thiazolidine-2,4-dione or a pharmaceutically acceptable salt thereof, preferably the potassium salt.

In yet another embodiment the present compounds may be administered in combination with the insulin sensitizers disclosed in WO 99/19313 such as (-) 3-[4-[2-Phenoxazin-10-yl)ethoxy]phenyl]-2-ethoxypropanoic acid or a pharmaceutically acceptable salts thereof, preferably the arginine salt.

In a further embodiment the present compounds are administered in combination with an α-glucosidase inhibitor e.g. miglitol or acarbose.

In another embodiment the present compounds are administered in combination with an agent acting on the ATP-dependent potassium channel of the β-cells e.g. tolbutamide, glibenclamide, glipizide, glicazide or repaglinide.

Furthermore, the present compounds may be administered in combination with nateglinide.

In still another embodiment the present compounds are administered in combination with an antihyperlipidemic agent or antilipidemic agent e.g. cholestyramine, colestipol, clofibrate, gemfibrozil, fenofibrate, bezafibrate, tesaglitazar, EML-4156, LY-818, MK-767, atorvastatin, fluvastatin, lovastatin, pravastatin, simvastatin, acipimox, probucol, ezetimibe or dextrothyroxine.

In a further embodiment the present compounds are administered in combination with more than one of the above-mentioned compounds e.g. in combination with a sulphonylurea and metformin, a sulphonylurea and acarbose, repaglinide and metformin, insulin and a sulphonylurea, insulin and metformin, insulin, insulin and lovastatin, etc.

Further, the present compounds may be administered in combination with one or more antihypertensive agents. Examples of antihypertensive agents are β-blockers such as alprenolol, atenolol, timolol, pindolol, propranolol, metoprolol, bisoprololfumerate, esmolol, acebutelol, metoprolol, acebutolol, betaxolol, celiprolol, nebivolol, tertatolol, oxprenolol, amusolalul, carvedilol, labetalol, β2-receptor blockers e.g. S-atenolol, OPC-1085, ACE (angiotensin converting enzyme) inhibitors such as quinapril, lisinopril, enalapril, captopril, benazepril, perindopril, trandolapril, fosinopril, ramipril, cilazapril, delapril, imidapril, moexipril, spirapril, temocapril, zofenopril, S-5590, fasidotril, Hoechst-Marion Roussel: 100240 (EP 00481522), omapatrilat, gemopatrilat and GW-660511, calcium channel blockers such as nifedipine, felodipine, nicardipine, isradipine, nimodipine, diltiazem, amlodipine, nitrendipine, verapamil, lacidipine, lercanidipine, aranidipine, cilnidipine, clevidipine, azelnidipine, barnidipine, efonodipine, iasidipine, iemildipine, iercanidipine, manidipine, nilvadipine, pranidipine, furnidipine, α-blockers such as doxazosin, urapidil, prazosin, terazosin, bunazosin and OPC-28326, diuretics such as thiazides/sulphonamides (e.g. bendroflumetazide, chlorothalidone, hydrochlorothiazide and clopamide), loop-diuretics (e.g. bumetanide, furosemide and torasemide) and potassium sparing diuretics (e.g. amiloride, spironolactone), endothelin ET-A antagonists such as ABT-546, ambrisetan, atrasentan, SB-234551, CI-1034, S-0139 and YM-598, endothelin antagonists e.g. bosentan and J-104133, renin inhibitors such as aliskiren, vasopressin V1 antagonists e.g. OPC-21268, vasopressin V2 antagonists such as tolvaptan, SR-121463 and OPC-31260, B-type natriuretic peptide agonists e.g. Nesiritide, angiotensin II antagonists such as irbesartan, candesartancilexetil, losartan, valsartan, telmisartan, eprosartan, candesartan, CL-329167, eprosartan, iosartan, olmesartan, pratosartan, TA-606, and YM-358, 5-HT2 agonists e.g. fenoldopam and ketanserin, adenosine A1 antagonists such as naftopidil, N-0861 and FK-352, thromboxane A2 antagonists such as KT2-962, endopeptidase inhibitors e.g. ecadotril, nitric oxide agonists such as LP-805, dopamine D1 antagonists e.g. MYD-37, dopamine D2 agonists such as nolomirole, n-3 fatty acids e.g. omacor, prostacyclin agonists such as treprostinil, beraprost, PGE1 agonists e.g. ecraprost, Na+/K+ ATPase modulators e.g. PST-2238, Potassium channel activators e.g. KR-30450, vaccines such as PMD-3117, Indapamides, CGRP-unigene, guanylate cyclase stimulators, hydralazines, methyldopa, docarpamine, moxonidine, CoAprovel, MondoBiotech-811.

Further reference can be made to Remington: The Science and Practice of Pharmacy, 19th Edition, Gennaro, Ed., Mack Publishing Co., Easton, PA, 1995.

Furthermore, the present compounds may be administered in combination with one or more glucocorticoid receptor agonists. Examples of such glucocorticoid receptor agonists are betametasone, dexamethasone, hydrocortisone, methylprednisolone, prednisolone, prednisone, beclomethasone, butixicort, clobetasol, flunisolide, flucatisone (and analogues), momethasone, triamcinolonacetonide, triamcinolonhexacetonide GW-685698, NXC-1015, NXC-1020, NXC-1021, NS-126, P-4112, P-4114, RU-24858 and T-25 series.

It should be understood that any suitable combination of the compounds according to the invention with one or more of the above-mentioned compounds and optionally one or more further pharmacologically active substances are considered to be within the scope of the present invention.

### PHARMACEUTICAL COMPOSITIONS

The compounds of the present invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well-known in the art.

Liquid dosage forms for oral administration include solutions, emulsions, suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, crèmes, gels, inhalants, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 2000 mg, e.g. from about 0.1 to about 1000 mg, from about 0.5 mg to about 500 mg., from about 1 mg to about 200 mg, e.g. about 100 mg.

For parenteral routes, such as intravenous, intrathecal, intramuscular and similar administration, typically doses are in the order of about half the dose employed for oral administration.

The compounds of this invention are generally utilized as the free substance or as a pharmaceutically acceptable salt thereof. Examples are an acid addition salt of a compound having the utility of a free base and a base addition salt of a compound having the utility of a free acid. The term "pharmaceutically acceptable salts" refers to non-toxic salts of the compounds for use according to the present invention which are generally prepared by reacting the free base with a suitable organic or inorganic acid or by reacting the acid with a suitable organic or inorganic base. When a compound for use according to the present invention, contains a free base such salts are prepared in a conventional manner by treating a solution or suspension of the compound with a chemical equivalent of a pharmaceutically acceptable acid. When a compounds for use according to the present invention, contains a free acid such salts are prepared in a conventional manner by treating a solution or suspension of the compound with a chemical equivalent of a pharmaceutically acceptable base. Physiologically acceptable salts of a compound with a hydroxy group include the anion of said compound in combination with a suitable cation such as sodium or ammonium ion. Other salts which are not pharmaceutically acceptable may be useful in the preparation of compounds for use according to the present invention and these form a further aspect of the present invention.

For parenteral administration, solutions of the present compounds in sterile aqueous solution, aqueous propylene glycol or sesame or peanut oil may be employed. Such aqueous solutions should be suitable buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of suitable carriers are water, salt solutions, alcohols, polyethylene glycols, polyhydroxyethoxylated castor oil, peanut oil, olive oil, syrup, phospholipids, gelatine, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents.

The pharmaceutical compositions formed by combining the compounds of the invention and the pharmaceutically acceptable carriers are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and which may include a suitable excipient. These formulations may be in the form of powder or granules, as a solution or suspension in an aqueous or non-aqueous liquid, or as an oil-in-water or water-in-oil liquid emulsion.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contain the active ingredient in admixture with non-toxic pharmaceutically-acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatine or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, incorporated herein by reference, to form osmotic therapeutic tablets for controlled release.

Formulations for oral use may also be presented as hard gelatine capsules where the active ingredient is mixed with an inert solid diluent, for example, calcium carbonate, calcium phosphate or kaolin, or a soft gelatine capsule wherein the active ingredient is mixed with water or an oil medium, for example peanut oil, liquid paraffin, or olive oil.

Aqueous suspensions may contain the active compounds in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present.

The pharmaceutical compositions comprising a compound for use according to the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, preservative and flavouring and colouring agent. The pharmaceutical compositions may be in the form of a sterile injectable aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the present invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the present invention are contemplated. For the purpose of this application, topical applications shall include mouth washes and gargles.

The compounds for use according to the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the compounds for use according to the present invention may form solvates with water or common organic solvents. Such solvates are also encompassed within the scope of the present invention.

Thus, in a further embodiment, there is provided a pharmaceutical composition comprising a compound for use according to the present invention, or a pharmaceutically acceptable salt, solvate, or prodrug thereof, and one or more pharmaceutically acceptable carriers, excipients, or diluents.

If a solid carrier is used for oral administration, the preparation may be tabletted, placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

A typical tablet which may be prepared by conventional tabletting techniques may contain:

### Core:

| | |
|---|---|
| Active compound (as free compound or salt thereof) | 5.0 mg |
| Lactosum Ph. Eur. | 67.8 mg |
| Cellulose, microcryst. (Avicel) | 31.4 mg |
| Amberlite^{®}IRP88* | 1.0 mg |
| Magnesii stearas Ph. Eur. | q.s. |

### Coating:

| | |
|---|---|
| Hydroxypropyl methylcellulose | approx. 9 mg |
| Mywacett 9-40 T** | approx. 0.9 mg |

| | |
|---|---|
| * Polacrillin potassium NF, tablet disintegrant, Rohm and Haas. ** Acylated monoglyceride used as plasticizer for film coating. | |

The compounds of the invention may be administered to a patient which is a mammal, especially a human in need thereof. Such mammals include also animals, both domestic animals, e.g. household pets, and non-domestic animals such as wildlife.

Any novel feature or combination of features described herein is considered essential to this invention.

The present invention also relate to the below methods of preparing the compounds of the invention.

The present invention is further illustrated in the following representative examples which are, however, not intended to limit the scope of the invention in any way.

### EXAMPLES

The following examples and general procedures refer to intermediate compounds and final products for general formula (I) identified in the specification and in the synthesis schemes. The preparation of the compounds of general formula (I) of the present invention is described in detail using the following examples. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, which is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed by either elemental analysis or nuclear magnetic resonance (NMR), where peaks assigned to characteristic protons in the title compounds are presented where appropriate. ¹H NMR shifts (δ_{H}) are given in parts per million (ppm) down field from tetramethylsilane as internal reference standard. M.p.: is melting point and is given in °C and is not corrected. Column chromatography was carried out using the technique described by W.C. Still et al., J. Org. Chem. 43: 2923 (1978) on Merck silica gel 60 (Art. 9385). HPLC analyses are performed using 5µm C18 4 x 250 mm column eluted with various mixtures of water and acetonitrile, flow = 1 ml/min, as described in the experimental section.

**Microwave oven synthesis:** The reaction was heated by microwave irradiation in sealed microwave vessels in a single mode Emrys Optimizer EXP from PersonalChemistry®.

**Preparative HPLC: Column:** 1.9 x 15 cm Waters XTerra RP-18. Buffer: linear gradient 5 - 95 % in 15 min, MeCN, 0.1 % TFA, flow rate of 15 ml/min. The pooled fractions are either evaporated to dryness *in vacuo*, or evaporated *in vacuo* until the MeCN is removed, and then frozen and freeze dried.

The abbreviations as used in the examples have the following meaning:
- TLC:: Thin layer chromatography
- CDCl₃:: Deuterio chloroform
- CD₃OD:: Tetradeuterio methanol
- DCM:: Dichloromethane
- DMF:: N,N-dimethylformamide
- DMSO-d₆:: Hexadeuterio dimethylsulfoxide
- DMSO:: Dimethylsulfoxide
- DIPEA:: Diisopropylethylamine
- EDAC:: 1-(3-Dimethylaminopropyl)-3-ethylcarbodiimide hydrochloride
- EtOAc:: Ethyl acetate
- THF:: Tetrahydrofuran
- DMF:: N,N-dimethylformamide
- HOBT:: 1-Hydroxy-benzotriazole
- MeCN:: Acetonitrile
- NMP:: N-Methylpyrrolidinone
- TFA:: Trifluoroacetic acid
- EDAC:: 1-(3-Dimethylaminopropyl)-3-ethylcarbodiimide, hydrochloride
- min:: minutes
- hrs:: hours

### General method A:

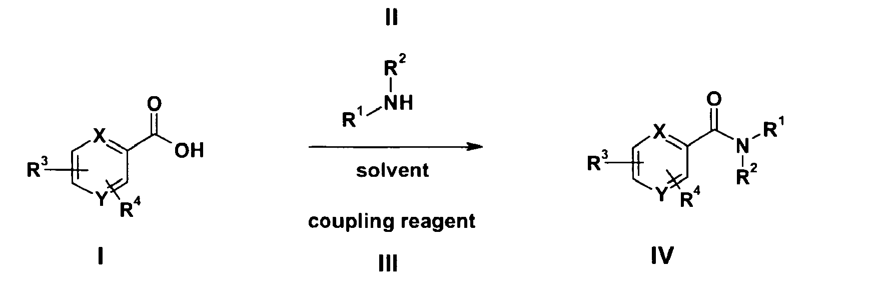

By allowing an acid (I) wherein R³, R⁴, X and Y are defined as above to be coupled with an amine (II) wherein R¹ and R² are defined as above under standard amide forming conditions using a coupling reagent (III) (e.g. HOBT, EDAC and DIPEA in dry THF) affording amide (IV) wherein R¹, R², R³, R⁴, X and Y are defined as above.

### General method B:

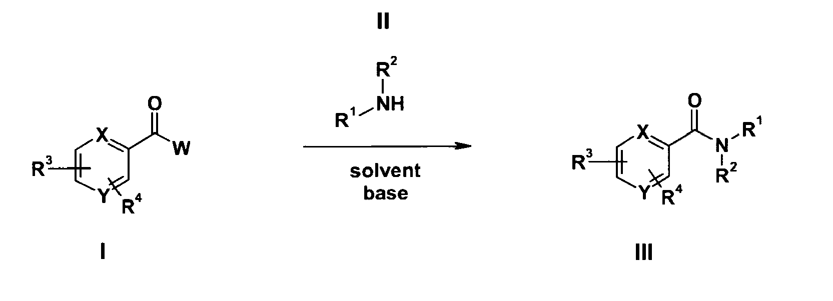

By allowing an acid derivative **(I)** wherein W is halo, R²⁰(C=O)O-, C₁-C₆alkyloxy or arylC₁-C₆alkyloxy, R²⁰ is C₁-C₆alkyl or arylC₁-C₆alkyl and R³, R⁴, X and Y are defined as above to react with an amine **(II)** wherein R¹ and R² are defined as above under basic conditions (e.g. triethylamine, K₂CO₃, NaH and the like) in a solvent (e.g. THF, DCM, DMF, NMP and the like) affording amide **(III);** wherein R¹, R², R³, R⁴, X and Y are defined as above.

### General method C:

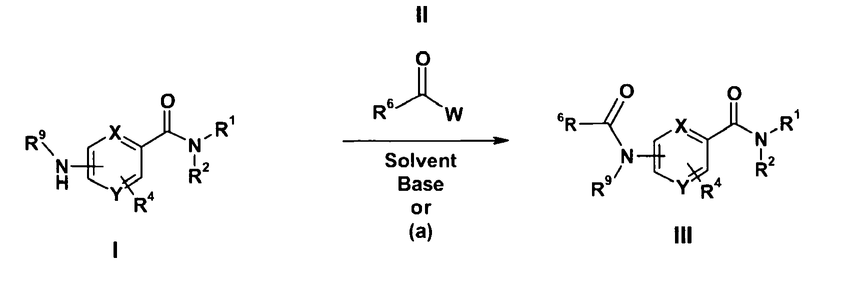

By allowing an amide derivative **(I)** wherein R¹, R², R⁴, R⁹, X and Y are defined as above to react with an acid derivative **(II)** wherein W is halo, R⁷(C=O)O-, C₁-C₆alkyloxy or arylC₁-C₆alkyloxy, R⁷ is C₁-C₆alkyl or arylC₁-C₆alkyl and R⁶ is C₃-C₁₀cycloalkyl, C₃-C₁₀hetcycloalkyl, aryl, hetaryl, R¹⁴C₁-C₆alkylcarbonyl-, C₁-C₆alkylS(O)ₙ-, arylS(O)ₙ- or arylC₁-C₆alkylS(O)ₙ-, wherein the alkyl, cycloalkyl, hetcycloalkyl, aryl groups optionally are substituted with one or more R¹¹ as defined above under basic conditions (e.g. triethylamine, K₂CO₃, NaH and the like) in a solvent (e.g. THF, DCM, DMF, NMP and the like) affording amide **(III);** wherein R¹, R², R⁴, R⁹, X and Y are defined as above and R⁶ is C₃-C₁₀cycloalkyl, C₃-C₁₀hetcycloalkyl, aryl, hetaryl, R¹⁴C₁-C₆alkyl-carbonyl-, C₁-C₆alkylS(O)ₙ-, arylS(O)ₙ- or arylC₁-C₆alkylS(O)ₙ-, wherein the alkyl, cycloalkyl, hetcycloalkyl, aryl groups optionally are substituted with one or more R¹¹ as defined above; or
when W is hydroxy the acid derivative **(II)** wherein R⁶ is as defined above is coupled with an amine (I) wherein R¹, R², R⁴, R⁹, X and Y are defined as above under standard amide forming conditions using a coupling reagent (a) (e.g. HOBT, EDAC and DIPEA in dry THF) affording amide **(III)** wherein R¹, R², R⁴, R⁹, X and Y are defined as above and R⁶ is C₃-C₁₀cycloalkyl, C₃-C₁₀hetcyclo-alkyl, aryl, hetaryl, R¹⁴C₁-C₆alkylcarbonyl-, C₁-C₆alkylS(O)ₙ-, arylS(O)ₙ- or arylC₁-C₆alkylS(O)ₙ-, wherein the alkyl, cycloalkyl, hetcycloalkyl, aryl groups optionally are substituted with one or more R¹¹ as defined above.

### General method D:

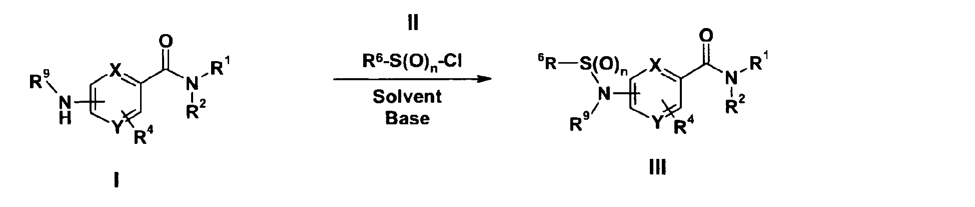

By allowing an amide derivative **(I)** wherein R¹, R², R⁴, R⁹, X and Y are defined as above to react with a sulfinyl (n = 1) or sulfonyl (n = 2) chloride derivative **(II)** wherein R⁶ is C₁-C₆alkyl, C₃-C₁₀cycloalkyl, C₃-C₁₀hetcycloalkyl, aryl, hetaryl or arylC₁-C₆alkyl, wherein the alkyl, cycloalkyl, hetcycloalkyl, aryl and hetaryl groups optionally are substituted with one or more R¹¹ as defined above under basic conditions (e.g. triethylamine, K₂CO₃, NaH and the like) in a solvent (e.g. THF, DCM, DMF, NMP and the like) to afford an amide **(III);** wherein R¹, R², R⁴, R⁹, n, X and Y are defined as above and R⁶ is C₁-C₆alkyl, C₃-C₁₀cycloalkyl, C₃-C₁₀hetcycloalkyl, aryl, hetaryl or arylC₁-C₆alkyl, wherein the alkyl, cycloalkyl, hetcycloalkyl, aryl and hetaryl groups optionally are substituted with one or more R¹¹ as defined above.

### General method E:

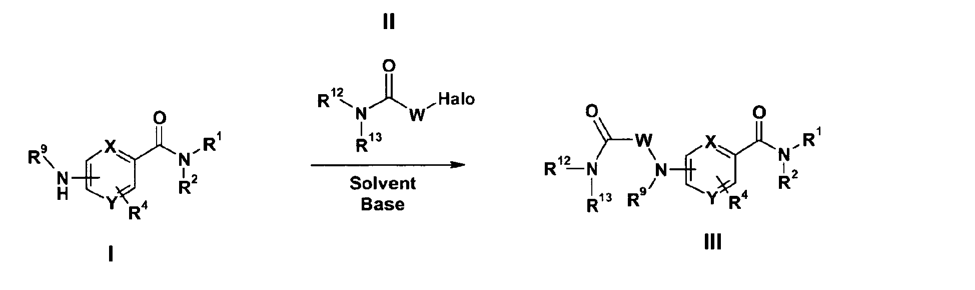

By allowing an amide derivative **(I)** wherein R¹, R², R⁴, R⁹, X and Y are defined as above to react with an amido alkyl halide derivative **(II)** wherein R¹², R¹³ and halo are defined above and W is C₁-C₆alkyl, wherein the alkyl group optionally is substituted with one or more R¹¹ as defined above under basic conditions (e.g. triethylamine, K₂CO₃, NaH and the like) in a solvent (e.g. THF, DCM, DMF, NMP and the like) to afford an amide **(III);** wherein R¹, R², R⁴, R⁹, R¹², R¹³, X and Y are defined as above and W is C₁-C₆alkyl, wherein the alkyl group optionally is substituted with one or more R¹¹ as defined above.

### General method F:

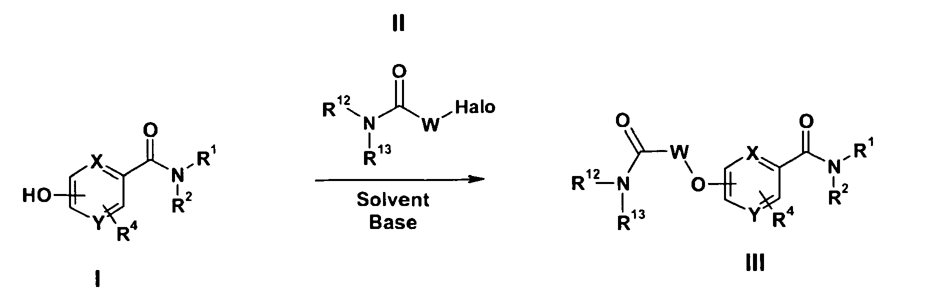

By allowing an amide derivative **(I)** wherein R¹, R², R⁴, X and Y are defined as above to react with an amido alkyl halide derivative **(II)** wherein R¹², R¹³ and halo are defined above and W is C₁-C₆alkyl, wherein the alkyl group optionally is substituted with one or more R¹¹ as defined above under basic conditions (e.g. triethylamine, K₂CO₃, NaH and the like) in a solvent (e.g. THF, DCM, DMF, NMP and the like) to afford an amide **(III);** wherein R¹, R², R⁴, R¹², R¹³, X and Y are defined as above and W is C₁-C₆alkyl, wherein the alkyl group optionally is substituted with one or more R¹¹ as defined above.

### NNC 0###-0000-####-Example 1{ TC "Example 1,6633expand;Bio1: # nM;Bio2: # nM" }

### [4-(1,3,3-Trimethyl-6-aza-bicyxlo[3.2.3]octane-6-carbonyl)-phenyl]carbamic acid tert-butyl ester

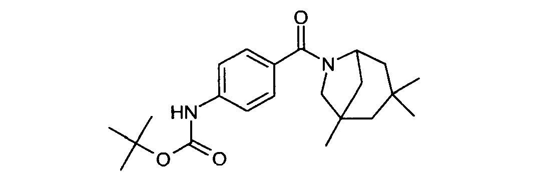

To a mixture of 4-*tert*-butoxycarbonylamino-benzoic acid (50 g, 0.21 mol) and HOBT (31,33 g, 0.231 mol) in dry THF (0.5 L) was added EDAC (44.44 g, 0.231 mol). The resulting mixture was stirred for 10 min followed by addition of a mixture of DIPEA (40.4 ml, 0.231 mol) and 1,3,3-trimethyl-6-aza-bicyclo[3.2.1]octane (39.4 ml, 0.231 mol). The reaction mixture was stirred for an additional 16 hrs. and evaporated to dryness. To the residue was added water (600 ml) and the resulting mixture was extracted with EtOAc (3 x 500 ml). The combined organic phases were dried (Na₂SO₄), filtered and evaporated in vacuo. The resulting residue was purified by column chromatography (silica gel) using a mixture of EtOAc-Heptane (1:2) as eluent. Pure fractions were collected and evaporated to dryness. To the solid residue was added diethyl ether (100 ml) and the precipitate was filtered off, washed with diethyl ether and dried in vacuo at 50°C affording 60.5 g (77 %) of the title compound as a solid.
H-NMR (300 MHz, CDCl₃) δ 0.92 (d, 3H), 1.02 (d, 3H), 1.11 (s, 3H), 1.2 - 1.4 (m, 3.5H), 1.52 (s, 9H), 1.55 - 2.27 (m, 2.5H), 3.17 - 3.29 (m, 1.5H), 3.57 (d, 0.5H), 4.01 and 4.58 (2xt, 1H), 6.72 (s, 1H), 7.36 - 7.44 (m, 4H).

### Reference NNC 0###-0000-####-Example 2{ TC "Example 2 6633expand;Bio1: # nM;Bio2: # nM" }

### (4-Amino-phenyl)(1,3,3-trimethyl-6-aza-bicyclo[3.2.1]oct-6-yl)-methanone

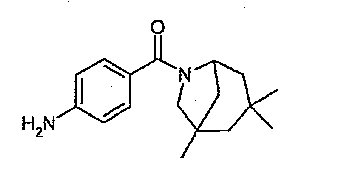

To a solution of [4-(1,3,3-trimethyl-6-aza-bicyclo[3.2.1]octane-6-carbonyl)-phenyl]carbamic acid *tert*-butyl ester (11.78 g, 31.61 mmol) in DCM (150 ml) was added TFA (50 ml). The resulting mixture was stirred for 48 hrs. at room temperature and evaporated in vacuo. To the residue was added water (150 ml) and diethyl ether (50 ml) and the pH was adjusted to 8 by addition of 32 % aq. sodium hydroxide. The mixture was stirred for 15 min., the precipitate filtered off, washed with water and dried in vacuo at 50°C for 18 hrs. affording 8.4 g (98 %) of the title compound as a solid.
¹H-NMR (300 MHz, CDCl₃) δ 0.93 (s, 3H), 1.02 (d, 3H), 1.12 (d, 3H), 1.35 - 1.76 (m, 5.5H), 2.24 (m, 0.5H), 3.21 - 3.36 (m, 1.5H), 3.54 (d, 0.5H), 3.83 (bs, 2H, N*H*₂), 4.08 and 4.58 (2xm, 1H), 6.64 (dd, 2H), 7.32 (t, 2H)

The following reference compound was made in a similar way as described in example 2 above:

| **No** | **Molecule** | **MW** | **IUPAC Name** |
|---|---|---|---|
| 2-1 | 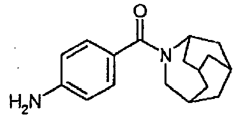 | 270.38 | (4-Amino-phenyl)-(4-azatricyclo[4.3.1.13,8]undec-4-yl)-methanone |

### NNC 0###-0000-####-Example 3{ TC "Example 3;Bio1: # nM;Bio2: # nM" }

### (4-Methylamino-phenyl)-(1,3,3-trimethyl-6-aza-bicyclo[3.2.1]oct-6-yl)-methanone

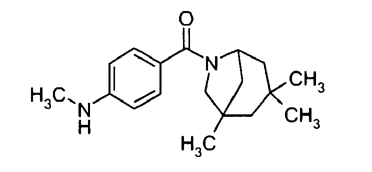

To a solution of [4-(1,3,3-trimethyl-6-aza-bicyclo[3.2.1]octane-6-carbonyl)-phenyl]carbamic acid *tert*-butyl ester (2.6 g, 6.98 mmol) in dry DMF (75 ml) was added NaH (0.35 g, 8.38 mmol 60 % in mineral oil) and the resulting mixture was stirred for 1 hr. Methyl iodide (521 µl, 8.38 mmol) was added and the stirring was continued for an additional 1 hr. The reaction mixture was quenched with water (50 ml) followed by extraction with diethyl ether (2 x 50ml). The combined organic phases were washed with saturated aqueous ammonium chloride, dried (Na₂SO₄), filtered and evaporated in vacuo. The residue was dissolved in DCM (40 ml) and TFA (25 ml) was added. The resulting mixture was stirred for 18 hrs. at room temperature and evaporated in vacuo. To the residue was added water (10 ml) and the pH was adjusted to 9 by addition of 1 N sodium hydroxide. The aqueous phase was extracted with diethyl ether (2 x 25 ml) and the combined organic phases were dried (Na₂SO₄), filtered and evaporated in vacuo affording 2.5 g crude product which was purified by column chromatography (silica gel) using a mixture of EtOAc-Heptane (1:1) as eluent. Pure fractions were collected and evaporated to dryness affording a slowly crystallizing oil which was washed with diethyl ether (10 ml), filtered off and dried in vacuo at 50 °C afforded 0.9 g (45 %) of the title compound as a solid.
An additional 0.7 g (35 %) was isolated from the filtrate.
TLC: EtOAc-Heptane (3:1), R_{f}: 0.37
¹H-NMR (300 MHz, CDCl₃) δ 0.94 (s, 3H), 1.02 (d, 3H), 1.12 (d, 3H), 1.21 - 1.76 (m, 5.5H), 2.25 (m, 0.5H), 2.85 (s, 3H), 3.25 - 3.39 (m, 1.5H), 3.54 (d, 0.5 H), 4.10 (m, 1.5H), 4.58 (m, 0.5H), 6.57 (m, 2H), 7.37 (t, 2H).

### NNC 0###-0000-####-Example 4{ TC "Example 4;Bio1: # nM;Bio2: # nM" }

### N-Methyl-N-[4-(1,3,3-trimethyl-6-aza-bicyclo[3.2.1]octane-6-carbonyl)-phenyl]-methanesulfonamide

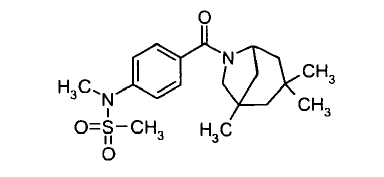

To a mixture of (4-methylamino-phenyl)-(1,3,3-trimethyl-6-aza-bicyclo[3.2.1]oct-6-yl)-methanone (0.7 g, 2.44 mmol), DCM (40 ml) and TEA (0.7 ml, 4.89 mmol) was added methansulfonyl chloride (285 µl, 3.67 mmol). The resulting mixture was stirred for 4 hrs. at room temperature followed by evaporation of the volatiles in vacuo. The residue was dissolved in diethyl ether (10 ml), washed with water (2x10 ml) evaporated and the residue purified by column chromatography (silica gel) using a mixture of EtOAc-Heptane (1:1) as eluent. Pure fractions were collected and evaporated to dryness. The residue was crystallized from diethyl ether (10 ml), filtered off and dried in vacuo at 50 °C affording 0.45 g (51 %) of the title compound as a solid.
TLC: EtOAc-Heptane (3:1), R_{f}: 0.25
¹H-NMR (300 MHz, CDCl₃) δ 0.94 (d, 3H), 1.03 (d, 3H), 1.13 (s, 3H), 1.19- 1.61 (m, 5.5H), 2.25 (m, 0.5H), 2.85 (d, 3H), 3.16 - 3.31 (m, 1.5H), 3.34 (d, 3H), 3.60 (d, 0.5H), 4.00 (t, 0.5H), 4.60 (t, 0.5H), 7.41 (m, 2H), 7.48 (t, 2H).

The following compounds were made in a similar way as described in example 4 above:

| **No** | **Molecule** | **MW** | **IUPAC Name** |
|---|---|---|---|
| 4-1 | 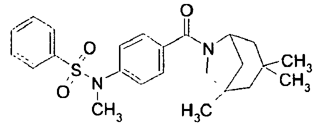 | 426.58 | N-Methyl-N-[4-(1,3,3-trimethyl-6-azabicyclo[3.2.1]octane-6-carbonyl)-phenyl]-benzenesulfonamide |
| 4-2 | 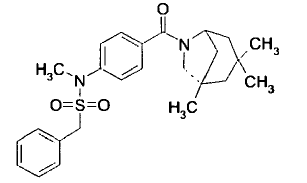 | 440.61 | N-Methyl-C-phenyl-N-[4-(1,3,3-trimethyl-6-aza-bicyclo[3.2.1]octane-6-carbonyl)-phenyl]-methanesulfonamide |

### NNC 0###-0000-####-Example 5{ TC "Example 5;Bio1: # nM;Bio2: # nM" }

### N-[4-(1,3,3-Trimethyl-6-aza-bicyclo[3.2.1]octane-6-carbonyl)-phenyl]-methanesulfonamide

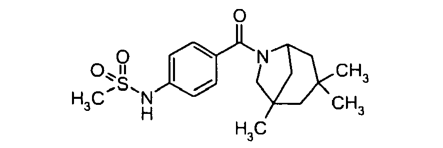

To a solution of [4-(1,3,3-trimethyl-6-aza-bicyclo[3.2.1]octane-6-carbonyl)-phenyl]carbamic acid *tert*-butyl ester (4.0 g, 10.74 mmol) in dry DMF (80 ml) was added sodium hydride (309 mg, 12.89 mmol in 60% mineral oil) and the mixture was stirred for 1 hr. Methane sulfonyl chloride (2.4 ml, 15.47 mmol) was added and the stirring was continued for an additional 20 hrs. The reaction was quenched by addition of water (100 ml) and extracted with diethyl ether (2 x 100 ml). The combined organic phases were dried (Na₂SO₄), filtered and evaporated in vacuo. The residue was purified by column chromatography (silica gel) using EtOAc-Heptane (1:2) as eluent. Pure fractions were collected and evaporated to dryness affording 3.0 g (62 %) of N-methanesulphonamide-N-[4-(1,3,3-trimethyl-6-aza-bicyclo[3.2.1]octane-6-carbonyl)-phenyl]carbamic acid *tert*-butyl ester as an oil.
¹H-NMR (400 MHz, CDCl₃) δ 0.93 (d, 3H), 1.03 (s, 3H), 1.13 (s, 3H), 1.18-1.41 (m, 3H), 1.45 (d, 9H), 1.51- 1.78 (m, 2.5 H), 2.23 (m, 0.5H), 3.14 (d, 0.5H), 3.27 (t, 1H), 3.45 (s, 3H), 3.59 (d, 0.5 H), 3.97 (t, 0.5H), 4.61 (m, 0.5H), 7.28 (m, 2H), 7.50 (m, 2H).

*N*-Methanesulphonamide-*N*-[4-(1,3,3-trimethyl-6-aza-bicyclo[3.2.1]octane-6-carbonyl)-phenyl]carbamic acid *tert*-butyl ester (3.0 g, 6.66 mmol) was dissolved in DCM (50 ml) and TFA (20 ml) was added. The resulting mixture was stirred for 36 hrs. at room temperature and evaporated in vacuo. To the residue was added water (50 ml) and the pH was adjusted to 9 by addition of 1 N sodium hydroxide. The aqueous phase was extracted with EtOAc (2 x 50 ml) and the combined organic phases were dried (Na₂SO₄), filtered and evaporated in vacuo affording a foam which was crystallized by addition of diethyl ether (30 ml) containing a few ml of EtOAc. The precipitate was filtered off and dried in vacuo at 50 °C affording 1.58 g (68 %) of the title compound as a solid.
¹H-NMR (400 MHz, CDCl₃) δ 0.94 (d, 3H), 1.04 (d, 3H), 1.13 (s, 3H), 1.19- 1.61 (m, 4.5H), 1.77 (m, 1H), 2.23 (m, 0.5H), 3.03 (s, 3H), 3.1 - 3.3 (m, 1.5H), 3.59 (d, 0.5H), 3.99 (t, 0.5H), 4.60 (t, 0.5H), 6.86 (bs, 1H), 7.22 (m, 2H), 7.46 (t, 2H).

### NNC 0###-0000-####-Example 6{ TC "Example 6;Bio1: # nM;Bio2: # nM" }

### N-Ethyl-N-[4-(1,3,3-trimethyl-6-aza-bicyclo[3.2.1]octane-6-carbonyl)-phenyl]-methanesulfonamide

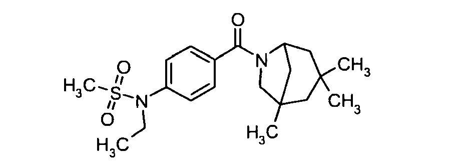

To a solution of N-[4-(1,3,3-trimethyl-6-aza-bicyclo[3.2.1]octane-6-carbonyl)-phenyl]-methanesulfonamide (300 mg, 0.86 mmol) in dry DMF (25 ml) was added sodium hydride (42 mg, 1.73 mmol in 60 % mineral oil) and the mixture was stirred for 1 hr. Ethyl iodide (83 µl, 1.03 mmol) was added and the stirring was continued for an additional 16 hrs. The reaction was quenched by addition of water (25 ml) and extracted with diethyl ether (2x 50 ml). The combined organic phases were dried (Na₂SO₄), filtered and evaporated in vacuo. The residue was purified by column chromatography (silica gel) using EtOAc-Heptane (1:1) as eluent. Pure fractions were collected and evaporated to dryness affording a slowly crystallizing oil which was suspended in diethyl ether and the solid filtered off affording 140 mg (43 %) of the title compound as a solid.
¹H-NMR (400 MHz, CDCl₃) δ 0.95 (d, 3H), 1.04 (s, 3H), 1.13 - 1.80 (m, 8.5H), 2.24 (m, 0.5H), 2.90 (s, 3H), 3.17 (d, 0.5H), 3.29 (d, 1H), 3.59 (d, 0.5H), 3.76 (q, 2H), 4.01 (t, 0.5H), 4.61 (m, 0.5H), 7.38 (m, 2H), 7.17 (m, 2H), 7.50 (m, 2H).

The following compound was made in a similar way as described in example 6 above:

| **No** | **Molecule** | **MW** | **IUPAC Name** |
|---|---|---|---|
| 6-1 | 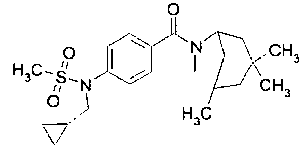 | 404.58 | N-Cyclopropylmethyl-N-[4-(1,3,3-trimethyl-6-aza-bicyclo[3.2.1]octane-6-carbonyl)-phenyl]-methanesulfonamide |

### NNC 0###-0000-####-Example 7{ TC "Example 7;Bio1: # nM;Bio2: # nM" }

### N-[4-(1,3,3-Trimethyl-6-aza-bicyclo[3.2.1]octane-6-carbonyl)-phenyl]-isonicotinamide

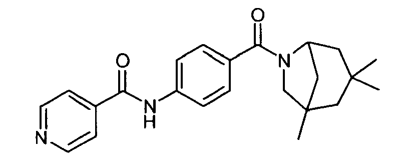

To a solution of (4-amino-phenyl)-(1,3,3-trimethyl-6-aza-bicyclo[3.2.1]oct-6-yl)-methanone (400 mg, 1.47 mmol) in dry THF (25 ml) was added dropwise a solution of isonicotinoyl chloride (312 mg, 2.20 mmol) in dry THF (20 ml). The mixture was stirred for 2 hrs. and evaporated followed by addition of water 20 (ml). The aqueous phase was extracted with EtOAc (2 x 20 ml) and the combined organic phases were dried (Na₂SO₄), filtered and evaporated in vacuo. The resulting residue was purified by column chromatography (silica gel) using first EtOAc (300 ml) followed by a 4% mixture of TEA in EtOAc as eluents. Pure fractions were collected and evaporated to dryness. The residue was crystallized from a mixture of diethyl ether (20 ml) and EtOAc (5 ml) affording after filtering and drying in vacuo at 50 °C 200 mg (36 %) of the title compound as a solid. ¹H-NMR (400 MHz, CDCl₃) δ 0.94 (d, 3H), 1.02 (d, 3H), 1.12 (d, 3H), 1.19 - 1.77 (m, 5.5H), 2.21 (m, 0.5H), 3.16 - 3.27 (m, 1.5H), 3.57 (d, 0.5H), 4.0 and 4.54 (2xm, 1H), 7.30 (t, 2H), 7.55 (t, 2H), 7.87 (d, 2H), 8.76 (d, 2H), 9.19 (s, 1H).

The following compounds were made in a similar way as described in example 7 above:

| **No** | **Molecule** | **MW** | **IUPAC Name** |
|---|---|---|---|
| 7-1 | 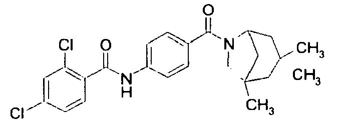 | 445.39 | 2,4-Dichloro-N-[4-(1,3,3-trimethyl-6-aza-bicyclo[3.2.1]octane-6-carbonyl)-phenyl]-benzamide |
| 7-2 | 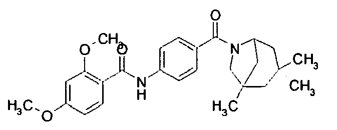 | 436.56 | 2,4-Dimethoxy-N-[4-(1,3,3-trimethyl-6-aza-bicyclo[3.2.1]octane-6-carbonyl)-phenyl]-benzamide |
| 7-3 | 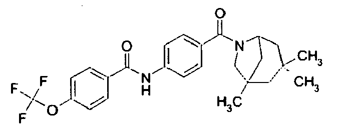 | 460.50 | 4-Trifluoromethoxy-N-[4-(1,3,3-trimethyl-6-aza-bicyclo[3.2.1]octane-6-carbony)-phenyl]-benzamide |
| 7-4 | 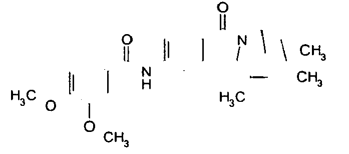 | 436.56 | 3,4-Dimethoxy-N-[4-(1,3,3-trimethyl-6-aza-bicyclo[3.2.1]octane-6-carbonyl)-phenyl]-benzamide |
| 7-5 | 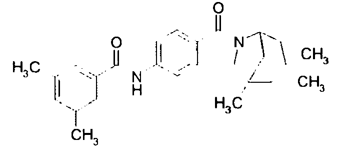 | 404.56 | 3,5-Dimethyl-N-[4-(1,3,3-trimethyl-6-aza-bicyclo[3.2.1]octane-6-carbonyl)-phenyl]-benzamide |
| 7-6 | 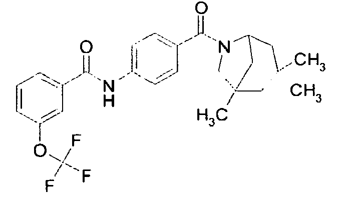 | 460.50 | 3-Trifluoromethoxy-N-[4-(1,3,3-trimethyl-6-aza-bicyclo[3.2.1]octane-6-carbonyl)-phenyl]-benzamide |
| 7-7 | 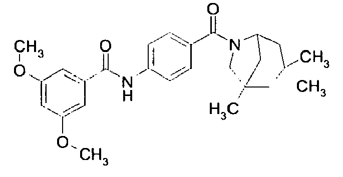 | 436.56 | 3,5-Dimethoxy-N-[4-(1,3,3-trimethyl-6-aza-bicyclo[3.2.1]octane-6-carbonyl)-phenyl]-benzamide |
| 7-8 | 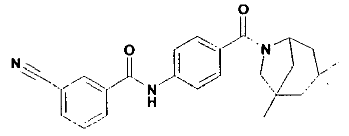 | 401.51 | 3-Cyano-N-[4-(1,3,3-trimethyl-6-aza-bicyclo[3.2.1]octane-6-carbonyl)-phenyl]-benzamide |
| 7-9 | 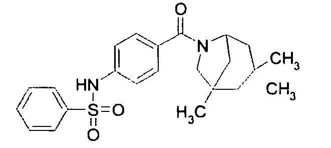 | 412.56 | N-[4-(1,3,3-Trimethyl-6-aza-bicyclo[3.2.1]octane-6-carbonyl)-phenyl]-benzenesulfonamide |
| 7-10 | 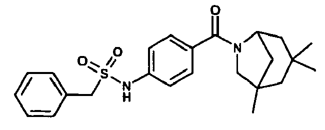 | 426.58 | Phenyl-N-[4-(1,3,3-trimethyl-6-aza-bicyclo[3.2.1]octane-6-carbonyl)-phenyl]-methanesulfonamide |
| 7-11 | 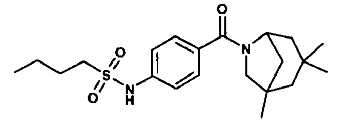 | 392.57 | Butane-1-sulfonic acid [4-(1,3,3-trimethyl-6-aza-bicyclo[3.2.1]octane-6-carbonyl)-phenyl]-amide |
| 7-12 | 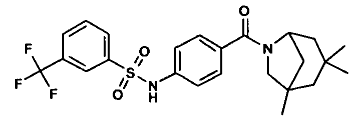 | 480.55 | 3-Trifluoromethyl-N-[4-(1,3,3-trimethyl-6-aza-bicyclo[3.2.1]octane-6-carbonyl)-phenyl]-benzenesulfonamide |
| 7-13 | 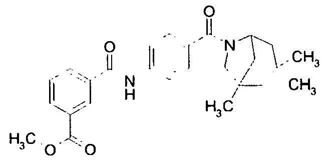 | 434.54 | N-[4-(1,3,3-Trimethyl-6-aza-bicyclo[3.2.1]octane-6-carbonyl)-phenyl]-isophthalamic acid methyl ester |
| 7-14* | 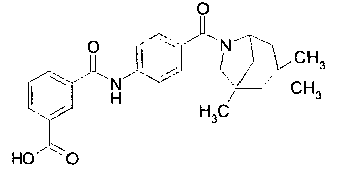 | 420.51 | N-[4-(1,3,3-Trimethyl-6-aza-bicyclo[3.2.1]octane-6-carbonyl)-phenyl]-isophthalamic acid |
| 7-15 | 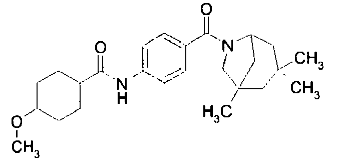 | 412.58 | 4-Methoxy-cyclo-hexanecarboxylic acid [4-(1,3,3-trimethyl-6-aza-bicyclo[3.2.1]octane-6-carbonyl)-phenyl]-amide |
| 7-16 | 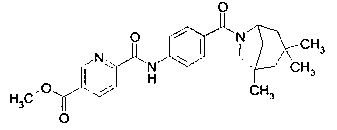 | 435.53 | 6-[4-(1,3,3-Trimethyl-6-aza-bicyclo[3.2.1]octane-6-carbonyl)-phenylcarbamoyl]-nicotinic acid methyl ester |
| 7-17* | 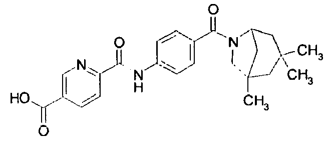 | 421.5 | 6-[4-(1,3,3-Trimethyl-6-aza-bicyclo[3.2.1]octane-6-carbonyl)-phenylcarbamoyl]-nicotinic acid |
| 7-18 | 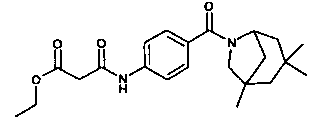 | 386.5 | N-[4-(1,3,3-Trimethyl-6-aza-bicyclo[3.2.1]octane-6-carbonyl)-phenyl]-malonamic acid ethyl ester |
| 7-19 | 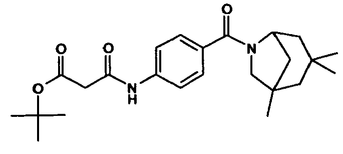 | 414.55 | N-[4-(1,3,3-Trimethyl-6-aza-bicyclo[3.2.1]octane-6-carbonyl)-phenyl]-malonamic acid *tert*-butyl ester |
| 7-20 | 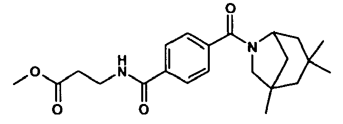 | 386.5 | 3-[4-(1,3,3-Trimethyl-6-aza-bicyclo[3.2.1]octane-6-carbonyl)-benzoylamino]-propionic acid methyl ester |
| 7-21* | 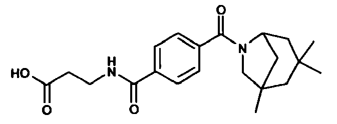 | 372.47 | 3-[4-(1,3,3-Trimethyl-6-aza-bicyclo[3.2.1]octane-6-carbonyl)-benzoylamino]-propionic acid |
| 7-22 | 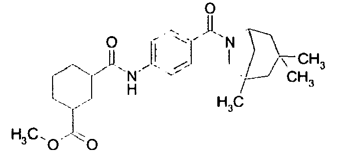 | 440.59 | 3-[4-(1,3,3-Trimethyl-6-aza-bicyclo[3.2.1]octane-6-carbonyl)-phenylcarbamoyl]-cyclohexanecarboxylic acid methyl ester |
| 7-23* | 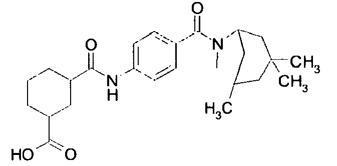 | 426.56 | 3-[4-(1,3,3-Trimethyl-6-aza-bicyclo[3.2.1]octane-6-carbonyl)-phenylcarbamoyl]-cyclohexanecarboxylic acid |
| 7-24 | 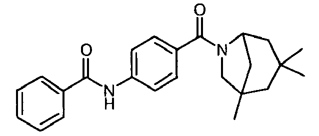 | 376.50 | N-[4-(1,3,3-Trimethyl-6-aza-bicyclo[3.2.1]octane-6-carbonyl)-phenyl]-benzamide |
| 7-25 | 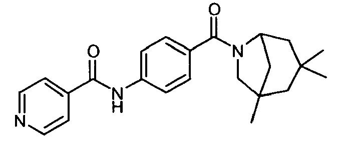 | 377.49 | N-[4-(1,3,3-Trimethyl-6-aza-bicyclo[3.2.1]octane-6-carbonyl)-phenyl]-isonicotinamide |
| 7-26 | 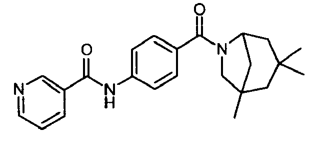 | 377.49 | N-[4-(1,3,3-Trimethyl-6-aza-bicyclo[3.2.1]octane-6-carbonyl)-phenyl]-nicotinamide |
| 7-27 | 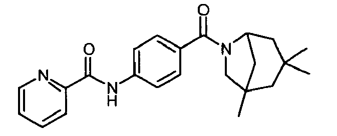 | 377.49 | Pyridine-2-carboxylic acid [4-(1,3,3-trimethyl-6-aza-bicyclo[3.2.1]octane-6-carbonyl)-phenyl]-amide |
| 7-28 | 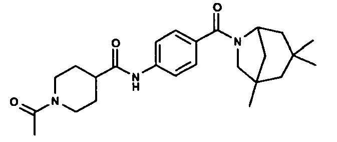 | 425.58 | 1-Acetyl-piperidine-4-carboxylic acid [4-(1,3,3-trimethyl-6-aza-bicyclo[3.2.1]octane-6-carbonyl)-phenyl]-amide |
| 7-29 | 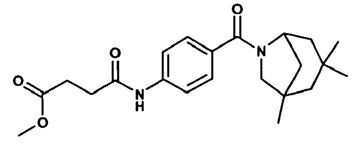 | 386.5 | N-[4-(1,3,3-Trimethyl-6-aza-bicyclo[3.2.1]octane-6-carbonyl)-phenyl]-succinamic acid methyl ester |
| 7-30* | 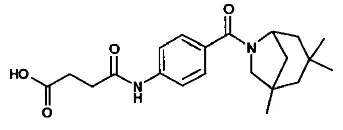 | 372.47 | N-[4-(1,3,3-Trimethyl-6-aza-bicyclo[3.2.1]octane-6-carbonyl)-phenyl]-succinamic acid |
| 7-31* | 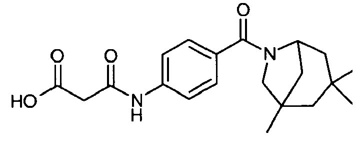 | 358.44 | N-[4-(1,3,3-Trimethyl-6-aza-bicyclo[3.2.1]octane-6-carbonyl)-phenyl]-malonamic acid |
| 7-32 | 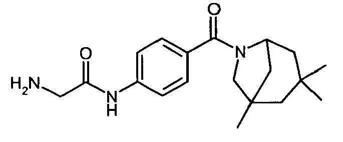 | 329.45 | 2-Amino-N-[4-(1,3,3-trimethyl-6-aza-bicyclo[3.2.1]octane-6-carbonyl)-phenyl]-acetamide |
| 7-33 | 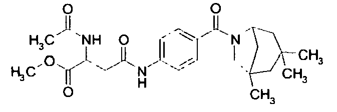 | 443.55 | 2-Acetylamino-N-[4-(1,3,3-trimethyl-6-aza-bicyclo[3.2.1]octane-6-carbonyl)-phenyl]-succinamic acid methyl ester |
| 7-34* | 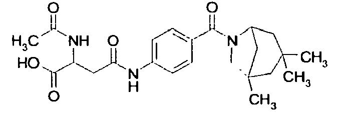 | 429.52 | 2-Acetylamino-N-[4-(1,3,3-trimethyl-6-aza-bicyclo[3.2.1]octane-6-carbonyl)-phenyl]-succinamic acid |
| 7-35 | 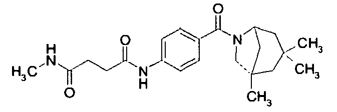 | 385.51 | N-Methyl-N'-[4-(1,3,3-trimethyl-6-aza-bicyclo[3.2.1]octane-6-carbonyl)-phenyl]-succinamide |
| 7-36 | 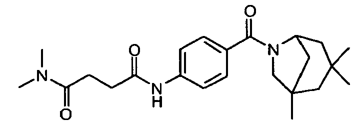 | 399.5 | N,N-Dimethyl-N'-[4-(1,3,3-trimethyl-6-aza-bicyclo[3.2.1]octane-6-carbonyl)-phenyl]-succinamide |
| 7-37 | 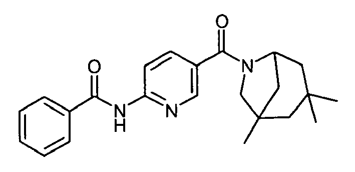 | 377.5 | N-[5-(1,3,3-Trimethyl-6-aza-bicyclo[3.2.1]octane-6-carbonyl)-pyridin-2-yl]-benzamide |

| | | | |
|---|---|---|---|
| * Obtained via alkaline hydrolysis of the corresponding methyl or ethyl ester. | | | |

### NNC 0###-0000-####-Example 8{ TC "Example 8;Bio1: # nM;Bio2: # nM" }

### N-Methyl-N-[4-(1,3,3-trimethyl-6-aza-bicyclo[3.2.1]octane-6-carbonyl)-phenyl]-benzamide

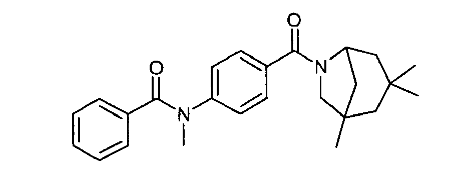

To a solution of N-[4-(1,3,3-trimethyl-6-aza-bicyclo[3.2.1]octane-6-carbonyl)-phenyl]-benzamide (500 mg, 1.33 mmol) in dry THF (40 ml) was added sodium hydride (64 mg, 1.594 mmol in 60% mineral oil) and the mixture was stirred for 30 min. Methyl iodide (99 µl, 1.594 mmol) was added and the stirring was continued for an additional 2 hrs. The volatiles were evaporated in vacuo and the residue was purified by column chromatography (silica gel) using EtOAc-Heptane (1:1) as eluent. Pure fractions were collected and evaporated to dryness affording 300 mg (58 %) of the title compound as a solid.
TLC: EtOAc:Heptane (2:1), R_{f}: 0.27
¹H-NMR (400 MHz, CDCl₃) δ 0.92 (d, 3H), 1.01 (d, 3H), 1.10 (d, 3H), 1.14-1.77 (m, 5.5H), 2.20 (m, 0.5 H), 3.05 (d, 0.5H), 3.15 (d, 0.5H), 3.23 (d, 0.5H), 3.51 (s, 3H), 3.55 (d, 0.5H), 3.85 (m, 0.5H), 4.58 (m, 0.5H), 7.05 (m, 2H), 7.17 (m, 2H), 7.30 (m, 5H).

The following compounds were made in a similar way as described in example 8 above:

| **No** | **Molecule MW** | | **IUPAC Name** |
|---|---|---|---|
| 8-1 | 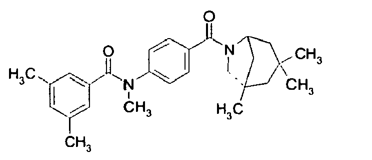 418.58 | | 3,5,N-Trimethyl-N-[4-(1,3,3-trimethyl-6-aza-bicyclo[3,2,1]octane-6-carbonyl)-phenyl)-benzamide |
| 8-2 | 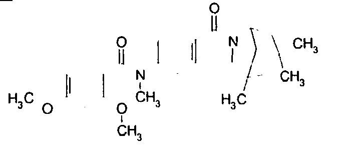 | 450.58 | 2,4-Dimethoxy-N-methyl-N-[4-(1,3,3-trimethyl-6-aza-bicyclo[3.2.1]octane-6-carbonyl)-phenyl]-benzamide |
| 8-3 | 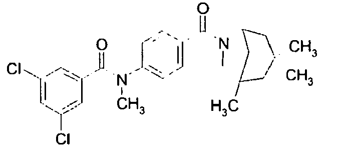 | 459.42 | 3,5-Dichloro-N-methyl-N-[4-(1,3,3-trimethyl-6-aza-bicyclo[3.2.1]octane-6-carbonyl)-phenyl]-benzamide |
| 8-4 | 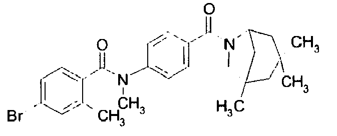 | 483.45 | 4-Bromo-N,N-dimethyl-N-[4-(1,3,3-trimethyl-6-aza-bicyclo-[3.2.1]octane-6-carbonyl)-phenyl]-benzamide |
| 8-5 | 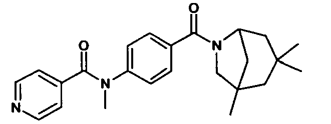 | 391.52 | N-Methyl-N-[4-(1,3,3-trimethyl-6-aza-bicyclo[3.2.1]octane-6-carbonyl)-phenyl]-isonicotinamide |
| 8-6 | 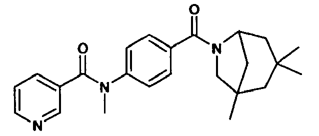 | 391.52 | N-Methyl-N-[4-(1,3,3-trimethyl-6-aza-bicyclo[3.2.1]octane-6-carbonyl)-phenyl]-nicotinamide |

### NNC 0###-0000-####-Example 9{ TC "Example 9;Bio1: # nM;Bio2: # nM" }

### 2-Piperidin-1-yl-N-[4-(1,3,3-trimethyl-6-aza-bicyclo[3.2.1]octane-6-carbonyl)-phenyl]-acetamide

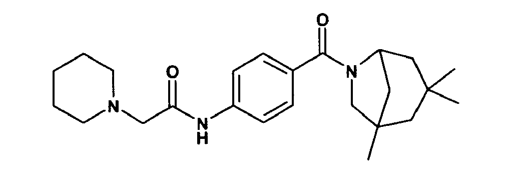

To a solution of (4-amino-phenyl)-(1,3,3-trimethyl-6-aza-bicyclo[3.2.1]oct-6-yl)-methanone (3.0 g, 11.01 mmol) and TEA (3.1 ml, 22.03 mmol) in dry THF (100 ml) was added dropwise chloroacetyl chloride (1.05 ml, 13.22 mmol). The mixture was stirred for 75 min. and evaporated followed by addition of water 75 (ml). The aqueous phase was extracted with EtOAc (2 x 50 ml) and the combined organic phases were dried (Na₂SO₄), filtered and evaporated in vacuo. The resulting residue was dissolved in EtOAc (40 ml) and filtered through a 2.5 cm path of silicagel. The filtrated was evaporated to dryness affording 3.3 g (86 %) of 2-chloro-N-[4-(1,3,3-trimethyl-6-aza-bicyclo[3.2.1]octane-6-carbonyl)-phenyl]-acetamide as an oil.
¹H-NMR (400 MHz, CDCl₃) δ 0.93 (d, 3H), 1.03 (d, 3H), 1.13 (d, 3H), 1.17 - 1.79 (m, 5.5H), 2.23 (m, 0.5H), 3.08 - 3.29 (m, 1.5H), 3.59 (d, 0.5H), 4.0 and 4.59 (2xm, 1H), 4.19 (s, 2H), 7.44 (m, 2H), 7.57 (t, 2H), 8.55 (s, 1H).

A solution of 2-chloro-N-[4-(1,3,3-trimethyl-6-aza-bicyclo[3.2.1]octane-6-carbonyl)-phenyl]-acetamide (40 mg, 0.115 mmol) in acetone (250 µl) was added to a mixture of piperidine (17.1 µl, 0.173 mmol) and DIPEA (40 µl, 0.229 mmol) in MeCN (250 µl). The resulting reaction mixture was heated and stirred in a microwave oven for 10 min. at 100 °C. After cooling to room temperature the volatiles were removed in vacuo and the residue purified on a prep. Gilson HPLC. Pure fractions were collected, evaporated in vauco and dried at 50 °C for 16 hours affording 43.8 mg (75 %) of the title compounds as a TFA salt.
LC/MS m/z: 398 H⁺
¹H-NMR (400 MHz, CDCl₃) δ 0.93 (d, 3H), 1.03 (d, 3H), 1.13 (d, 3H), 1.17 - 1.79 (m, 5.5H), 2.23 (m, 0.5H), 3.08 - 3.29 (m, 1.5H), 3.59 (d, 0.5H), 4.0 and 4.59 (2xm, 1H), 4.19 (s, 2H), 7.44 (m, 2H), 7.57 (t, 2H), 8.55 (s, 1H).

The following compounds were made in a similar way as described in example 9 above:

| **No** | **Molecule** | **MW** | **IUPAC Name** |
|---|---|---|---|
| 9-1 | 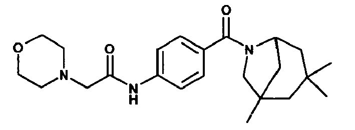 | 399.54 | 2-Morpholin-4-yl-N-[4-(1,3,3-trimethyl-6-aza-bicyclo[3.2.1]octane-6-carbonyl)-phenyl]-acetamide |
| 9-2 | 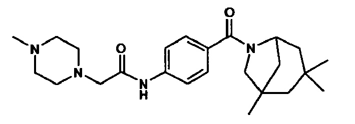 | 412.58 | 2-(4-Methyl-piperazin-1-yl)-N-[4-(1,3,3-trimethyl-6-aza-bicyclo[3.2.1]octane-6-carbonyl)-phenyl]-acetamide |
| 9-3 | 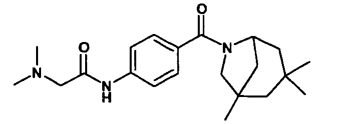 | 357.5 | 2-Dimethylamino-N-[4-(1,3,3-trimethyl-6-aza-bicyclo[3.2.1]octane-6-carbonyl)-phenyl]-acetamide |
| 9-4 | 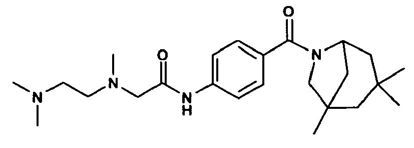 | 414.6 | 2-[(2-Dimethylamino-ethyl)-methyl-amino]-N-[4-(1,3,3-trimethyl-6-aza-bicyclo[3.2.1]octane-6-carbonyl)-phenyl]-acetamide |
| 9-5 | 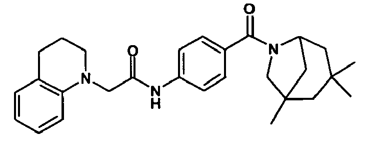 | 445.61 | 2-(3,4-Dihydro-2*H*-quinolin-1-yl)-N-[4-(1,3,3-trimethyl-6-aza-bicyclo[3.2.1]octane-6-carbonyl)-phenyl]-acetamide |
| 9-6 | 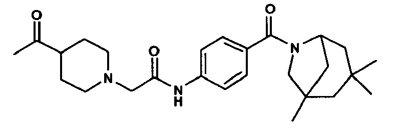 | 439.60 | 2-(4-Acetyl-piperidin-1-yl)-N-[4-(1,3,3-trimethyl-6-aza-bicyclo[3.2.1]octane-6-carbonyl)-phenyl]-acetamide |
| 9-7 | 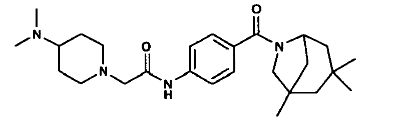 | 440.63 | 2-(4-Dimethylamino-piperidin-1-yl)-N-[4-(1,3,3-trimethyl-6-aza-bicyclo[3.2.1]octane-6-carbonyl)-phenyl]-acetamide |
| 9-8 | 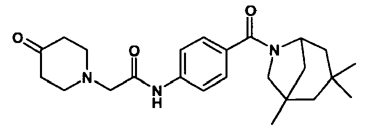 | 411.55 | 2-(4-Oxo-piperidin-1-yl)-N-[4-(1,3,3-trimethyl-6-aza-bicyclo[3.2.1]octane-6-carbonyl)-phenyl]-acetamide |
| 9-9 | 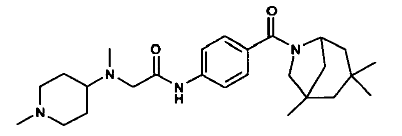 | 440.63 | 2-[Methyl-(1-methyl-piperidin-4-yl)-amino]-N-[4-(1,3,3-trimethyl-6-aza-bicyclo[3.2.1]octane-6-carbonyl)-phenyl]-acetamide |
| 9-10 | 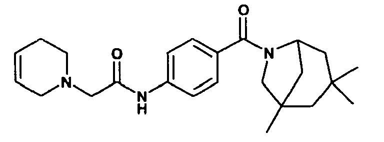 | 395.55 | 2-(3,6-Dihydro-2H-pyridin-1-yl)-N-[4-(1,3,3-trimethyl-6-aza-bicyclo[3.2.1]octane-6-carbonyl)-phenyl]-acetamide |
| 9-11 | 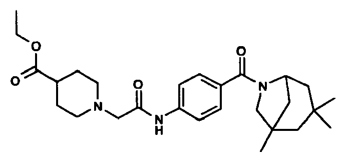 | 469.63 | 1-{[4-(1,3,3-Trimethyl-6-aza-bicyclo[3.2.1]octane-6-carbonyl)-phenylcarbamoyl]-methyl}-piperidine-4-carboxylic acid ethyl ester |
| 9-12 | 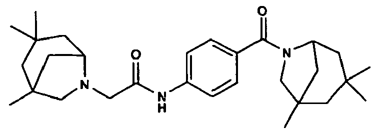 | 465.69 | N-[4-(1,3,3-Trimethyl-6-aza-bicyclo[3.2.1]octane-6-carbonyl)-phenyl]-2-(1,3,3-trimethyl-6-aza-bicyclo[3.2.1]oct-6-yl)acetamide |
| 9-13 | 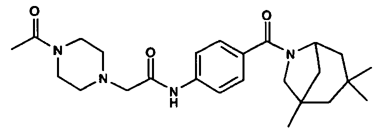 | 440.59 | 2-(4-Acetyl-piperazin-1-yl)-N-[4-(1,3,3-trimethyl-6-aza-bicyclo[3.2.1]octane-6-carbonyl)-phenyl]-acetamide |
| 9-14 | 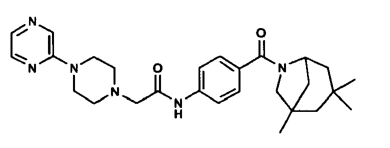 | 476.63 | 2-(2,3,5,6-Tetrahydro-[1,2']bipyrazinyl-4-yl)-N-[4-(1,3,3-trimethyl-6-aza-bicyclo[3.2.1]octane-6-carbonyl)-phenyl]-acetamide |
| 9-15 | 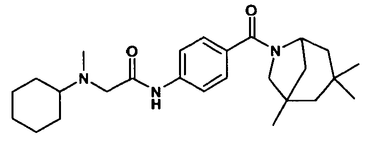 | 425.62 | 2-(Cyclohexyl-methyl-amino)-N-[4-(1,3,3-trimethyl-6-aza-bicyclo[3.2.1]octane-6-carbonyl)-phenyl]-acetamide |
| 9-16 | 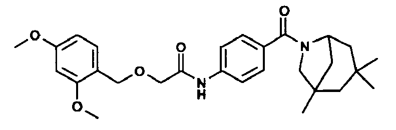 | 480.61 | 2-(2,4-Dimethoxy-benzyloxy)-N-[4-(1,3,3-trimethyl-6-aza-bicyclo[3.2.1]octane-6-carbonyl)-phenyl]-acetamide |
| 9-17 | 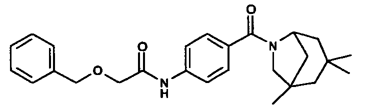 | 420.56 | 2-Benzyloxy-N-[4-(1,3,3-trimethyl-6-aza-bicyclo[3.2.1]octane-6-carbonyl)-phenyl]-acetamide |
| 9-18 | 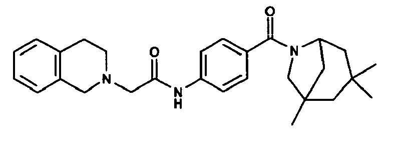 | 445.61 | 2-(3,4-Dihydro-1H-isoquinolin-2-yl)-N-[4-(1,3,3-trimethyl-6-aza-bicyclo[3.2.1]octane-6-carbonyl)-phenyl]-acetamide |
| 9-19 | 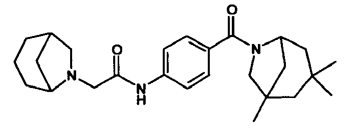 | 423.60 | 2-(6-Aza-bicyclo[3.2.1]oct-6-yl)-N-[4-(1,3,3-trimethyl-6-aza-bicyclo[3.2.1]octane-6-carbonyl)-phenyl]-acetamide |
| 9-20 | 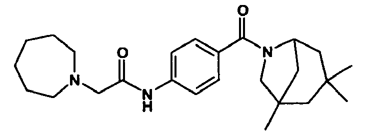 | 411.59 | 2-Azepan-1-yl-N-[4-(1,3,3-trimethyl-6-aza-bicyclo[3.2.1]octane-6-carbonyl)- phenyl]-acetamide |
| 9-21 | 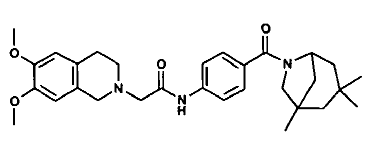 | 505.66 | 2-(6,7-Dimethoxy-3,4-dihydro-1*H*- isoquinolin-2-yl)-N-[4-(1,3,3-trimethyl-6-aza-bicyclo[3.2.1]octane-6-carbonyl)-phenyl]-acetamide |
| 9-22 | 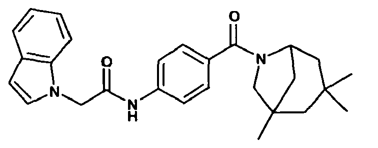 | 429.57 | 2-Indol-1-yl-N-[4-(1,3,3-trimethyl-6-aza-bicyclo[3.2.1]octane-6-carbonyl)-phenyl]-acetamide |
| 9-23 | 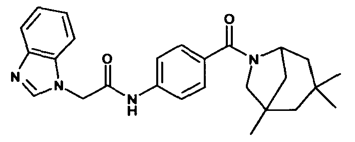 | 430.56 | 2-Benzoimidazol-1-yl-N-[4-(1,3,3-trimethyl-6-aza-bicyclo[3.2.1]octane-6-carbonyl)-phenyl]-acetamide |
| 9-24 | 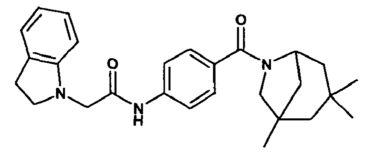 | 431.58 | 2-(2,3-Dihydro-indol-1-yl)-N-[4-(1,3,3-trimethyl-6-aza-bicyclo[3.2.1]octane-6-carbonyl)-phenyl]-acetamide |
| 9-25 | 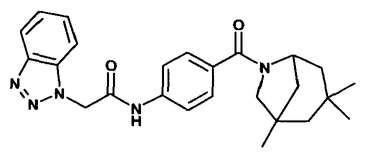 | 431.54 | 2-Benzotriazol-1-yl-N-[4-(1,3,3-trimethyl-6-aza-bicyclo[3.2.1]octane-6-carbonyl)-phenyl]-acetamide |
| 9-26 | 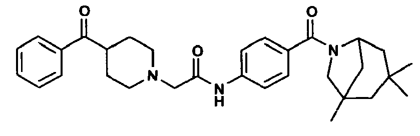 | 501.7 | 2-(4-Benzoyl-piperidin-1-yl)-N-[4-(1,3,3-trimethyl-6-aza-bicyclo[3.2.1]octane-6-carbonyl)-phenyl]-acetamide |
| 9-27 | 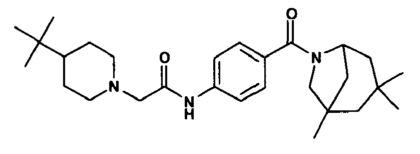 | 453.7 | 2-(4-tert-Butyl-piperidin-1-yl)-N-[4-(1,3,3-trimethyl-6-aza-bicyclo[3.2.1]octane-6-carbonyl)-phenyl]-acetamide |
| 9-28 | 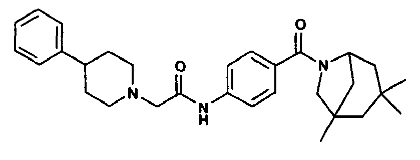 | 473.7 | 2-(4-Phenyl-piperidin-1-yl)-N-[4-(1,3,3-trimethyl-6-aza-bicyclo[3.2.1]octane-6-carbonyl)- phenyl]-acetamide |
| 9-29 | 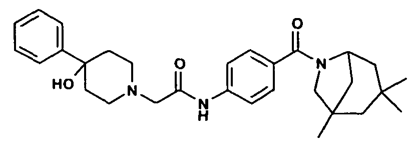 | 489.7 | 2-(4-Hydroxy-4-phenyl-piperidin-1- yl)-N-[4-(1,3,3-trimethyl-6-aza-bicyclo[3.2.1]octane-6-carbonyl)-phenyl]-acetamide |
| 9-30 | 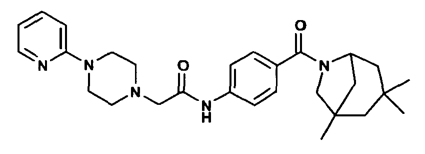 | 475.6 | 2-(4-Pyridin-2-yl-piperazin-1-yl)-N-[4-(1,3,3-trimethyl-6-aza-bicyclo[3.2.1]octane-6-carbonyl)-phenyl]-acetamide |
| 9-31 | 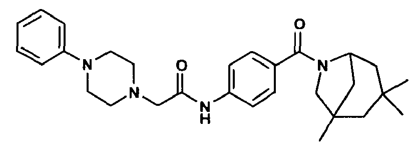 | 474.7 | 2-(4-Phenyl-piperazin-1-yl)-N-[4-(1,3,3-trimethyl-6-aza-bicyclo[3.2.1]octane-6-carbonyl)-phenyl]-acetamide |
| 9-32 | 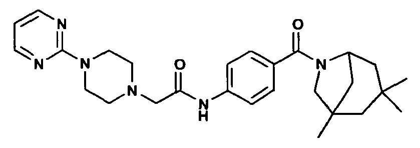 | 476.6 | 2-(4-Pyrimidin-2-yl-piperazin-1-yl)-N-[4-(1,3,3-trimethyl-6-aza-bicyclo[3.2.1]octane-6-carbonyl)-phenyl]-acetamide |
| 9-33 | 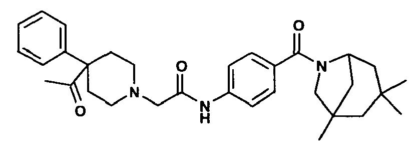 | 515.7 | 2-(4-Acetyl-4-phenyl-piperidin-1-yl)-N-[4-(1,3,3-trimethyl-6-aza-bicyclo[3.2.1]octane-6-carbonyl)-phenyl]-acetamide |
| 9-34 | 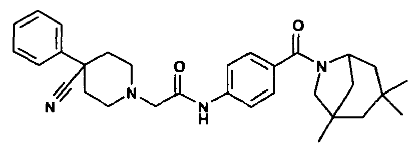 | 498.7 | 2-(4-Cyano-4-phenyl-piperidin-1-yl)-N-[4-(1,3,3-trimethyl-6-aza-bicyclo[3.2.1]octane-6-carbonyl)-phenyl]-acetamide |
| 9-35 | 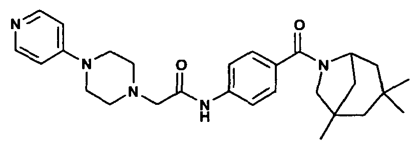 | 475.6 | 2-(4-Pyridin-4-yl-piperazin-1-yl)-N-[4-(1,3,3-trimethyl-6-aza-bicyclo[3.2.1]octane-6-carbonyl)-phenyl]-acetamide |
| 9-36 | 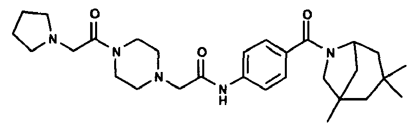 | 509.7 | 2-[4-(2-Pyrrolidin-1-yl-acetyl)-piperazin-1-yl]-N-[4-(1,3,3-trimethyl-6-aza-bicyclo[3.2.1]octane-6-carbonyl)-phenyl]-acetamide |
| 9-37 | 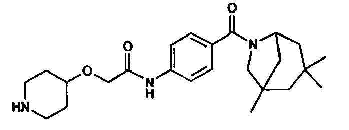 | 413.6 | 2-(Piperidin-4-yloxy)-N-[4-(1,3,3-trimethyl-6-aza-bicyclo[3.2.1]octane-6-carbonyl)-phenyl]-acetamide |

### NNC 0###-0000-####-Example 10{ TC "Example 10;Bio1: # nM;Bio2: # nM" }

### 2-(2H-Tetrazol-5-yl)-N-[4-(1,3,3-trimethyl-6-aza-bicyclo[3.2.1]octane-6-carbonyl)-phenyl]-acetamide

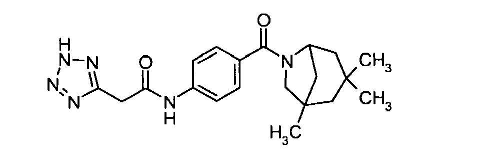

To a mixture of (2*H*-tetrazol-5-yl)-acetic acid ethyl ester (2.0 g, 12.81 mmol), trityl chloride (3.7 g, 13.45 mmol) in dry THF (75 ml) was added TEA (3.7 ml). The resulting mixture was stirred for 18 hrs. at room temperature. Water (10 ml) was added and the precipitate filtered off, redissolved in EtOAc (25 ml) and washed with saturated aqueous ammonium chloride (2 x 15 ml). The organic phase was dried (Na₂SO₄), filtered and evaporated in vacuo. The residue was suspended in ethanol (150 ml) and potassium hydroxide (0.8 g, 13.45 mmol) dissolved in ethanol (100 ml) was added. The resulting mixture was stirred for 18 hrs. at room temperature. The precipitate was filtered off and dissolved in water (150 ml) and acidified with conc. HCl (use diluted HCl). The precipitate was filtered off and washed with diethyl ether and dried in vacuo at 50 °C affording 0.7 g (15 %) of (2-trityl-2*H*-tetrazol-5-yl)-acetic acid as a solid.
¹H-NMR (400 MHz, CDCl₃) δ 4.07 (s, 2H), 7.10 (d, 6H), 7.34 (m, 9H).

To a solution of (2-trityl-2*H*-tetrazol-5-yl)-acetic acid (0.82 g, 2.20 mmol) in dry THF (50 ml) was added CDI (0.4 g, 2.39 mmol) and the resulting mixture was stirred for 10 min. To this mixture was added (4-amino-phenyl)-(1,3,3-trimethyl-6-aza-bicyclo[3.2.1]oct-6-yl)-methanone (0.5 g, 1.84 mmol) and the stirring was continued for 18 hrs. at room temperature. The mixture was evaporated and the residue purified by column chromatography (silica gel) using EtOAc-Heptane (4:1) as eluent. Pure fractions were collected and evaporated to dryness affording 150 mg (13 %) of *N*-[4-(1,3,3-trimethyl-6-aza-bicyclo[3.2.1]octane-6-carbonyl)-phenyl]-2-(2-trityl-2*H*-tetrazol-5-yl-acetamide which was dissolved in THF (25 ml) followed by addition of 20 % aqueous HCl (20 ml). The mixture was stirred for 2 hrs. at room temperature at which time the volatiles were removed in vacuo. The aqueous residue was extracted with EtOAc (2 x 50 ml), dried (Na₂SO₄), filtered and evaporated in vacuo affording 30 mg (4 %) of the title compound as a solid.
LC/MS: m/z: 383 H⁺
¹H-NMR (400 MHz, CDCl₃) δ 0.92 (d, 3H), 1.05 (s, 3H), 1.14 (d, 3H), 1.17 - 1.84 (m, 5.5H), 2.22 (m, 0.5H), 3.24 - 3.27 (m, 1.5H), 3.66 (d, 0.5H), 4.01 (bs, 2.5H), 4.61 (m, 0.5H), 7.24 (m, 2H), 7.47 (m, 2H), 9.68 (bs, 1H).

### NNC 0###-0000-####-Example 11{ TC "Example 11;Bio1: # nM;Bio2: # nM" }

### 3-Piperidin-1-yl-N-[4-(1,3,3-trimethyl-6-aza-bicyclo[3.2.1]octane-6-carbonyl)phenyl]-propionamide

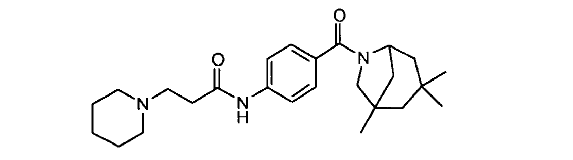

To a solution of (4-amino-phenyl)-(1,3,3-trimethyl-6-aza-bicyclo[3.2.1]oct-6-yl)-methanone (3.0 g, 11.01 mmol) and TEA (3.1 ml, 22.03 mmol) in dry THF (40 ml) was added dropwise acryloyl chloride (1.07 ml, 13.22 mmol). The mixture was stirred for 45 min., the volatiles evaporated and to the residue added water 25 (ml). The aqueous phase was extracted with EtOAc (2 x 25 ml) and the combined organic phases were dried (Na₂SO₄), filtered and evaporated in vacuo. The residue was purified on column chromatography (silicagel) using EtOAc-Heptane 1:1 as eluent. Pure fractions were collected, the solvent evaporated in vacuo affording 1.7 g (47 %) of N-[4-(1,3,3-trimethyl-6-aza-bicyclo[3.2.1]octane-6-carbonyl)-phenyl]-acrylamide as an oil.
¹H-NMR (400 MHz, CDCl₃) δ 0.93 (d, 3H), 1.03 (d, 3H), 1.12 (d, 3H), 1.17 - 1.76 (m, 5.5H), 2.24 (m, 0.5H), 3.18 (d, 0.5H), 3.27 (d, 1H), 3.59 (d, 0.5 H), 4.01 and 4.59 (2xm, 1H), 5.76 (d, 1H), 6.31 (dd, 2H), 6.44 (d, 1H), 7.36 (dd, 2H), 7.54 (d, 2H), 8.13 (m, 1H).

To a mixture of N-[4-(1,3,3-trimethyl-6-aza-bicyclo[3.2.1]octane-6-carbonyl)-phenyl]-acrylamide (0.2 g, 0.613 mmol) and piperidine (120 µl, 1.23 mmol) in EtOH (10 ml) was added two drops of TEA. The resulting mixture was heated in a microwave oven at 100 °C for 25 min. The volatiles were evaporated in vacuo and the residue purified on column chromatography (silicagel) using first EtOAc followed by 4% TEA in EtOAc-EtOH 9:1 as eluents. Semi pure fractions were collected, the solvent evaporated in vacuo and the residue dissolved in 1 N HCl (2.5 ml). Water (5 ml) was added followed by diethyl ether (10 ml) and the mixture was stirred for 30 min. The organic phase was discarded and the pH of the aqueous phase adjusted to 10 with 1 N NaOH followed by extraction with diethyl ether (2x10 ml). The combined organic phases were dried (Na2SO4), filtered and evaporated in vacuo affording 55 mg (22 %) of the title compound as an oil.
LC/MS: m/z: 412 H⁺
¹H-NMR (400 MHz, CDCl₃) δ 0.93 (d, 3H), 1.02 (d, 3H), 1.13 (d, 3H), 1.17 - 1.79 (m, 11H), 1.93 (bs, 0.5H), 2.24 (m, 0.5H), 2.47 - 2.68 (m, 8H), 3.20 (d, 0.5H), 3.28 (d, 1H), 3.57 (d, 0.5H), 4.03 and 4.60 (2xm, 1H), 7.42 (dd, 2H), 7.57 (t, 2H), 11.57 (s, 1H).

The following compound was made in a similar way as described in example 11 above:

| **No** | **Molecule** | **MW** | **IUPAC Name** |
|---|---|---|---|
| 11-1 | 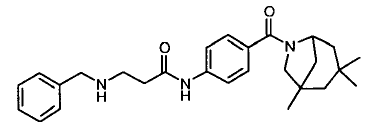 | 433.6 | 3-Benzylamino-N-[4-(1,3,3-trimethyl-6-aza-bicyclo[3.2.1]octane-6-carbonyl)-phenyl]-propionamide |

### NNC 0###-0000-####-Example 12{ TC "Example 12;Bio1: # nM;Bio2: # nM"}

### 2-(3,4-Dihydro-2H-quinolin-1-yl)-N-methyl-N-[4-(1,3,3-trimethyl-6-aza-bicyclo[3.2.1]octane-6-carbonyl)-phenyl]-acetamide

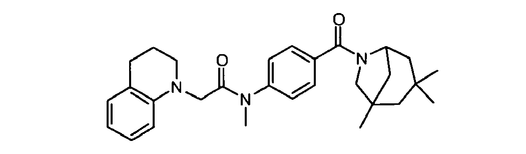

To a mixture of (4-methylamino-phenyl)-(1,3,3-trimethyl-6-aza-bicyclo[3.2.1]oct-6-yl)-methanone (0.6 g, 2.1 mmol) and TEA (0.6 ml, 4.19 mmol) in dry THF (40 ml) was added dropwise chloroacetyl chloride (0.25 ml, 3.14 mmol). The mixture was stirred for 75 min. and evaporated in vacuo followed by addition of water 75 (ml). The aqueous phase was extracted with EtOAc (2 x 25 ml) and the combined organic phases were dried (Na₂SO₄), filtered and evaporated in vacuo. The residue was purified on column chromatography (silicagel) using EtOAc-Heptane 1:1 as eluent. Pure fractions were collected; the solvent evaporated in vacuo affording 400 mg (53 %) of 2-chloro-N-methyl-N-[4-(1,3,3-trimethyl-6-aza-bicyclo[3.2.1]octane-6-carbonyl)-phenyl]-acetamide as a solid.
TLC: EtOAc-Heptane (3:1), R_{f}: 0.24
¹H-NMR (400 MHz, CDCl₃) δ 0.96 (d, 3H), 1.06 (d, 3H), 1.14 (s, 3H), 1.20 - 1.82 (m, 5.5H), 2.24 (m, 0.5H), 3.08 - 3.28 (m, 1.5H), 3.33 (s, 3H), 3.61 (d, 0.5 H), 3.86 (s, 2H), 4.0 and 4.62 (2xm, 1H), 7.31 (dd, 2H), 7.57 (dd, 2H).

To a mixture of 2-chloro-*N*-methyl-*N*-[4-(1,3,3-trimethyl-6-aza-bicyclo[3.2.1]octane-6-carbonyl)-phenyl]-acetamide (0.1 g, 0.276 mmol) and 1,2,3,4-tetrahydro-quinoline (55 mg, 0.413 mmol) in MeCN (5 ml) was added DIPEA (0.1 ml, 0.551 mmol). The resulting mixture was heated in a microwave oven at 100 °C for 10 min. The volatiles were evaporated in vacuo and the residue purified on column chromatography (silicagel) using EtOAc-Heptane 2:1 as eluent. Pure fractions were collected; the solvent evaporated in vacuo 50 mg (39 %) of the title compound as an oil.
TLC: EtOAc, R_{f}: 0.42
LC/MS: m/z: 460 H⁺
¹H-NMR (400 MHz, CDCl₃) δ 0.95 (d, 3H), 1.05 (d, 3H), 1.14 (d, 3H), 1.21 - 1.88 (m, 7.5H), 2.25 (m, 0.5H), 2.71 (bs, 2H), 3.15 - 3.30 (m, 6H), 3.61 (d, 1H), 3.80 (s, 2H), 3.95 (m, 0.5H), 4.61 (m, 0.5H), 6.26 (d, 1H), 6.57 (t, 1H), 6.91 (d, 1H), 6.98 (t, 1H), 7.30 (t, 2H), 7.53 (m, 2H).

### NNC 0###-0000-####-Example 13{TC "Example 13;Bio1: # nM;Bio2: # nM" }

### 1-Morpholin-4-yl-2-[4-(1,3,3-trimethyl-6-aza-bicyclo[3.2.1]octane-6-carbonyl)-phenylamino]-ethanone

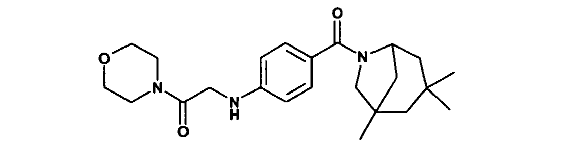

To a solution of [4-(1,3,3-trimethyl-6-aza-bicyclo[3.2.1]octane-6-carbonyl)-phenyl]carbamic acid *tert*-butyl ester (0.5 g, 1.34 mmol) in dry DMF (40 ml) was added sodium hydride (80 mg, 3.33 mmol in 60% mineral oil) and the mixture was stirred for 30 min. 2-Chloro-1-morpholin-4-yl-ethanone (264 mg, 1.61 mmol) was added and the stirring was continued for an additional 1 hr. at 50 °C. The reaction was quenched by addition of water (50 ml) and extracted with diethyl ether (2 x 25 ml). The combined organic phases were dried (Na₂SO₄), filtered and evaporated in vacuo. The residue was dissolved in DCM (20 ml) and TFA (10 ml) was added. The resulting mixture was stirred for 4 hrs. at room temperature and evaporated in vacuo. The residue was purified by column chromatography (silica gel) using first EtOAc (500 ml) followed by 4% TEA in EtOAc as eluents. Semi-pure fractions were collected and evaporated to dryness affording 0.4 g as an oil which was dissolved in EtOAc (25 ml) and washed with water (2 x 25 ml), dried (Na₂SO₄), filtered and evaporated in vacuo affording 180 mg (34%) of the title compound as a oil.
TLC: (EtOAc, 4% TEA), R_{f}: 0.22
¹H-NMR (400 MHz, CDCl₃) δ 0.94 (s, 3H), 1.02 (d, 3H), 1.11 (d, 3H), 1.21 - 1.75 (m, 5.5H), 2.25 (d, 0.5H), 3.27 (t, 1H), 3.35 (d, 0.5H), 3.47 - 3.55 (m, 2.5H), 3.71 (m, 6H), 3.89 (s, 2H), 4.11 (m, 0.5H), 4.57 (m, 0.5H), 6.58 (t, 2H), 7.37 (dd, 2H).

The following compound was made in a similar way as described in example 13 above:

| **No** | **Molecule** | **MW** | **IUPAC Name** |
|---|---|---|---|
| 13-1 | 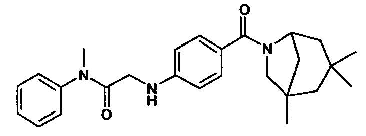 | 419.6 | N-Methyl-N-phenyl-2-[4-(1,3,3-trimethyl-6-aza-bicyclo[3.2.1]octane-6-carbonyl)-phenylamino]-acetamide |
| 13-2 | 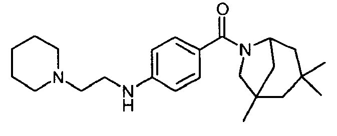 | 383.6 | [4-(2-Piperidin-1-yl-ethylamino)-phenyl]-(1,3,3-trimethyl-6-aza-bicyclo[3.2.1]oct-6-yl)methanone |

### NNC 0###-0000-####-Example 14{ TC "Example 14;Bio1: # nM;Bio2: # nM" }

### 4-(2-Methoxy-ethylamino)-phenyl]-(1,3,3-trimethyl-6-aza-bicyclo[3.2.1]oct-6-yl)-methanone

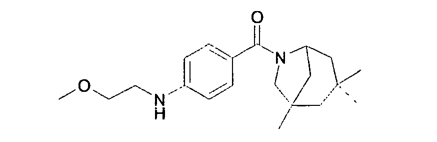

To a solution of [4-(1,3,3-trimethyl-6-aza-bicyclo[3.2.1]octane-6-carbonyl)-phenyl]carbamic acid *tert*-butyl ester (0.5 g, 1.34 mmol) in dry DMF (25 ml) was added sodium hydride (75 mg, 1.88 mmol in 60% mineral oil) and the mixture was stirred for 30 min. 1-Bromo-2-methoxy-ethane (224 mg, 1.61 mmol) was added and the mixture was heated to reflux and allowed to cool to room temperature with stirring. The reaction was quenched by addition of water (20 ml) and extracted with diethyl ether (2 x 25 ml). The combined organic phases were washed with saturated aqueous ammonium chloride (25 ml), dried (Na₂SO₄), filtered and evaporated in vacuo. The residue was dissolved in DCM (15 ml) and TFA (10 ml) was added. The resulting mixture was stirred for 5 hrs. at room temperature and evaporated in vacuo. To the residue was added water (10 ml) and diethyl ether (20 ml) and the pH was adjusted to 9 by addition of 1 N sodium hydroxide. The organic phase was separated and dried (Na₂SO₄), filtered and evaporated in vacuo. The residue was purified by column chromatography (silica gel) using EtOAc/heptane (1:1) as eluent. Pure fractions were collected and evaporated to dryness affording 0.3 g (68 %) of the title compound as an oil.
TLC: (EtOAc), R_{f}: 0.46
¹H-NMR (400 MHz, CDCl₃) δ 0.93 (s, 3H), 1.03 (d, 3H), 1.11 (d, 3H), 1.21 - 1.75 (m, 5.5H), 2.25 (d, 0.5H), 3.30 (m, 3H), 3.39 (s, 3H), 3.52 (d, 0.5H), 3.60 (t, 2H), 3.70 (m, 0.5H), 4.10 (m, 0.5H), 4.58 (m, 0.5H), 6.58 (t, 2H), 7.36 (dd, 2H).

### NNC 0###-0000-####-Example 15{ TC "Example 15;Bio1: # nM;Bio2: # nM" }

### N-(2-Methoxy-ethyl)-N-[4-(1,3,3-trimethyl-6-aza-bicyclo[3.2.1]octane-6-carbonyl)-phenyl]-benzamide

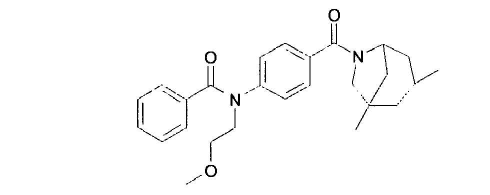

To a mixture of [4-(2-methoxy-ethylamino)-phenyl]-(1,3,3-trimethyl-6-aza-bicyclo[3.2.1]oct-6-yl)-methanone (0.1 g, 0.303 mmol) and TEA (0.13 ml, 0.91 mmol) in dry THF (20 ml) was added dropwise benzoyl chloride (0.07 ml, 0.61 mmol). The mixture was stirred for 1 hr. and evaporated in vacuo. The residue was purified on column chromatography (silicagel) using EtOAc-Heptane (1:1) as eluent. Pure fractions were collected; the solvent evaporated in vacuo affording 90 mg (68 %) of the title compound as an oil.
TLC: (EtOAc), R_{f}: 0.46
¹H-NMR (400 MHz, CDCl₃) δ 0.93 (d, 3H), 1.01 (d, 3H), 1.09 (d, 3H), 1.21- 1.76 (m, 5.5H), 2.19 (d, 0.5H), 3.05 - 3.15 (m, 1H), 3.32 (d, 0.5H), 3.37 (d, 3H), 3.56 (m, 0.5H), 3.67 (t, 2H), 3.84 (m, 0.5H), 4.10 (m, 2H), 4.59 (m, 0.5H), 7.15 (m, 3H), 7.29 (m, 6H).

The following compound was made in a similar way as described in examples 14 and 15 above:

| **No** | **Molecule** | **MW** | **IUPAC Name** |
|---|---|---|---|
| 14-1 | 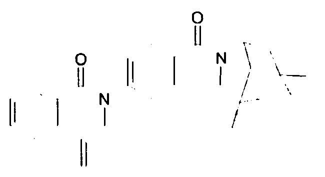 | 416.6 | N-Allyl-N-[4-(1,3,3-trimethyl-6-aza-bicyclo[3.2.1]octane-6-carbonyl)-phenyl]-benzamide |

### NNC 0###-0000-####-Example 16{TC "Example ;Bio1: # nM;Bio2: # nM" }

### (4-Benzyloxy-phenyl)-(1,3,3-trimethyl-6-aza-bicyclo[3.2.1]oct-6-yl)-methanone

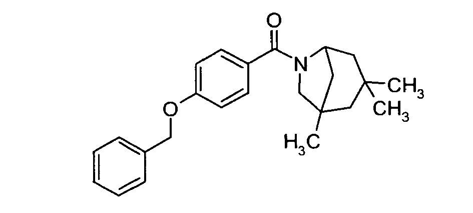

To a mixture of 4-benzyloxy-benzoic acid (0.5 g, 2.191 mmol) and HOBT (326 mg, 2.41 mmol) in dry THF (50 ml) was added EDAC (462 mg, 2.41 mmol). The resulting mixture was stirred for 10 min. followed by addition of a mixture of DIPEA (420 µl, 2.41 mmol) and 1,3,3-trimethyl-6-aza-bicyclo[3.2.1]octane (410 µl, 2.41 mmol). The reaction mixture was stirred for an additional 16 hrs. and evaporated to dryness. To the residue was added water (50 ml) and the resulting mixture was extracted with EtOAc (2 x 50 ml). The combined organic phases were dried (Na₂SO₄), filtered and evaporated in vacuo. The resulting residue was purified by column chromatography (silica gel) using a mixture of EtOAc-Heptane (1:3) as eluent. Pure fractions were collected and evaporated to dryness affording 610 mg (77 %) of the title compound as an oil.
TLC (EtOAc-Heptane (1:1) R_{f}: 0.4
¹H-NMR (400 MHz, CDCl₃) δ 0.93 (d, 3H), 1.02 (d, 3H), 1.12 (d, 3H), 1.30 - 1.5 (m, 4H), 1.59 (m, 1H), 1.74 (m, 0.5H), 2.25 (dd, 0.5H), 3.20 (d, 0.5H), 3.29 (m, 1H), 3.56 (d, 0.5H), 4.03 (m, 0.5H), 4.59 (m, 0.5H), 5.08 (s, 2H), 6.96 (m, 2H), 7.3 - 7.46 (m, 7H).

### PHARMACOLOGICAL METHODS

### 11βHSD1 enzyme assay

### Materials

³H-cortisone and anti-rabbit lg coated scintillation proximity assay (SPA) beads were purchased from Amersham Pharmacia Biotech, β-NADPH was from Sigma and rabbit anti-cortisol antibodies were from Fitzgerald. An extract of yeast transformed with h-11βHSD1 (Hult et al., FEBS Lett, 441, 25 (1998)) was used as the source of enzyme. The test compounds were dissolved in DMSO (10 mM). All dilutions were performed in a buffer containing 50 mM TRIS-HCl (Sigma Chemical Co), 4 mM EDTA (Sigma Chemical Co), 0.1 % BSA (Sigma Chemical Co), 0.01% Tween-20 (Sigma Chemical Co) and 0.005% bacitracin (Novo Nordisk A/S), pH=7.4. Optiplate 96 wells plates were supplied by Packard. The amount of ³H-cortisol bound to the SPA beads was measured on TopCount NXT, Packard.

### Methods

h-11βHSD1, 120 nM ³H-cortisone, 4 mM β-NADPH, antibody (1:200), serial dilutions of test compound and SPA particles (2 mg/well) were added to the wells. The reaction was initiated by mixing the different components and was allowed to proceed under shaking for 60 min at 30°C. The reaction was stopped be the addition of 10 fold excess of a stopping buffer containing 500 µM carbenoxolone and 1 µM cortisone. Data was analysed using GraphPad Prism software.

While the invention has been described and illustrated with reference to certain preferred embodiments thereof, those skilled in the art will appreciate that various changes, modifications, and substitutions can be made therein without departing from the spirit and scope of the present invention. Likewise, the specific pharmacological responses observed may vary according to and depending on the particular active compound selected or whether there are present pharmaceutical carriers, as well as the type of formulation and mode of administration employed, and such expected variations or differences in the results are contemplated in accordance with the objects and practices of the present invention. Accordingly, the invention is not to be limited as by the appended claims.

The features disclosed in the foregoing description and/or in the claims may both separately and in any combination thereof be material for realising the invention in diverse forms thereof.

## Claims

1. A compound of the general formula (1): 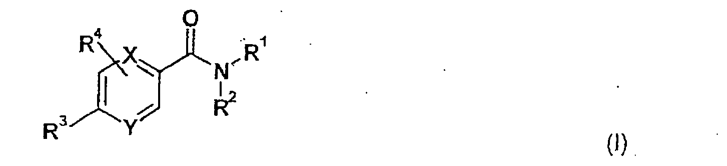 wherein
R¹ and R² together with the nitrogen to which they are attached is 6-aza-bicyclo[3.2.1]octane optionally substituted with at least one of C₁-C₆alkyl;
R³ is C₁-C₆alkyl, -NR⁸R⁹, -C(=O)NR⁸R⁹ or -OR¹⁰, wherein the alkyl group is optionally substituted with one or more of R¹¹;
R⁴ is hydrogen, halo, hydroxy, cyano, trihalomethyl or C₁-C₆alkyl;
R⁸ is hydrogen, C₁-C₆alkyl, C₃-C₁₀cycloalkyl, C₃-C₁₀cycloalkylC₁-C₆alkyl, C₁-C₆alkyloxyC₁-C₆alkyl, arylC₁-C₆alkyloxyC₁-C₆alkyl or C₂-C₆alkenyl;
R⁹ is C₁-C₆alkyl, C₃-C₁₀cycloalkyl, C₃-C₁₀cycloalkylC₁-C₆alkyl, C₃-C₁₀cycloalkylcarbonyl-, C₃-C₁₀hetcycloalkylcarbonyl-, arylcarbonyl-, hetarylcarbonyl-, C₁-C₆alkyloxyC₁-C₆alkyl, NR¹²R¹³carbonylC₁-C₆alkyl-, R¹⁴C₁-C₆alkylcarbonyl-, -COR¹⁵, C₁-C₆alkylS(O)ₙ-, arylS(O)ₙ-, arylC₁-C₆alkylS(O)ₙ-, arylC₁-C₆alkyl or hetarylC₁-C₆alkyl, wherein the alkyl, cycloalkyl, aryl and hetaryl groups independently are optionally substituted with one or more R¹¹;
R¹⁰ is C₁-C₆alkyl, arylC₁-C₆alkyl or NR¹²R¹³carbonylC₁-C₆alkyl, wherein the alkyl and aryl groups independently are optionally substituted with one or more R¹¹;
R¹¹ is R⁵, R⁶, halo, hydroxy, oxo, cyano, -COR¹⁵, C₁-C₈alkyl, C₁-C₈alkyloxy, C₃-C₁₀cycloalkyl, trihalomethyl, trihalomethyloxy, aryl, arylC₁-C₆alkyl, C₁-C₆alkyloxyC₁-C₆alkyl, aryloxyC₁-C₆alkyl, arylC₁-C₆alkyloxyC₁-C₆alkyl, hetaryl, hetarylC₁-C₆alkyl, hetaryloxyC₁-C₆alkyl, hetarylC₁-C₆alkyloxyC₁-C₆alkyl, -NR¹²R¹³, -SO₂NR¹²R¹³, NR¹²R¹³carbonylC₁-C₆alkyl, R¹⁶carbonylN(R¹²)-, arylS(O)ₙ-, hetarylS(O)ₙ- or R¹⁷S(O)ₙN(R¹²)-; wherein the aryl and hetaryl groups independently are optionally substituted with one or more R¹⁸;
R¹² and R¹³ independently are hydrogen, C₁-C₈alkyl, C₃-C₁₀cycloalkyl, C₃-C₁₀cycloalkylC₁-C₆-alkyl, C₃-C₁₀hetcycloalkyl, aryl, hetaryl, arylC₁-C₆alkyl or hetarylC₁-C₆alkyl wherein the alkyl, aryl and hetaryl groups independently are optionally substituted with one or more of R¹⁸; or
R¹² and R¹³ together with the nitrogen to which they are attached, are forming a saturated or partially saturated cyclic, bicyclic or tricyclic ring system containing from 4 to 12 carbon atoms and from 0 to 2 additional heteroatoms selected from nitrogen or oxygen, the ring system optionally being substituted with at least one R⁵, R⁵oxy-, R⁶, halo, cyano, hydroxy, oxo, C₁-C₈alkyl, aryl, hetaryl, arylC₁-C₆alkyl, hetarylC₁-C₆alkyl, C₁-C₆alkyloxyC₁-C₆alkyl, NR¹²R¹³carbonylC₁-C₆alkyl, NR¹²R¹³C₁-C₆alkylcarbonyl-, R¹⁴C₁-C₆alkylcarbonyl- or -COR¹⁵;
R¹⁴ is C₁-C₆alkyloxy, C₃-C₁₀cycloalkyloxy-, C₃-C₁₀cydoalkylC₁-C₆alkyloxy-, C₃-C₁₀hetcycloalkyloxy-, aryl, hetaryl, arylC₁-C₆alkyloxy, hetarylC₁-C₆alkyloxy, -NR¹²R¹³, -COR¹⁵, wherein the alkyl, cycloalkyl, hetcycloalkyl, aryl and hetaryl groups independently are optionally substituted with one or more of R²⁰;
R¹⁵ is C₁-C₅alkyl, hydroxy, C₁-C₈alkyloxy, -NR¹²R¹³, aryl, aryloxy or arylC₁-C₆alkyloxy;
R¹⁶ is R⁶, C₁-C₆alkyl, C₂-C₆alkenyl, aryl, arylC₁-C₆alkyl, hetaryl, hetarylC₁-C₆alkyl, C₃-C₁₀-cycloalkyl, C₃-C₁₀hetcycloalkyl, arylC₁-C₆alkyloxyC₁-C₆alkyl-, hetarylC₁-C₆alkyloxyC₁-C₆alkyl- or R¹²R¹³NC₁-C₆alkyl- wherein the alkyl, alkenyl, cycloalkyl, hetcycloalkyl, aryl and hetaryl groups are optionally substituted with R¹⁹;
R¹⁷ is C₁-C₆alkyl, C₃-C₁₀cycloalkyl, C₃-C₁₀hetcycloalkyl, aryl, arylC₁-C₆alkyl, hetaryl, hetarylC₁-C₆alkyl;
R¹⁸ is R⁶, -NR¹²R¹³, oxo, C₁-C₆alkyl, C₃-C₁₀cycloalkyl or C₃-C₁₀hetcycloalkyl, wherein the alkyl, cycloalkyl, hetcycloalkyl, aryl and hetaryl groups independently are optionally substituted with one or more of R⁷;
R¹⁹ is hydrogen, halo, hydroxy, oxo, nitro, cyano or -COR¹⁵;
R²⁰ is hydrogen; C₁-C₈alkyl, -NR¹²R¹³, C₁-C₆alkyloxy or arylC₁-C₆alkyl;
R⁵ is C₁₋C₆alkylcarbonyl-, C₃-C₁₀cycloalkylcarbonyl-, C₃-C₁₀cycloalkylC₁-C₆alkylcarbonyl-, arylcarbonyl-, arylC₁-C₆alkylcarbonyl-, hetarylcarbonyl- or hetarylC₁-C₆alkylcarbonyl-;
R⁶ is C₁-C₆alkyloxy-, aryloxy-, arylC₁-C₆alkyloxy-, hetaryloxy- or hetarylC₁-C₆alkyloxy-;
R⁷ is hydrogen, C₁-C₈alkyl, C₁-C₆alkyloxy or arylC₁-C₆alkyl;
X and Y independently are -CH- or nitrogen;
n is 1 or 2; or
a salt thereof with a pharmaceutically acceptable acid or base, or any optical isomer or mixture of optical isomers, including a racemic mixture, or any tautomeric forms, for use as a medicament.

2. The compound for use as a medicament according to claim 1 wherein X and Y are -CH-.

3. The compound for use as a medicament according to claim 1 or 2 wherein R³ is -NR⁸R⁹, wherein R⁸ and R⁹ are as defined in claim 1.

4. The compound for use as a medicament according to any of the claims 1-3 wherein R⁴ is hydrogen or halo.

5. The compound for use as a medicament according any of the claims 1-4 selected from the group consisting of: [4-(1,3,3-Trimethyl-6-aza-bicyclo[3.2.1]octane-6-carbonyl)-phenyl]carbamic acid *tert*-butyl ester;
(4-Amino-phenyl)-(1,3,3-trimethyl-6-aza-bicyclo[3.2.1]oct-6-yl)-methanone;
(4-Methylamino-phenyl)-(1,3,3-trimethyl-6-aza-bicyclo[3.2.1]oct-6-yl)-methanone;
N-Methyl-N-[4-(1,3,3-trimethyl-6-aza-bicyclo[3.2.1]octane-6-carbonyl)-phenyl]-methanesulfonamide;
N-Methyl-N-[4-(1,3,3-trimethyl-6-aza-bicyclo[3.2.1]octane-6-carbonyl)-phenyl]-benzenesulfonamide;
N-Methyl-C-phenyl-N-[4-(1,3,3-trimethyl-6-aza-bicyclo[3.2.1]octane-6-carbonyl)-phenyl]-methanesulfonamide;
N-[4-(1,3,3-Trimethyl-6-aza-bicyclo[3.2.1]octane-6-carbonyl)phenyl]-methanesulfonamide;
N-Ethyl-N-[4-(1,3,3-trimethyl-6-aza-bicyclo[3.2.1]octane-6-carbonyl)-phenyl]-methanesulfonamide;
N-Cyclopropylmethyl-N-[4-(1,3,3-trimethyl-6-aza-bicyclo[3.2.1]octane-6-carbonyl)-phenyl]-methanesulfonamide;
N-[4-(1,3,3-Trimethyl-6-aza-bicyclo[3.2.1]octane-6-carbonyl)-phenyl]-isonicotinamide;
2,4-Dichloro-N-[4-(1,3,3-trimethyl-6-aza-bicyclo[3.2.1]octane-6-carbonyl)phenyl]-benzamide;
2,4-Dimethoxy-N-[4-(1,3,3-trimethyl-6-aza-bicyclo[3.2.1]octane-6-carbonyl)-phenyl]-benzamide;
4-Trifluoromethoxy-N-[4-(1,3,3-frimethyl-8-aza-bicyclo[3.2.1]octane-6-carbonyl)phenyl]-benzamide;
3,4-Dimethoxy-N-[4-(1,3,3-trimethyl-6-aza-bicyclo[3.2.1]octane-6-carbonyl)-phenyl]-benzamide;
3,5-Dimethyl-N-[4-(1,3,3-trimethyl-6-aza-bicyclo[3.2.1]octane-6-carbonyl)-phenyl]-benzamide;
3-Trifluoromethoxy-N-[4-(1,3,3-trimethyl-6-aza-bicyclo[3.2.1]octane-6-carbonyl)-phenyl]-benzamide;
3,5-Dimethoxy-N-[4-(1,3,3-trimethyl-6-aza-bicyclo[3.2.1]octane-6-carbonyl)-phenyl]-benzamide;
3-Cyano-N-[4-(1,3,3-trimethyl-6-aza-bicyclo[3.2.1]octane-6-carbonyl)-phenyl]-benzamide;
N-[4-(1,3,3-Trimethyl-6-aza-bicyclo[3.2.1]octane-6-carbonyl)phenyl]-benzenesulfonamide;
Phenyl-N-[4-(1,3,3-trimethyl-6-aza-bicyclo[3.2.1]octane-6-carbonyl)-phenyl]-methanesulfonamide;
Butane-1-sulfonic acid [4-(1,3,3-trimethyl-6-aza-bicyclo[3.2.1]octane-6-carbonyl)-phenyl]-amide;
3-Trifluoromethyl-N-[4-(1,3,3-trimethyl-6-aza-bicyclo[3.2.1]octane-6-carbonyl)-phenyl]-benzenesulfonamide;
N-[4-(1,3,3-Trimethyl-6-aza-bicyclo[3.2.1]octane-6-carbonyl)-phenyl]-isophthalamic acid methyl ester;
N-[4-(1,3,3-Trimethyl-6-aza-bicyclo[3.2.1]octane-6-carbonyl)-phenyl]-isophthalamic acid;
4-Methoxy-cyclo-hexanecarboxylic acid [4-(1,3,3-trimethyl-6-aza-bicyclo[3.2.1]octane-6-carbonyl)-phenyl]-amide;
6-[4-(1,3,3-Trimethyl-6-aza-bicyclo[3.2.1]octane-6-carbonyl)phenylcarbamoyl]-nicotinic acid methyl ester;
6-[4-(1,3,3-Trimethyl-6-aza-bicyclo[3.2.1]octane-6-carbonyl)-phenylcarbamoyl]-nicotinic acid;
N-[4-(1,3,3-Trimethyl-6-aza-bicyclo[3.2.1]octane-6-carbonyl)-phenyl]-malonamic acid ethyl ester;
N-[4-(1,3,3-Trimethyl-6-aza-bicyclo[3.2.1]octane-6-carbonyl)-phenyl]-malonamic acid *tert-*butyl ester;
3-[4-(1,3,3-Trimethyl-6-aza-bicyclo[3.2.1]octane-6-carbonyl)-phenylcarbamoyl]-cyclohexanecarboxylic acid methyl ester;
3-[4-(1,3,3-Trimethyl-6-aza-bicyclo[3.2.1]octane-6-carbonyl)-phenylcarbamoyl]-cyclohexanecarboxylic acid;
N-[4-(1,3,3-Trimethyl-6-aza-bicyclo[3.2.1]octane-6-carbonyl)-phenyl]-benzamide;
N-[4-(1,3,3-Trimethyl-6-aza-bicyclo[3.2.1]octane-6-carbonyl)-phenyl]-isonicotinamide;
N-[4-(1,3,3-Trimethyl-6-aza-bicyclo[3.2.1]octane-6-carbonyl)-phenyl]-nicotinamide;
Pyridine-2-carboxylic acid [4-(1,3,3-trimethyl-6-aza-bicyclo[3.2.1]octane-6-carbonyl)-phenyl]-amide;
1-Acetyl-piperidine-4-carboxylic acid [4-(1,3,3-trimethyl-6-aza-bicyclo[3.2.1]octane-6-carbonyl)-phenyl]-amide;
N-[4-(1,3,3-Trimethyl-6-aza-bicyclo[3.2.1]octane-6-carbonyl)-phenyl]-succinamic acid methyl ester;
N-[4-(1,3,3-Trimethyl-6-aza-bicyclo[3.2.1]octane-6-carbonyl)-phenyl]-succinamic acid;
N-[4-(1,3,3-Trimethyl-6-aza-bicyclo[3.2.1]octane-6-carbonyl)-phenyl]-malonamic acid;
2-Amino-N-[4-(1,3,3-trimethyl-6-aza-bicyclo[3.2.1]octane-6-carbonyl)phenyl]-acetamide;
2-Acetylamino-N-[4-(1,3,3-trimethyl-6-aza-bicyclo[3.2.1]octane-6-carbonyl)-phenyl]-succinamic acid methyl ester;
2-Acetylamino-N-[4-(1,3,3-trimethyl-6-aza-bicyclo[3.2.1]octane-6-carbonyl)-phenyl]-succinamic acid;
N-Methyl-N'-[4-(1,3,3-trimethyl-6-aza-bicyclo[3.2.1]octane-6-carbonyl)-phenyl]-succinamide;
N,N-Dimethyl-N'-[4-(1,3,3-trimethyl-6-aza-bicyclo[3.2.1]octane-6-carbonyl)-phenyl]-succinamide;
N-[5-(1,3,3-Trimethyl-6-aza-bicyclo[3.2.1]octane-6-carbonyl)-pyridin-2-yl]-benzamide;
N-Methyl-N-[4-(1,3,3-trimethyl-6-aza-bicyclo[3.2.1]octane-6-carbonyl)-phenyl]-benzamide;
3,5,N-Trimethyl-N-[4-(1,3,3-trimethyl-6-aza-bicyclo[3,2,1]octane-6-carbonyl)-phenyl)-benzamide;
2,4-Dimethoxy-N-methyl-N-[4-(1,3,3-trimethyl-6-aza-bicyclo[3.2.1]octane-6-carbonyl)-phenyl]-benzamide;
3,5-Dichloro-N-methyl-N-[4-(1,3,3-trimethyl-6-aza-bicyclo[3.2.1]octane-6-carbonyl)-phenyl]-benzamide;
4-Bromo-N,N-dimethyl-N-[4-(1,3,3-trimethyl-6-aza-bicyclo[3.2.1]octane-6-carbonyl)-phenyl]-benzamide;
N-Methyl-N-[4-(1,3,3-trimethyl-6-aza-bicyclo[3.2.1]octane-6-carbonyl)-phenyl]-isonicotinamide;
N-Methyl-N-[4-(1,3,3-trimethyl-6-aza-bicyclo[3.2.1]octane-6-carbonyl)-phenyl]-nicotinamide;
2-Piperidin-1-yl-N-[4-(1,3,3-trimethyl-6-aza-bicyclo[3.2.1]octane-6-carbonyl)-phenyl]-acetamide;
2-Morpholin-4-yl-N-[4-(1,3,3-trimethyl-6-aza-bicyclo[3.2.1]octane-6-carbonyl)-phenyl]-acetamide;
2-(4-Methyl-piperazin-1-yl)-N-[4-(1,3,3-trimethyl-6-aza-bicyclo[3.2.1]octane-6-carbonyl)-phenyl]-acetamide;
2-Dimethylamino-N-[4-(1,3,3-trimethyl-6-aza-bicyclo[3.2.1]octane-6-carbonyl)-phenyl]-acetamide;
2-[(2-Dimethylamino-ethyl)-methyl-amino]-N-[4-(1,3,3-trimethyl-6-aza-bicyclo[3.2.1]octane-6-carbonyl)-phenyl]-acetamide;
2-(3,4-Dihydro-2*H*-quinolin-1-yl)-N-[4-(1,3,3-trimethyl-6-aza-bicyclo[3.2.1]octane-6-carbonyl)-phenyl]-acetamide;
2-(4-Acetyl-piperidin-1-yl)-N-[4-(1,3,3-trimethyl-6-aza-bicyclo[3.2.1]octane-6-carbonyl)-phenyl]-acetamide;
2-(4-Dimethylamino-piperidin-1-yl)-N-[4-(1,3,3-trimethyl-6-aza-bicyclo[3.2.1]octane-6-carbonyl)-phenyl]-acetamide;
2-(4-Oxo-piperidin-1-yl)-N-[4-(1,3,3-trimethyl-6-aza-bicyclo[3.2.1]octane-6-carbonyl)-phenyl]-acetamide;
2-[Methyl-(1-methyl-piperidin-4-yl)-amino]-N-[4-(1,3,3-trimethyl-6-aza-bicyclo[3.2.1]octane-6-carbonyl)-phenyl]-acetamide;
2-(3,6-Dihydro-2*H*-pyridin-1-yl)-N-[4-(1,3,3-trimethyl-6-aza-bicyclo[3.2.1]octane-6-carbonyl)-phenyl]-acetamide;
1-{[4-(1,3,3-Trimethyl-6-aza-bicyclo[3.2.1]octane-6-carbonyl)-phenylcarbamoyl]-methyl}-piperidine-4-carboxylic acid ethyl ester;
N-[4-(1,3,3-Trimethyl-6-aza-bicyclo[3.2.1]octane-6-carbonyl)-phenyl]-2-(1,3,3-trimethyl-6-aza-bicyclo[3.2.1]oct-6-yl)-acetamide;
2-(4-Acetyl-piperazin-1-yl)-N-[4-(1,3,3-trimethyl-6-aza-bicyclo[3.2.1]octane-6-carbonyl)-phenyl]-acetamide;
2-(2,3,5,6-Tetrahydro-[1,2']bipyrazinyl-4-yl)-N-[4-(1,3,3-trimethyl-6-aza-bicyclo[3.2.1]octane-6-carbonyl)-phenyl]-acetamide;
2-(Cyclohexyl-methyl-amino)-N-[4-(1,3,3-trimethyl-6-aza-bicyclo[3.2.1]octane-6-carbonyl)-phenyl]-acetamide;
2-(2,4-Dimethoxy-benzyloxy)-N-[4-(1,3,3-trimethyl-6-aza-bicyclo[3.2.1]octane-6-carbonyl)-phenyl]-acetamide;
2-Benzyloxy-N-[4-(1,3,3-trimethyl-6-aza-bicyclo[3.2.1]octane-6-carbonyl)-phenyl]-acetamide;
2-(3,4-Dihydro-1*H*-isoquinolin-2-yl)-N-[4-(1,3,3-trimethyl-6-aza-bicyclo[3.2.1]octane-6-carbonyl)-phenyl]-acetamide;
2-(6-Aza-bicyclo[3.2.1]oct-6-yl)-N-[4-(1,3,3-trimethyl-6-aza-bicyclo[3.2.1]octane-6-carbonyl)-phenyl]-acetamide;
2-Azepan-1-yl-N-[4-(1,3,3-trimethyl-6-aza-bicyclo[3.2.1]octane-6-carbonyl)-phenyl]-acetamide;
2-(6,7-Dimethoxy-3,4-dihydro-1*H*-isoquinolin-2-yl)-N-[4-(1,3,3-trimethyl-6-aza-bicyclo[3.2.1]-octa ne-6-ca rbonyl)-phenyl]-acetamide;
2-Indol-1-yl-N-[4-(1,3,3-trimethyl-6-aza-bicyclo[3.2.1]octane-6-carbonyl)-phenyl]-acetamide;
2-Benzoimidazol-t-yl-N-[4-(1,3,3-trimethyl-6-aza-bicyclo[3.2.1]octane-6-carbonyl)-phenyl]-acetamide;
2-(2,3-Dihydro-indol-1-yl)-N-[4-(1,3,3-trimethyl-6-aza-bicyclo[3.2.1]octane-6-carbonyl)-phenyl]-acetamide;
2-Benzotriazol-1-yl-N-[4-(1,3,3-trimethyl-6-aza-bicyclo[3.2.1]octane-6-carbonyl)-phenyl]-acetamide;
2-(4-Benzoyl-piperidin-1-yl)-N-[4-(1,3,3-trimethyl-6-aza-bicyclo[3.2.1]octane-6-carbonyl)-phenyl]-acetamide;
2-(4-*tert*-Butyl-piperidin-1-yl)-N-[4-(1,3,3-trimethyl-6-aza-bicyclo[3.2.1]octane-6-carbonyl)-phenyl]-acetamide;
2-(4-Phenyl-piperidin-1-yl)-N-[4-(1,3,3-trimethyl-6-aza-bicyclo[3.2.1]octane-6-carbonyl)-phenyl]-acetamide;
2-(4-Hydroxy-4-phenyl-piperidin-1-yl)-N-[4-(1,3,3-trimethyl-6-aza-bicyclo[3.2.1]octane-6-carbonyl)-phenyl]-acetamide;
2-(4-Pyridin-2-yl-piperazin-1-yl)-N-[4-(1,3,3-trimethyl-6-aza-bicyclo[3.2.1]octane-6-carbonyl)-phenyl]-acetamide; 2-(4-Phenyl-piperazin-1-yl)-N-[4-(1,3,3-trimethyl-6-aza-bicyclo[3.2.1]octane-6-carbonyl)-phenyl]-acetam ide;
2-(4-Pyrimidin-2-yl-piperazin-1-yl)-N-[4-(1,3,3-trimethyl-6-aza-bicyclo[3.2.1]octane-6-carbonyl)-phenyl]-acetamide;
2-(4-Acetyl-4-phenyl-piperidin-1-yl)-N-[4-(1,3,3-trimethyl-6-aza-bicyclo[3.2.1]octane-6-carbonyl)-phenyl]-acetamide;
2-(4-Cyano-4-phenyl-piperidin-1-yl)-N-[4-(1,3,3-trimethyl-6-aza-bicyclo[3.2.1]octane-6-carbonyl)-phenyl]-acetamide;
2-(4-Pyridin-4-yl-piperazin-1-yl)-N-[4-(1,3,3-trimethyl-6-aza-bicyclo[3.2.1]octane-6-carbonyl)-phenyl]-acetamide;
2-[4-(2-Pyrrolidin-1-yl-acetyl)-piperazin-1-yl]-N-[4-(1,3,3-trimethyl-6-aza-bicyclo[3.2.1]octane-6-carbonyl)-phenyl]-acetamide;
2-(Piperidin-4-yloxy)-N-[4-(1,3,3-trimethyl-6-aza-bicyclo[3.2.1]octane-6-carbonyl)-phenyl]-acetamide;
2-(*2H*-Tetrazol-5-yl)-N-[4-(1,3,3-trimethyl-6-aza-bicyclo[3.2.1]octane-6-carbonyl)-phenyl]-acetamide;
3-Piperidin-1-yl-N-[4-(1,3,3-trimethyl-6-aza-bicyclo[3.2.1]octane-6-carbonyl)-phenyl]-propionamide;
3-Benzylamino-N-[4-(1,3,3-trimethyl-6-aza-bicyclo[3.2.1]octane-6-carbonyl)-phenyl]-propionamide;
2-(3,4-Dihydro-2*H*-quinolin-1-yl)-N-methyl-N-[4-(1,3,3-trimethyl-6-aza-bicyclo[3.2.1]octane-6-carbonyl)-phenyl]-acetamide;
1-Morpholin-4-yl-2-[4-(1,3,3-trimethyl-6-aza-bicyclo[3.2.1]octane-6-carbonyl)-phenylamino]-ethanone;
[4-(2-Piperidin-1-yl-ethylamino)-phenyl]-(1,3,3-trimethyl-6-aza-bicyclo[3.2.1]oct-6-yl)-methanone;
N-Methyl-N-phenyl-2-[4-(1,3,3-trimethyl-6-aza-bicyclo[3.2.1]octane-6-carbonyl)-phenyl-amino]-acetamide;
N-(2-Methoxy-ethyl)-N-[4-(1,3,3-trimethyl-6-aza-bicyclo[3.2.1]octane-6-carbonyl)-phenyl]-benzamide;
N-Allyl-N-[4-(1,3,3-trimethyl-6-aza-bicyclo[3.2.1]octane-6-carbonyl)-phenyl]-benzamide;
[4-(2-methoxy-ethylamino)-phenyl]-(1,3,3-trimethyl-6-aza-bicyclo[3.2.1]oct-6-yl)-methanone;
(4-Benzyloxy-phenyl)-(1,3,3-trimethyl-6-aza-bicyclo[3.2.1]oct-6-yl)-methanone;
3-[4-(1,3,3-Trimethyl-6-aza-bicyclo[3.2.1]octane-6-carbonyl)-benzoylamino]-propionic acid methyl ester;
3-[4-(1,3,3-Trimethyl-6-aza-bicyclo[3.2.1]octane-6-carbonyl)-benzoylamino]-propionic acid;
(4-Benzyloxy-phenyl)-(1,3,3-trimethyl-6-aza-bicyclo[3.2.1]oct-6-yl)-methanone;
or a salt thereof with a pharmaceutically acceptable acid or base, or any optical isomer or mixture of optical isomers, including a racemic mixture, or any tautomeric forms.

6. The compound according to any of the claims 1-5, for the treatment, prevention and/or prophylaxis of any conditions, disorders and diseases wherein a modulation or an inhibition of the activity of 11βHSD1 is beneficial

7. The compound according to claim 6 wherein the condition, disorder or disease is selected from metabolic syndrome, insulin resistance, dyslipidemia, hypertension, obesity, type 2 diabetes, impaired glucose tolerance (IGT), impaired fasting glucose (IFG), Latent Autoimmune Diabetes in the Adult (LADA), type 1 diabetes, diabetic late complications including cardiovascular diseases, cardiovascular disorders, disorders of lipid metabolism, neurodegenerative and psychiatric disorders, dysregulation of intraocular pressure including glaucoma, immune disorders, inappropriate immune responses, musculo-skeletal disorders, gastrointestinal disorders, polycystic ovarie syndrome (PCOS), reduced hair growth or other diseases, disorders or conditions that are influenced by intracellular glucocorticoid levels, adverse effects of increased blood levels of active endogenous or exogenous glucocorticoid, and any combination thereof, adverse effects of increased plasma levels of endogenous active glucocorticoid, Cushing's disease, Cushing's syndrome, adverse effects of glucocorticoid receptor agonist treatment of autoimmune diseases, adverse effects of glucocorticoid receptor agonist treatment of inflammatory diseases, adverse effects of glucocorticoid receptor agonist treatment of diseases with an inflammatory component, adverse effects of glucocorticoid receptor agonist treatment as a part of cancer chemotherapy, adverse effects of glucocorticoid receptor agonist treatment for surgical/post-surgical or other trauma, adverse effects of glucocorticoid receptor agonist therapy in the context of organ or tissue transplantation or adverse effects of glucocorticoid receptor agonist treatment in other diseases, disorders or conditions where glucocorticoid receptor agonists provide clinically beneficial effects.

8. A combination comprising a compound as defined in any one of claims 1-5 and one or more further pharmaceutically active substances.

9. The combination according to claim 8 wherein said further active substances are selected from antiobesity agents, antidiabetics, agents modifying the lipid metabolism, antihypertensive agents, glucocorticoid receptor agonists, agents for the treatment and/or prevention of complications resulting from or associated with diabetes and agents for the treatment and/or prevention of complications and disorders resulting from or associated with obesity.

10. A pharmaceutical composition comprising a compound as defined in any one of claims 1-5, or a combination as defined in claim 8 or claim 9, together with one or more pharmaceutically acceptable carriers or diluents.

11. A combination as defined in claim 8 or 9 or a composition as defined in claim 10 for use as a medicament.

12. A compound of the general formula (I): 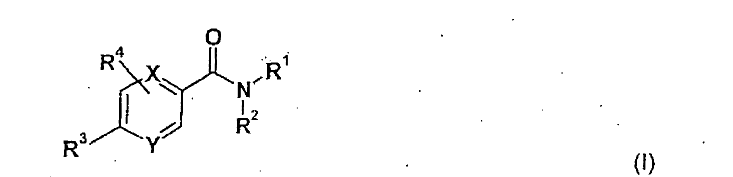 wherein
R¹ and R² together with the nitrogen to which they are attached is 6-aza-bicyclo[3.2.1]octane optionally substituted with at least one of C₁-C₆alkyl;
R³ is C₁-C₆alkyl, -NR⁸R⁹, -C(=O)NR⁸R⁹ or -OR¹⁰, wherein the alkyl group is optionally substituted with one or more of R¹¹;
R⁴ is hydrogen, halo, hydroxy, cyano, trihalomethyl or C₁-C₆alkyl;
R⁸ is hydrogen, C₁-C₆alkyl, C₃-C₁₀cycloalky), C₃-C₁₀cycloalkylC₁-C₆alkyl, C₁-C₆alkyloxyC₁-C₆alkyl, arylC₁-C₆alkyloxyC₁-C₆alkyl or C₂-C₆alkenyl;
R⁹ is C₁-C₆alkyl, C₃-C₁₀cycloalkyl, C₃-C₁₀cycloalkylC₁-C₆alkyl, C₃-C₁₀cycloalkylcarbonyl-, C₃-C₁₀hetcycloalkylcarbonyl-, arylcarbonyl-, hetarylcarbonyl-, C₁-C₆alkyloxyC₁-C₆alkyl, NR¹²R¹³carbonylC₁-C₆alkyl-, R¹⁴C₁-C₆alkylcarbonyl-, -COR¹⁵, C₁-C₆alkylS(O)ₙ-, arylS(O)ₙ-, arylC₁-C₆alkylS(O)ₙ-, arylC₁-C₆alkyl or hetarylC₁-C₆alkyl, wherein the alkyl, cycloalkyl, aryl and hetaryl groups independently are optionally substituted with one or more R¹¹;
R¹⁰ is C₁-C₆alkyl, arylC₁-C₆alkyl or NR¹²R¹³carbonylC₁-C₆alkyl, wherein the alkyl and aryl groups independently are optionally substituted with one or more R¹¹;
R¹¹ is R⁵, R⁶, halo, hydroxy, oxo, cyano, -COR¹⁵, C₁-C₈alkyl, C₁-C₈alkyloxy, C₃-C₁₀cycloalkyl, trihalomethyl, trihalomethyloxy, aryl, arylC₁-C₆alkyl, C₁-C₆alkyloxyC₁-C₆alkyl, aryloxyC₁-C₆alkyl, arylC₁-C₆alkyloxyC₁-C₆alkyl, hetaryl, hetarylC₁-C₆alkyl, hetaryloxyC₁-C₆alkyl, hetarylC₁-C₆alkyloxyC₁-C₆alkyl, -NR¹²R¹³, -SO₂NR¹²R¹³, NR¹²R¹³carbonylC₁-C₆alkyl, R¹⁶carbonylN(R¹²)-, arylS(O)ₙ-, hetarylS(O)ₙ- or R¹⁷S(O)ₙN(R¹²)-; wherein the aryl and hetaryl groups independently are optionally substituted with one or more R¹⁸;
R¹² and R¹³ independently are hydrogen, C₁-C₈alkyl, C₃-C₁₀cycloalkyl, C₃-C₁₀cycloalkylC₁-C₆-alkyl, C₃-C₁₀hetcyctoalkyl, aryl, hetaryl, arylC₁-C₆alkyl or hetarylC₁-C₆alkyl wherein the alkyl, aryl and hetaryl groups independently are optionally substituted with one or more of R¹⁸; or
R¹² and R¹³ together with the nitrogen to which they are attached, are forming a saturated or partially saturated cyclic, bicyclic or tricyclic ring system containing from 4 to 12 carbon atoms and from 0 to 2 additional heteroatoms selected from nitrogen or oxygen, the ring system optionally being substituted with at least one R⁵, R⁵oxy-, R⁶, halo, cyano, hydroxy, oxo, C₁-C₈alkyl, aryl, hetaryl, arylC₁-C₆alkyl, hetarylC₁-C₆alkyl, C₁-C₆alkyloxyC₁-C₆alkyl; NR¹²R¹³carbonylC₁C₆alkyl, NR¹²R¹³C₁-C₆alkylcarbonyl-, R¹⁴C₁-C₆alkylcarbonyl- or -COR¹⁵;
R¹⁴ is C₁-C₆alkyloxy, C₃-C₁₀cycloalkyloxy-, C₃-C₁₀cycloalkylC₁-C₆alkyloxy-, C₃-C₁₀hetcycloalkyloxy-, aryl, hetaryl, arylC₁-C₆alkyloxy, hetarylC₁-C₆alkyloxy, -NR¹²R¹³, -COR¹⁵, wherein the alkyl, cycloalkyl, hetcycloalkyl, aryl and hetaryl groups independently are optionally substituted with one or more of R²⁰;
R¹⁵ is C₁-C₆alkyl, hydroxy, C₁-C₈alkyloxy, -NR¹²R¹³, aryl, aryloxy or arylC₁-C₆alkyloxy;
R¹⁶ is R⁶, C₁-C₆alkyl, C₂-C₆alkenyl, aryl, arylC₁-C₆alkyl, hetaryl, hetarylC₁-C₆alkyl, C₃-C₁₀-cycloalkyl, C₃-C₁₀hetcycloalkyl, arylC₁-C₆alkyloxyC₁-C₆alkyl-, hetarylC₁-C₆alkyloxyC₁-C₆alkyl-or R¹²R¹³NC₁-C₆alkyl- wherein the alkyl, alkenyl, cycloalkyl, hetcycloalkyl, aryl and hetaryl groups are optionally substituted with R¹⁹;
R¹⁷ is C₁-C₆alkyl, C₃-C₁₀cycloalkyl, C₃-C₁₀hetcycloalkyl, aryl, arylC₁-C₆alkyl, hetaryl, hetarylC₁-C₆alkyl;
R¹⁸ is R⁶, -NR¹²R¹³, oxo, C₁-C₆alkyl, C₃-C₁₀cycloalkyl or C₃-C₁₀hetcycloalkyl, wherein the alkyl, cycloalkyl, hetcycloalkyl, aryl and hetaryl groups independently are optionally substituted with one or more of R⁷;
R¹⁹ is hydrogen, halo, hydroxy, oxo, nitro, cyano or -COR¹⁵;
R²⁰ is hydrogen, C₁-C₈alkyl, -NR¹²R¹³, C₁-C₆alkyloxy or arylC₁-C₆alkyl;
R⁵ is C₁-C₆alkylcarbonyl-, C₃-C₁₀acycloalkylcarbonyl-, C₃-C₁₀cycloalkylC₁-C₆alkylcarbonyl-, arylcarbonyl-, arylC₁-C₆alkylcarbonyl-, hetarylcarbonyl- or hetarylC₁-C₆alkylcarbonyl-;
R⁶ is C₁-C₆alkyloxy-, aryloxy-, arylC₁-C₆alkyloxy-, hetaryloxy- or hetarylC₁-C₆alkyloxy-; R⁷ is hydrogen, C₁-C₈alkyl, C₁-C₆alkyloxy or arylC₁-C₆alkyl;
X and Y independently are -CH- or nitrogen;
n is 1 or 2; or
a salt thereof with a pharmaceutically acceptable acid or base, or any optical isomer or mixture of optical isomers, including a racemic mixture, or any tautomeric forms;
with the proviso that the compound is not

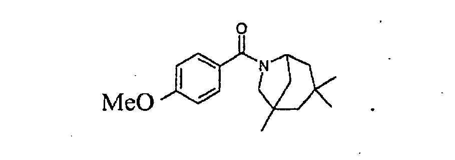

13. The compound according to claim 12 wherein X and Y are -CH-.

14. The compound according to claim 12 or 13 wherein R³ is -NR⁸R⁹, wherein R⁸ and R⁹ are as defined in claim 1.

15. The compound according to any of the claims 12 to 14 wherein R⁴ is hydrogen or halo.

16. The compound according any of the claims 12 to 15 selected from the group consisting of: [4-(1,3,3-Trimethyl-6-aza-bicyclo[3.2.1]octane-6-carbonyl)-phenyl]carbamic acid *tert*-butyl ester;
(4-Amino-phenyl)-(1,3,3-trimethyl-6-aza-bicyclo[3.2.1]oct-6-yl)-methanone;
(4-Methylamino-phenyl)-(1,3,3-trimethyl-6-aza-bicyclo[3.2.1]oct-6-yl)-methanone;
N-Methyl-N-[4-(1,3,3-trimethyl-6-aza-bicyclo[3.2.1]octane-6-carbonyl)-phenyl]-methanesulfonamide;
N-Methyl-N-[4-(1,3,3-trimethyl-6-aza-bicyclo[3.2.1]octane-6-carbonyl)-phenyl]-benzenesulfonamide;
N-Methyl-C-phenyl-N-[4-(1,3,3-trimethyl-6-aza-bicyclo[3.2.1]octane-6-carbonyl)-phenyl]-methanesulfonamide; N-[4-(1,3,3-Trimethyl-6-aza-bicyclo[3.2.1]octane-6-carbonyl)-phenyl]-methanesulfonamide;
N-Ethyl-N-[4-(1,3,3-trimethyl-6-aza-bicyclo[3.2.1]octane-6-carbonyl)phenyl]-methanesulfonamide;
N-Cyclopropylmethyl-N-[4-(1,3,3-trimethyl-6-aza-bicyclo[3.2.1]octane-6-carbonyl)-phenyl]-methanesulfonamide;
N-[4-(1,3,3-Trimethyl-6-aza-bicyclo[3.2.1]octane-6-carbonyl)-phenyl]-isonicotinamide;
2,4-Dichloro-N-[4-(1,3,3-trimethyl-6-aza-bicyclo[3.2.1]octane-6-carbonyl)-phenyl]-benzamide;
2,4-Dimethoxy-N-[4-(1,3,3-trimethyl-6-aza-bicyclo[3.2.1]octane-6-carbonyl)-phenyl]-benzamide;
4-Trifluoromethoxy-N-[4-(1,3,3-trimethyl-6-aza-bicyclo[3.2.1]octane-6-carbonyl)-phenyl]-benzamide;
3,4-Dimethoxy-N-[4-(1,3,3-trimethyl-6-aza-bicyclo[3.2.1]octane-6-carbonyl)-phenyl]-benzamide;
3,5-Dimethyl-N-[4-(1,3,3-trimethyl-6-aza-bicyclo[3.2.1]octane-6-carbonyl)-phenyl]-benzamide;
3-Trifluoromethoxy-N-[4-(1,3,3-trimethyl-6-aza-bicyclo[3.2.1]octane-6-carbonyl)-phenyl]-benzamide;
3,5-Dimethoxy-N-[4-(1,3,3-trimethyl-6-aza-bicyclo[3.2.1]octane-6-carbonyl)-phenyl]-benzamide;
3-Cyano-N-[4-(1,3,3-trimethyl-6-aza-bicyclo[3.2.1]octane-6-carbonyl)-phenyl]-benzamide; N-[4-(1,3,3-Trimethyl-6-aza-bicyclo[3.2.1]octane-6-carbonyl)-phenyl]-benzenesulfonamide;
Phenyl-N-[4-(1,3,3-trimethyl-6-aza-bicyclo[3.2.1]octane-6-carbonyl)-phenyl]-methanesulfonamide;
Butane-1-sulfonic acid [4-(1,3,3-trimethyl-6-aza-bicyclo[3.2.1]octane-6-carbonyl)phenyl]-amide;
3-Trifluoromethyl-N-[4-(1,3,3-trimethyl-6-aza-bicyclo[3.2.1]octane-6-carbonyl)-phenyl]-benzenesulfonamide;
N-[4-(1,3,3-Trimethyl-6-aza-bicyclo[3.2.1]octane-6-carbonyl)-phenyl]-isophthalamic acid methyl ester;
N-[4-(1,3,3-Trimethyl-6-aza-bicyclo[3.2.1]octane-6-carbonyl)-phenyl]-isophthalamic acid;
4-Methoxy-cyclo-hexanecarboxylic acid [4-(1,3,3-trimethyl-6-aza-bicyclo[3.2.1]octane-6-carbonyl)-phenyl]-amide;
6-[4-(1,3,3-Trimethyl-6-aza-bicyclo[3.2.1]octane-6-carbonyl)-phenylcarbamoyl]-nicotinic acid methyl ester;
6-[4-(1,3,3-Trimethyl-6-aza-bicyclo[3.2.1]octane-6-carbonyl)-phenylcarbamoyl]-nicotinic acid;
N-[4-(1,3,3-Trimethyl-6-aza-bicyclo[3.2.1]octane-6-carbonyl)-phenyl]-malonamic acid ethyl ester;
N-[4-(1,3,3-Trimethyl-6-aza-bicyclo[3.2.1]octane-6-carbonyl)-phenyl]-malonamic acid *tert-*butyl ester;
3-[4-(1,3,3-Trimethyl-6-aza-bicyclo[3.2.1]octane-6-carbonyl)phenylcarbamoyl]-cyclohexanecarboxylic acid methyl ester;
3-[4-(1,3,3-Trimethyl-6-aza-bicyclo[3.2.1]octane-6-carbonyl)-phenylcarbamoyl]-cyclohexanecarboxylic acid; N-[4-(1,3,3-Trimethyl-6-aza-bicyclo[3.2.1]octane-6-carbonyl)-phenyl]-benzamide; N-[4-(1,3,3-Trimethyl-6-aza-bicyclo[3.2.1]octane-6-carbonyl)-phenyl]-isonicotinamide; N-[4-(1,3,3-Trimethyl-6-aza-bicyclo[3.2.1]octane-6-carbonyl)-pheny;]-nicotinamide;
Pyridine-2-carboxylic acid [4-(1,3,3-trimethyl-6-aza-bicyclo[3.2.1]octane-6-carbonyl)-phenyl]-amide;
1-Acetyl-piperidine-4-carboxylic acid [4-(1,3,3-trimethyl-6-aza-bicyclo[3.2.1]octane-6-carbonyl)-phenyl]-amide;
N-[4-(1,3,3-Trimethyl-6-aza-bicyclo[3.2.1]octane-6-carbonyl)-phenyl]-succinamic acid methyl ester;
N-[4-(1,3,3-Trimethyl-6-aza-bicyclo[3.2.1]octane-6-carbonyl)-phenyl]-succinamic acid;
N-[4-(1,3,3-Trimethyl-6-aza-bicyclo[3.2.1]octane-6-carbonyl)-phenyl]-malonamic acid;
2-Amino-N-[4-(1,3,3-trimethyl-6-aza-bicyclo[3.2.1]octane-6-carbonyl)-phenyl]-acetamide;
2-Acetylamino-N-[4-(1,3,3-trimethyl-6-aza-bicycto[3.2.1]octane-6-carbonyl)-phenyl]-succinamic acid methyl ester;
2-Acetylamino-N-[4-(1,3,3-trimethyl-6-aza-bicyclo[3.2.1]octane-6-carbonyl)-phenyl]-succinamic acid;
N-Methyl-N'-[4-(1,3,3-trimethyl-6-aza-bicyclo[3.2.1]octane-6-carbonyl)-phenyl]-succinamide;
N,N-Dimethyl-N'-[4-(1,3,3-trimethyl-6-aza-bicyclo[3.2.1]octane-6-carbonyl)-phenyl]-succinamide;
N-[5-(1,3,3-Trimethyl-6-aza-bicyclo[3.2.1]octane-6-carbonyl)-pyridin-2-yl]-benzamide;
N-Methyl-N-[4-(1,3,3-trimethyl-6-aza-bicyclo[3.2.1]octane-6-carbonyl)-phenyl]-benzamide;
3,5,N-Trimethyl-N-[4-(1,3,3-trimethyl-6-aza-bicyclo[3,2,1]octane-6-carbonyl)-phenyl)-benzamide;
2,4-Dimethoxy-N-methyl-N-[4-(1,3,3-trimethyl-6-aza-bicyclo[3.2.1]octane-6-carbonyl)-phenyl]-benzamide;
3,5-Dichloro-N-methyl-N-[4-(1,3,3-trimethyl-6-aza-bicyclo[3.2.1]octane-6-carbonyl)-phenyl]-benzamide;
4-Bromo-N,N,-dimethyl-N-[4-(1,3,3-trimethyl-6-aza-bicyclo[3.2.1]octane-6-carbonyl)-phenyl]-benzamide;
N-Methyl-N-[4-(1,3,3-trimethyl-6-aza-bicyclo[3.2.1]octane-6-carbonyl)phenyl]-isonicotinamide;
N-Methyl-N-[4-(1,3,3-trimethyl-6-aza-bicyclo[3.2.1]octane-6-carbonyl)-phenyl]-nicotinamide;
2-Piperidin-1-yl-N-[4-(1,3,3-trimethyl-6-aza-bicyclo[3.2.1]octane-6-carbonyl)-phenyl]-acetamide;
2-Morpholin-4-yl-N-[4-(1,3,3-trimethyl-6-aza-bicyclo[3.2.1]octane-6-carbonyl)-phenyl]-acetamide;
2-(4-Methyl-piperazin-1-yl)-N-[4-(1,3,3-trimethyl-6-aza-bicyclo[3.2.1]octane-6-carbonyl)-phenyl]-acetamide;
2-Dimethylamino-N-[4-(1,3,3-trimethyl-6-aza-bicyclo[3.2.1]octane-6-carbonyl)-phenyl]-acetamide;
2-[(2-Dimethylamino-ethyl)-methyl-amino]-N-[4-(1,3,3-trimethyl-6-aza-bicyclo[3.2.1]octane-6-carbonyl)-phenyl]-acetamide;
2-(3,4-Dihydro-2*H*-quinolin-1-yl)-N-[4-(1,3,3-trimethyl-6-aza-bicyclo[3.2.1]octane-6-carbonyl)-phenyl]-acetamide;
2-(4-Acetyl-piperidin-1-yl)-N-[4-(1,3,3-trimethyl-6-aza-bicyclo[3.2.1]octane-6-carbonyl)-phenyl]-acetamide;
2-(4-Dimethylamino-piperidin-1-yl)-N-[4-(1,3,3-trimethyl-6-aza-bicycio[3.2.1]octane-6-carbonyl)-phenyl]-acetamide;
2-(4-Oxo-piperidin-1-yl)-N-[4-(1,3,3-trimethyl-6-aza-bicyclo[3.2.1]octane-6-carbonyl)-phenyl]-acetamide;
2-[Methyl-(1-methyl-piperidin-4-yl)-amino]-N-[4-(1,3,3-trimethyl-6-aza-bicyclo[3.2.1]octane-6-carbonyl)-phenyl]-acetamide;
2-(3,6-Dihydro-2*H*-pyridin-1-yl)-N-[4-(1,3,3-trimethyl-6-aza-bicyclo[3.2.1]octane-6-carbonyl)-phenyl]-acetamide;
1-{[4-(1,3,3-Trimethyl-6-aza-bicyclo[3.2.1]octane-6-carbonyl)-phenylcarbamoyl]-methyl}-piperidine-4-carboxylic acid ethyl ester;
N-[4-(1,3,3-Trimethyl-6-aza-bicyclo[3.2.1]octane-6-carbonyl)-phenyl]-2-(1,3,3-trimethyl-6-aza-bicyclo[3.2.1]oct-6-yl)-acetamide;
2-(4-Acetyl-piperazin-1-yl)-N-[4-(1,3,3-trimethyl-6-aza-bicyclo[3.2.1]octane-6-carbonyl)-pheriyl]-acetamide;
2-(2,3,5,6-Tetrahydro-[1,2']bipyrazinyl-4-yl)-N-[4-(1,3,3-trimethyl-6-aza-bicyclo[3.2.1]octane-6-carbonyl)-phenyl]-acetamide;
2-(Cyclohexyl-methyl-amino)N-[4-(1,3,3-trimethyl-6-aza-bicyclo[3.2.1]octane-6-carbonyl)-phenyl]-acetamide;
2-(2,4-Dimethoxy-benzyloxy)-N-[4-(1,3,3-trimethyl-6-aza-bicyclo[3.2.1]octane-6-carbonyl)-phenyl]-acetamide;
2-Benzyloxy-N-[4-(1,3,3-trimethyl-6-aza-bicyclo[3.2.1]octane-6-carbonyl)phenyl]-acetamide;
2-(3,4-Dihydro-1*H*-isoquinolin-2-yl_-N-[4-(1,3,3-trimethyl-6-aza-bicyclo[3.2.1]octane-6-carbonyl)-phenyl]-acetamide;
2-(6-Aza-bicyclo[3.2.1]oct-6-yl)-N-[4-(1,3,3-trimethyl-6-aza-bicyclo[3.2.1]octane-6-carbonyl)-phenyl]-acetamide;
2-Azepan-1-yl-N-[4-(1,3,3-trimethyl-6-aza-bicyclo[3.2.1]octane-8-carbonyl)-phenyl]-acetamide;
2-(6,7-Dimethoxy-3,4-dihydro-*1H*-isoquinolin-2-yl)-N-[4-(1,3,3-trimethyl-6-aza-bicyclo[3.2.1]-octane-6-carbonyl)-phenyl]-acetamide;
2-Indol-1-yl-N-[4-(1,3,3-trimethyl-6-aza-bicyclo[3.2.1]octane-6-carbonyl)-phenyl]-acetamide;
2-Benzoimidazol-1-yl-N-[4-(1,3,3-trimethyl-6-aza-bicyclo[3.2.1]octane-6-carbonyl)-phenyl]-acetamide;
2-(2,3-Dihydro-indol-1-yl)-N-[4-(1,3,3-trimethyl-6-aza-bicyclo[3.2.1]octane-6-carbonyl)-phenyl]-acetamide;
2-Benzotriazol-1-yl-N-[4-(1,3,3-trimethyl-6-aza-bicyclo[3.2.1]octane-6-carbonyl)-phenyl]-acetamide;
2-(4-Benzoyl-piperidin-1-yl)-N-[4-(1,3,3-trimethyl-6-aza-bicyclo[3.2.1]octane-6-carbonyl)-phenyl]-acetamide;
2-(4-*tert*-Butyl-piperidin-1-yl)-N-[4-(1,3,3-trimethyl-6-aza-bicyclo[3.2.1]octane-6-carbonyl)-phenyl]-acetamide;
2-(4-Phenyl-piperidin-1-yl)-N-[4-(1,3,3-trimethyl-6-aza-bicyclo[3.2.1]octane-6-carbonyl)-phenyl]-acetamide;
2-(4-Hydroxy-4-phenyl-piperidin-1-yl)-N-[4-(1,3,3-trimethyl-6-aza-bicyclo[3.2.1]octane-6-carbonyl)-phenyl]-acetamide;
2-(4-Pyridin-2-yl-piperazin-1-yl)-N-[4-(1,3,3-trimethyl-6-aza-bicyclo[3.2.1]octane-6-carbonyl)-phenyl]-acetamide;
2-(4-Phenyl-piperazin-1-yl)-N-[4-(1,3,3-trimethyf-6-aza-bicyclo[3.2.1]octane-6-carbonyl)-phenyl]-acetamide;
2-(4-Pyrimidin-2-yl-piperazin-1-yl)-N-[4-(1,3,3-trimethyl-6-aza-bicyclo[3.2.1]octane-6-carbonyl)-phenyl]-acetamide;
2-(4-Acetyl-4-phenyl-piperidin-1-yl)N-[4-(1,3,3-trimethyl-6-aza-bicyclo[3.2.1]octane-6-carbonyl)phenyl]-acetamide;
2-(4-Cyano-4-phenyl-piperidin-1-yl)-N-[4-(1,3,3-trimethyl-6-aza-bicyclo[3.2.1]octane-6-carbonyl)-phenyl]-acetamide;
2-(4-Pyridin-4-yl-piperazin-1-yl)-N-[4-(1,3,3-trimethyl-6-aza-bicyclo[3.2.1]octane-6-carbonyl)-phenyl]-acetamide;
2-[4-(2-Pyrrolidin-1-yl-acetyl)-piperazin-1-yl]-N-[4-(1,3,3-trimethyl-6-aza-bicyclo[3.2.1]octane-6-carbonyl)-phenyl]-acetamide;
2-(Piperidin-4-yloxy)-N-[4-(1,3,3-trimethyl-6-aza-bicyclo[3.2.1]octane-6-carbonyl)-phenyl]-acetamide;
2-(2*H*-Tetrazol-5-yl)-N-[4-(1,3,3-trimethyl-6-aza-bicyclo[3.2.1]octane-6-carbonyl)-phenyl]-acetamide;
3-Piperidin-1-yl-N-[4-(1,3,3-trimethyl-6-aza-bicyclo[3.2.1]octane-6-carbonyl)-phenyl]-propionamide;
3-Benzylamino-N-[4-(1,3,3-trimethyl-6-aza-bicyclo[3.2.1]octane-6-carbonyl)-phenyl]-propionamide;
2-(3,4-Dihydro-2*H*-quinolin-1-yl)-N-methyl-N-[4-(1,3,3-trimethyl-6-aza-bicyclo[3.2.1]octane-6-carbonyl)-phenyl]-acetamide;
1-Morpholin-4-yl-2-[4-(1,3,3-trimethyl-6-aza-bicyclo[3.2.1]octane-6-carbonyl)-phenylamino]-ethanone;
[4-(2-Piperidin-1-yl-ethylamino)-phenyl]-(1,3,3-trimethyl-6-aza-bicyclo[3.2.1]oct-6-yl)-methanone;
N-Methyl-N-phenyl-2-[4-(1,3,3-trimethyl-6-aza-bicyclo[3.2.1]octane-6-carbonyl)-phenylamino]-acetamide;
N-(2-Methoxy-ethyl)N-[4-(1,3,3-trimethyl-6-aza-bicyclo[3.2.1]octane-6-carbonyl)-phenyl]-benzamide;
N-Allyl-N-[4-(1,3,3-trimethyl-6-aza-bicyclo[3.2.1]octane-6-carbonyl)-phenyl]-benzamide;
[4-(2-methoxy-ethylamino)-phenyl]-(1,3,3-trimethyl-6-aza-bicyclo[3.2.1]oct-6-yl)-methanone;
(4-Benzyloxy-phenyl)-(1,3,3-trimethyl-6-aza-bicyclo[3.2.1]oct-6-yl)-methanone; 3-[4-(1,3,3-Trimethyl-6-aza-bicyclo[3.2.1]octane-6-carbonyl)-benzoylamino]-propionic acid methyl ester;
3-[4-(1,3,3-Trimethyl-6-aza-bicyclo[3.2.1]octane-6-carbonyl)-benzoylamino]-propionic acid;
(4-Benzyloxy-phenyl)-(1,3,3-trimethyl-6-aza-bicyclo[3.2.1]oct-6-yl)-methanone;
or a salt thereof with a pharmaceutically acceptable acid or base, or any optical isomer or mixture of optical isomers, including a racemic mixture or any tautomeric forms.

17. A process for preparing a compound as defined in any of claims 12 to 16, said process selected from (A) to (F), below: 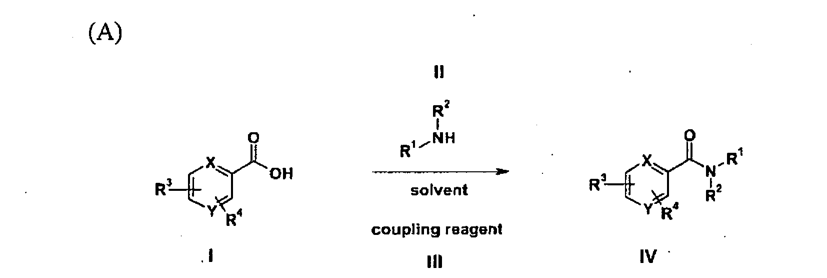 an acid (I) wherein R³, R⁴, X and Y are defined in claim 12 is coupled with an amine (II) wherein R¹ and R² are defined in claim 12 under standard amide forming conditions using a coupling reagent (III) affording amide (IV) wherein R¹, R², R³, R⁴, X and Y are defined as above; or 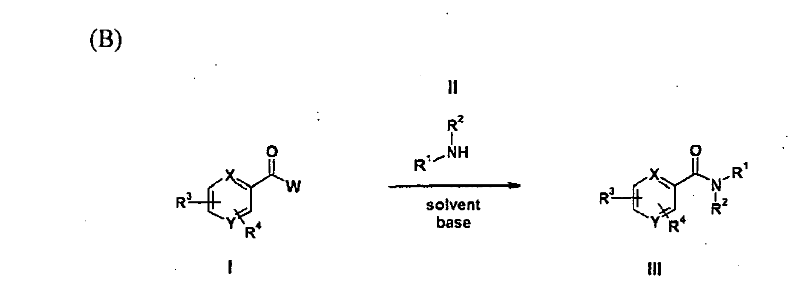 an acid derivative (I) wherein W is halo, R²⁰(C=O)O-, C₁₋C₆alkyloxy or arylC₁₋C₆alkyloxy, R²⁰ is C₁₋C₆alkyl or arylC₁₋C₆alk-yl and R³, R⁴, X and Y are defined in claim 12 is reacted with an amine (II) wherein R¹ and R² are defined as in claim 12 under basic conditions in a solvent affording amide (III); wherein R¹, R², R³, R⁴, X and Y are defined as above; or 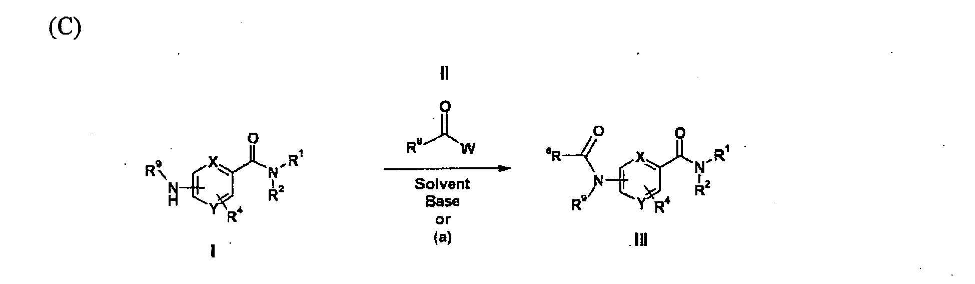
(i) an amide derivative (I) wherein R¹, R², R⁴, R⁹, X and Y are defined as in claim 12 is reacted with an acid derivative (II) wherein W is halo, R⁷(C=O)O-, C₁₋C₆alkyloxy or arylC₁₋C₆alkyloxy, R⁷ is C₁₋C₆alkyl or arylC₁₋ C₆alkyl and R⁶ is C₃-C₁₀cycloalkyl, C₃₋C₁₀hetcycloalkyl, aryl, hetaryl, R¹⁴C₁₋ C₆alkylcarbonyl-, C₁-C₆alkylS(O)ₙ-, arylS(O)ₙ- or arylC₁-C₆alkylS(O)ₙ-, wherein the alkyl, cycloalkyl, hetcycloalkyl, aryl groups optionally are substituted with one or more R¹¹ as defined in claim 12 under basic conditions in a solvent affording amide (III); wherein R¹, R², R⁴, R⁹, X and Y are defined as in claim 12 and R⁶ is C₃-C₁₀cycloalkyl, C₃₋C₁₀hetcycloalkyl, aryl, hetaryl, R¹⁴ C₁₋C₆alkyl-carbonyl-, C₁₋C₆alkylS(O)ₙ-, arylS(O)ₙ- or arylC₁₋C₆alkylS(O)ₙ-, wherein the alkyl, cycloalkyl, hetcycloalkyl, aryl groups optionally are substituted with one or more R¹¹ as defined above; or
(ii) when W is hydroxy the acid derivative (II) wherein R⁶ is as defined above is coupled with an amine (I) wherein R¹, R², R⁴, R⁹, X and Y are defined as above under standard amide forming conditions using a coupling reagent (a) affording amide (III) wherein R¹, R², R⁴, R⁹, X and Y are defined as above and R⁶ is C₃₋C₁₀cycloalkyl, C₃₋C₁₀hetcyclo-alkyl, aryl, hetaryl, R¹⁴ C₁₋ C₆alkylcarbonyl-, C₁₋C₆alkylS(O)ₙ-, arylS(O)ₙ- or arylC₁₋C₆alkylS(O)ₙ-,
wherein the alkyl, cycloalkyl, hetcycloalkyl, aryl groups optionally are substituted with one or more R¹¹ as defined above; or 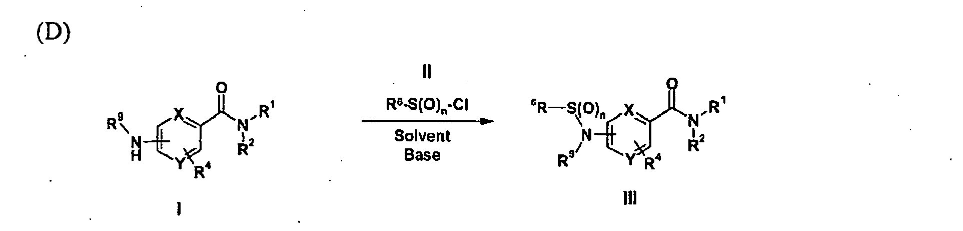 an amide derivative (I) wherein R¹, R², R⁴, R⁹, X and Y are defined as in claim 12 is reacted with a sulfinyl (n = 1) or sulfonyl (n = 2) chloride derivative (II) wherein R⁶ is C₁₋C₆alkyl, C₃₋C₁₀cycloalkyl, C₃₋C₁₀hetcycloalkyl, aryl, hetaryl or arylC₁₋C₆alkyl, wherein the alkyl, cycloalkyl, hetcycloalkyl, aryl and hetaryl groups optionally are substituted with one or more R¹¹ as defined in claim 12 under basic conditions in a solvent to afford an amide (III); wherein R¹, R², R⁴, R⁹, n, X and Y are defined as above and R⁶ is C₁₋C₆alkyl, C₃₋C₁₀cyloalkyl, C₃₋C₁₀hetcycloalkyl, aryl, hetaryl or arylC₁₋C₆alkyl, wherein the alkyl, cycloalkyl, hetcycloalkyl, aryl and hetaryl groups optionally are substituted with one or more R¹¹ as defined above; or 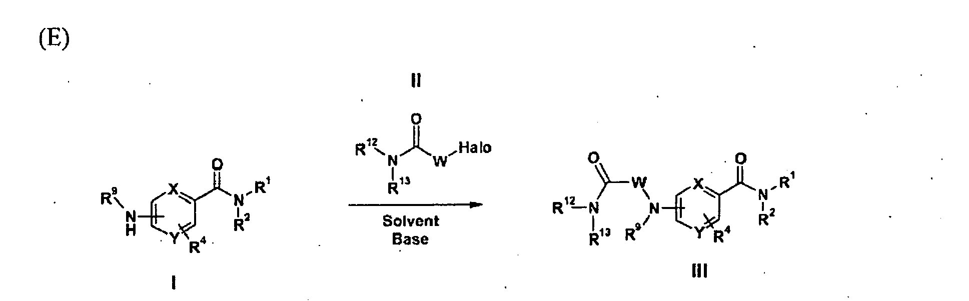 an amide derivative (I) wherein R¹, R², R⁴, R⁹, X and Y are defined as in claim 12 is reacted with an amido alkyl halide derivative (II) wherein R¹², R¹³ and halo are defined in claim 12 and W is C₁₋C₆alkyl, wherein the alkyl group optionally is substituted with one or more R¹¹ as defined in claim 12 under basic conditions in a solvent to afford an amide (III); wherein R¹, R², R⁴, R⁹, R¹², R¹³, X and Y are defined as above and W is C₁₋C₆alkyl, wherein the alkyl group optionally is substituted with one or more R¹¹ as defined above; or 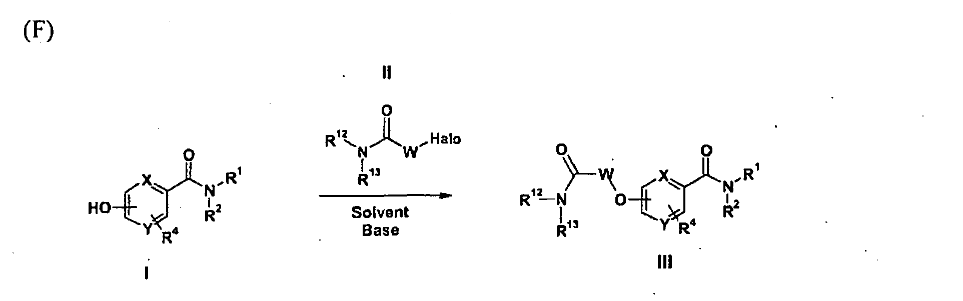 an amide derivative (I) wherein R¹, R², R⁴, X and Y are defined as in claim 12 is reacted with an amido alkyl halide derivative (II) wherein R¹², and R¹³ are defined in claim 12 and W is C₁₋C₆alkyl, wherein the alkyl group optionally is substituted with one or more R¹¹ as defined in claim 12 under basic conditions in a solvent to afford an amide (III); wherein R¹, R², R⁴, R¹², R¹³, X and Y are defined as above and W is C₁₋C₆alkyl, wherein the alkyl group optionally is substituted with one or more R¹¹ as defined above;
and optionally converting the product of any of (A) to (F) into a pharmaceutically acceptable salt.

## Patentansprüche

1. Verbindung der allgemeinen Formel (I): 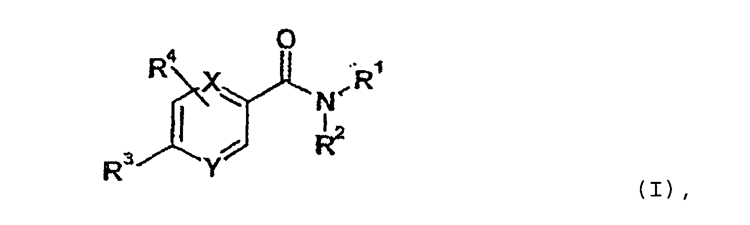 in der
R¹ und R² zusammen mit dem Stickstoff, an den sie angelagert sind, 6-Azabicyclo[3.2.1]octan sind, das gegebenenfalls mit mindestens einem C₁-C₆-Alkyl substituiert ist;
R³ C₁-C₆-Alkyl, -NR⁸R⁹, -C(=O)NR⁸R⁹ oder -OR¹⁰ ist, wobei die Alkylgruppe gegebenenfalls mit einem oder mehreren R¹¹ gegebenenfalls substituiert ist;
R⁴ Wasserstoff, Halogen, Hydroxy, Cyano, Trihalogenmethyl oder C₁-C₆-Alkyl ist;
R⁸ Wasserstoff, C₁-C₆-Alkyl, C₃-C₁₀-Cycloalkyl, C₃-C₁₀-Cycloalkyl-C₁-C₆-alkyl, C₁-C₆-Alkyloxy-C₁-C₆-alkyl, Aryl-C₁-C₆-alkyloxy-C₁-C₆-alkyl oder C₂-C₆-Alkenyl ist;
R⁹ C₁-C₆-Alkyl, C₃-C₁₀-Cycloalkyl, C₃-C₁₀-Cycloalkyl-C₁-C₆-alkyl, C₃-C₁₀-Cycloalkylcarbonyl-, C₃-C₁₀-Hetcycloalkylcarbonyl-, Arylcarbonyl-, Hetarylcarbonyl-, C₁-C₆-Alkyloxy-C₁-C₆-alkyl, NR¹²R¹³-Carbonyl-C₁-C₆-alkyl-, R¹⁴C₁-C₆-Alkylcarbonyl-, -COR¹⁵, C₁-C₆-Alkyl-S(O)ₙ-, Aryl-S(O)ₙ-, Aryl-C₁-C₆-alkyl-S(O)ₙ-, Aryl-C₁-C₆-alkyl oder Hetaryl-C₁-C₆-alkyl ist, wobei die Alkyl-, Cycloalkyl-, Aryl- und Hetarylgruppen unabhängig gegebenenfalls mit einem oder mehreren R¹¹ substituiert sind;
R¹⁰ C₁-C₆-Alkyl, Aryl-C₁-C₆-alkyl oder NR¹²R¹³-Carbonyl-C₁-C₆-alkyl ist, wobei die Alkyl- und Arylgruppen unabhängig gegebenenfalls mit einem oder mehreren R¹¹ substituiert sind;
R¹¹ R⁵, R⁶, Halogen, Hydroxy, Oxo, Cyano, -COR¹⁵, C₁-C₈-Alkyl, C₁-C₈-Alkyloxy, C₃-C₁₀-Cycloalkyl, Trihalogenmethyl, Trihalogenmethyloxy, Aryl, Aryl-C₁-C₆-alkyl, C₁-C₆-Alkyloxy-C₁-C₆-alkyl, Aryloxy-C₁-C₆-alkyl, Aryl-C₁-C₆-alkyloxy-C₁-C₆-alkyl, Hetaryl, Hetaryl-C₁-C₆-alkyl, Hetaryloxy-C₁-C₆-alkyl, Hetaryl-C₁-C₆-alkyloxy-C₁-C₆-alkyl, -NR¹²R¹³, -SO²NR¹²R¹³, NR¹²R¹³-Carbonyl-C₁-C₆-alkyl, R¹⁶-Carbonyl-N(R¹²)-, Aryl-S(O)ₙ-, Hetaryl-S(O)ₙ- oder R¹⁷S(O)ₙN(R¹²)- ist, wobei die Aryl- und Hetarylgruppen unabhängig gegebenenfalls mit einem oder mehreren R¹⁸ substituiert sind;
R¹² und R¹³ unabhängig Wasserstoff, C₁-C₈-Alkyl, C₃-C₁₀-Cycloalkyl, C₃-C₁₀-Cycloalkyl-C₁-C₆-alkyl, C₃-C₁₀-Hetcycloalkyl, Aryl, Hetaryl, Aryl-C₁-C₆-alkyl oder Hetaryl-C₁-C₆-alkyl ist, wobei die Alkyl-, Aryl- und Hetarylgruppen unabhängig gegebenenfalls mit einem oder mehreren R¹⁸ substituiert sind; oder
R¹² und R¹³ zusammen mit dem Stickstoff, an den sie angelagert sind, ein gesättigtes oder teilgesättigtes cyclisches, bicyclisches oder tricyclisches Ringsystem bilden, das von 4 bis 12 Kohlenstoffatome und von 0 bis 2 zusätzliche Heteroatome, die aus Stickstoff oder Sauerstoff ausgewählt sind, enthält, wobei das Ringsystem gegebenenfalls mit mindestens einem R⁵, R⁵-Oxy-, R⁶, Halogen, Cyano, Hydroxy, Oxo, C₁-C₈-Alkyl, Aryl, Hetaryl, Aryl-C₁-C₆-alkyl, Hetaryl-C₁-C₆-alkyl, C₁-C₆-Alkyloxy-C₁-C₆-alkyl, NR¹²R¹³-Carbonyl-C₁-C₆-alkyl, NR¹²R¹³C₁-C₆-Alkylcarbonyl-, R¹⁴C₁-C₆-Alkylcarbonyl- oder -COR¹⁵ substituiert ist;
R¹⁴ C₁-C₆-Alkyloxy, C₃-C₁₀-Cycloalkyloxy-, C₃-C₁₀-Cycloalkyl-C₁-C₆-alkyloxy-, C₃-C₁₀-Hetcycloalkyloxy-, Aryl, Hetaryl, Aryl-C₁-C₆-alkyloxy, Hetaryl-C₁-C₆-alkyloxy, -NR¹²R¹³, -COR¹⁵ ist, wobei die Alkyl-, Cycloalkyl-, Hetcycloalkyl-, Aryl- und Hetarylgruppen unabhängig gegebenenfalls mit einem oder mehreren R²⁰ substituiert sind;
R¹⁵ C₁-C₆-Alkyl, Hydroxy, C₁-C₈-Alkyloxy, -NR¹²R¹³, Aryl, Aryloxy oder Aryl-C₁-C₆-alkyloxy ist;
R¹⁶R⁶, C₁-C₆-Alkyl, C₂-C₆-Alkenyl, Aryl, Aryl-C₁-C₆-alkyl, Hetaryl, Hetaryl-C₁-C₆-alkyl, C₃-C₁₀-Cycloalkyl, C₃-C₁₀-Hetcycloalkyl, Aryl-C₁-C₆-alkyloxy-C₁-C₆-alkyl-, Hetaryl-C₁-C₆-alkyloxy-C₁-C₆-alkyl- oder R¹²R¹³NC₁-C₆-Alkyl- ist, wobei die Alkyl-, Alkenyl-, Cycloalkyl-, Hetcycloalkyl-, Aryl- und Hetarylgruppen gegebenenfalls mit R¹⁹ substituiert sind;
R¹⁷ C₁-C₆-Alkyl, C₃-C₁₀-Cycloalkyl, C₃-C₁₀-Hetcycloalkyl, Aryl, Aryl-C₁-C₆-alkyl, Hetaryl, Hetaryl-C₁-C₆-alkyl ist;
R¹⁸R⁶, -NR¹²R¹³, Oxo, C₁-C₆-Alkyl, C₃-C₁₀-Cycloalkyl oder C₃-C₁₀-Hetcycloalkyl ist, wobei die Alkyl-, Cycloalkyl-, Hetcycloalkyl-, Aryl- und Hetarylgruppen unabhängig gegebenenfalls mit einem oder mehreren R⁷ substituiert sind;
R¹⁹ Wasserstoff, Halogen, Hydroxy, Oxo, Nitro, Cyano oder -COR¹⁵ ist;
R²⁰ Wasserstoff, C₁-C₈-Alkyl, -NR¹²R¹³, C₁-C₆-Alkyloxy oder Aryl-C₁-C₆-alkyl ist;
R⁵ C₁-C₆-Alkylcarbonyl-, C₃-C₁₀-Cycloalkylcarbonyl-, C₃-C₁₀-Cycloalkyl-C₁-C₆-alkylcarbonyl-, Arylcarbonyl-, Aryl-C₁-C₆-alkylcarbonyl-, Hetarylcarbonyl- oder Hetaryl-C₁-C₆-alkylcarbonyl- ist;
R⁶ C₁-C₆-Alkyloxy-, Aryloxy-, Aryl-C₁-C₆-alkyloxy-, Hetaryloxy- oder Hetaryl-C₁-C₆-alkyloxy- ist;
R⁷ Wasserstoff, C₁-C₈-Alkyl, C₁-C₆-Alkyloxy oder Aryl-C₁-C₆-alkyl ist;
X und Y unabhängig -CH- oder Stickstoff sind;
n 1 oder 2 ist; oder
ein Salz davon mit einer pharmazeutisch unbedenklichen Säure oder Base oder ein beliebiges optisches Isomer oder eine Mischung von optischen Isomeren, einschließlich einer racemischen Mischung, oder beliebige tautomere Formen zur Verwendung als ein Arzneimittel.

2. Verbindung zur Verwendung als ein Arzneimittel nach Anspruch 1, in der X und Y -CH- sind.

3. Verbindung zur Verwendung als ein Arzneimittel nach Anspruch 1 oder 2, in der R³ -NR⁸R⁹ ist, wobei R⁸ und R⁹ wie in Anspruch 1 definiert sind.

4. Verbindung zur Verwendung als ein Arzneimittel nach einem der Ansprüche 1 - 3, in der R⁴ Wasserstoff oder Halogen ist.

5. Verbindung zur Verwendung als ein Arzneimittel nach einem der Ansprüche 1 - 4, die aus der Gruppe ausgewählt ist, die aus folgenden Verbindungen besteht: [4-(1,3,3-Trimethyl-6-azabicyclo[3.2.1]octan-6-carbonyl)phenyl]carbaminsäure-*tert*.-butylester;
(4-Aminophenyl)-(1,3,3-trimethyl-6-azabicyclo[3.2.1]oct-6-yl)methanon;
(4-Methylaminophenyl)-(1,3,3-trimethyl-6-azabicyclo[3.2.1]oct-6-yl)methanon;
N-Methyl-N-[4-(1,3,3-trimethyl-6-azabicyclo[3.2.1]octan-6-carbonyl)phenyl]methansulfonamid;
N-Methyl-N-[4-(1,3,3-trimethyl-6-azabicyclo[3.2.1]octan-6-carbonyl)phenyl]benzolsulfonamid;
N-Methyl-C-phenyl-N-[4-(1,3,3-trimethyl-6-azabicyclo[3.2.1]octan-6-carbonyl)phenyl]methansulfonamid;
N-[4-(1,3,3-Trimethyl-6-azabicyclo[3.2.1]octan-6-carbonyl)phenyl]methansulfonamid;
N-Ethyl-N-[4-(1,3,3-trimethyl-6-azabicyclo[3.2.1]octan-6-carbonyl)phenyl]methansulfonamid;
N-Cyclopropylmethyl-N-[4-(1,3,3-trimethyl-6-azabicyclo[3.2.1]octan-6-carbonyl)phenyl]methansulfonamid;
N-[4-(1,3,3-Trimethyl-6-azabicyclo[3.2.1]octan-6-carbonyl)phenyl]isonicotinamid; 2,4-Dichlor-N-[4-(1,3,3-trimethyl-6-azabicyclo[3.2.1]octan-6-carbonyl)phenyl]benzamid;
2,4-Dimethoxy-N-[4-(1,3,3-trimethyl-6-azabicyclo[3.2.1]octan-6-carbonyl)phenyl]benzamid;
4-Trifluormethoxy-N-[4-(1,3,3-trimethyl-6-azabicyclo[3.2.1]octan-6-carbonyl)phenyl]benzamid;
3,4-Dimethoxy-N-[4-(1,3,3-trimethyl-6-azabicyclo[3.2.1]octan-6-carbonyl)phenyl]benzamid;
3,5-Dimethyl-N-[4-(1,3,3-trimethyl-6-azabicyclo[3.2.1]octan-6-carbonyl)phenyl]benzamid;
3-Trifluormethoxy-N-[4-(1,3,3-trimethyl-6-azabicyclo[3.2.1]octan-6-carbonyl)phenyl]benzamid;
3,5-Dimethoxy-N-[4-(1,3,3-trimethyl-6-azabicyclo[3.2.1]octan-6-carbonyl)phenyl]benzamid;
3-Cyano-N-[4-(1,3,3-trimethyl-6-azabicyclo[3.2.1]octan-6-carbonyl)phenyl]benzamid;
N-[4-(1,3,3-Trimethyl-6-azabicyclo[3.2.1]octan-6-carbonyl)phenyl]benzolsulfonamid;
Phenyl-N-[4-(1,3,3-trimethyl-6-azabicyclo[3.2.1]octan-6-carbonyl)phenyl]methansulfonamid;
[4-(1,3,3-Trimethyl-6-azabicyclo[3.2.1]octan-6-carbonyl)phenyl]butan-1-sulfonsäureamid;
3-Trifluormethyl-N-[4-(1,3,3-trimethyl-6-azabicyclo[3.2.1]octan-6-carbonyl)phenyl]benzolsulfonamid;
N-[4-(1,3,3-Trimethyl-6-azabicyclo[3.2.1]octan-6-carbonyl)phenyl]isophthalamidsäuremethylester;
N-[4-(1,3,3-Trimethyl-6-azabicyclo[3.2.1]octan-6-carbonyl)phenyl]isophthalamidsäure;
[4-(1,3,3-Trimethyl-6-azabicyclo[3.2.1]octan-6-carbonyl)phenyl]-4-methoxycyclohexancarbonsäureamid;
6-[4-(1,3,3-Trimethyl-6-azabicyclo[3.2.1]octan-6-carbonyl)phenylcarbamoyl]nicotinsäuremethylester; 6-[4-(1,3,3-Trimethyl-6-azabicyclo[3.2.1]octan-6-carbonyl)phenylcarbamoyl]nicotinsäure;
N-[4-(1,3,3-Trimethyl-6-azabicyclo[3.2.1]octan-6-carbonyl)phenyl]malonamidsäureethylester;
N-[4-(1,3,3-Trimethyl-6-azabicyclo[3.2.1]octan-6-carbonyl)phenyl]malonamidsäure-*tert*.-butylester;
3-[4-(1,3,3-Trimethyl-6-azabicyclo[3.2.1]octan-6-carbonyl)phenylcarbamoyl]cyclohexancarbonsäuremethylester;
3-[4-(1,3,3-Trimethyl-6-azabicyclo[3.2.1]octan-6-carbonyl)phenylcarbamoyl]cyclohexancarbonsäure;
N-[4-(1,3,3-Trimethyl-6-azabicyclo[3.2.1]octan-6-carbonyl)phenyl]benzamid;
N-[4-(1,3,3-Trimethyl-6-azabicyclo[3.2.1]octan-6-carbonyl)phenyl]isonicotinamid;
N-[4-(1,3,3-Trimethyl-6-azabicyclo[3.2.1]octan-6-carbonyl)phenyl]nicotinamid;
[4-(1,3,3-Trimethyl-6-azabicyclo[3.2.1]octan-6-carbonyl)phenyl]pyridin-2-carbonsäureamid;
[4-(1,3,3-Trimethyl-6-azabicyclo[3.2.1]octan-6-carbonyl)phenyl]-1-acetylpiperidin-4-carbonsäureamid;
N-[4-(1,3,3-Trimethyl-6-azabicyclo[3.2.1]octan-6-carbonyl)phenyl]succinamidsäuremethylester;
N-[4-(1,3,3-Trimethyl-6-azabicyclo[3.2,1]octan-6-carbonyl)phenyl]succinamidsäure;
N-[4-(1,3,3-Trimethyl-6-azabicyclo[3.2.1]octan-6-carbonyl)phenyl]malonamidsäure;
2-Amino-N-[4-(1,3,3-trimethyl-6-azabicyclo[3.2.1]octan-6-carbonyl)phenyl]acetamid;
2-Acetylamino-N-[4-(1,3,3-trimethyl-6-azabicyclo[3.2.1]octan-6-carbonyl)phenyl]succinamidsäuremethylester;
2-Acetylamino-N-[4-(1,3,3-trimethyl-6-azabicyclo[3.2.1]octan-6-carbonyl)phenyl]succinamidsäure;
N-Methyl-N'-[4-(1,3,3-trimethyl-6-azabicyclo[3.2.1]octan-6-carbonyl)phenyl]succinamid;
N,N-Dimethyl-N'-[4-(1,3,3-trimethyl-6-azabicyclo[3.2.1]octan-6-carbonyl)phenyl]succinamid;
N-[5-(1,3,3-Trimethyl-6-azabicyclo[3.2.1]octan-6-carbonyl)pyridin-2-yl]benzamid;
N-Methyl-N-[4-(1,3,3-trimethyl-6-azabicyclo[3.2.1]octan-6-carbonyl)phenyl]benzamid;
3,5,N-Trimethyl-N-[4-(1,3,3-trimethyl-6-azabicyclo[3.2.1]octan-6-carbonyl)phenyl]benzamid;
2,4-Dimethoxy-N-methyl-N-[4-(1,3,3-trimethyl-6-azabicyclo[3.2.1]octan-6-carbonyl)phenyl]benzamid;
3,5-Dichlor-N-methyl-N-[4-(1,3,3-trimethyl-6-azabicyclo[3.2.1]octan-6-carbonyl)phenyl]benzamid;
4-Brom-N,N-dimethyl-N-[4-(1,3,3-trimethyl-6-azabicyclo[3.2.1]octan-6-carbonyl)phenyl]benzamid;
N-Methyl-N-[4-(1,3,3-trimethyl-6-azabicyclo[3.2.1]octan-6-carbonyl)phenyl]isonicotinamid;
N-Methyl-N-[4-(1,3,3-trimethyl-6-azabicyclo[3.2.1]octan-6-carbonyl)phenyl]nicotinamid;
2-Piperidin-1-yl-N-[4-(1,3,3-trimethyl-6-azabicyclo[3.2.1]octan-6-carbonyl)phenyl]acetamid;
2-Morpholin-4-yl-N-[4-(1,3,3-trimethyl-6-azabicyclo[3.2.1]octan-6-carbonyl)phenyl]acetamid;
2-(4-Methylpiperazin-1-yl)-N-[4-(1,3,3-trimethyl-6-azabicyclo[3.2.1]octan-6-carbonyl)phenyl]acetamid;
2-Dimethylamino-N-[4-(1,3,3-trimethyl-6-azabicyclo[3.2.1]octan-6-carbonyl)phenyl]acetamid;
2-[(2-Dimethylaminoethyl)methylamino]-N-[4-(1,3,3-trimethyl-6-azabicyclo[3.2.1]octan-6-carbonyl)phenyl]acetamid;
2-(3,4-Dihydro-2H-chinolin-1-yl)-N-[4-(1,3,3-trimethyl-6-azabicyclo[3.2.1]octan-6-carbonyl)phenyl]acetamid;
2-(4-Acetylpiperidin-1-yl)-N-[4-(1,3,3-trimethyl-6-azabicyclo[3.2.1]octan-6-carbonyl)phenyl]acetamid;
2-(4-Dimethylaminopiperidin-1-yl)-N-[4-(1,3,3-trimethyl-6-azabicyclo[3.2.1]octan-6-carbonyl)phenyl]acetamid;
2-(4-Oxopiperidin-1-yl)-N-[4-(1,3,3-trimethyl-6-azabicyclo[3.2.1]octan-6-carbonyl)phenyl]acetamid;
2-[Methyl-(1-methylpiperidin-4-yl)amino]-N-[4-(1,3,3-trimethyl-6-azabicyclo[3.2.1]octan-6-carbonyl)phenyl]acetamid;
2-(3,6-Dihydro-2H-pyridin-1-yl)-N-[4-(1,3,3-trimethyl-6-azabicyclo[3.2.1]octan-6-carbonyl)phenyl]acetamid;
1-{[4-(1,3,3-Trimethyl-6-azabicyclo[3.2,1]octan-6-carbonyl)phenylcarbamoyl]methyl}piperidin-4-carbonsäureethylester;
N-[4-(1,3,3-Trimethyl-6-azabicyclo[3.2.1]octan-6-carbonyl)phenyl]-2-(1,3,3-trimethyl-6-azabicyclo[3.2.1]oct-6-yl)acetamid;
2-(4-Acetylpiperazin-1-yl)-N-[4-(1,3,3-trimethyl-6-azabicyclo[3.2.1]octan-6-carbonyl)phenyl]acetamid;
2-(2,3,5,6-Tetrahydro-[1,2']-bipyrazinyl-4-yl)-N-[4-(1,3,3-trimethyl-6-azabicyclo[3.2.1]octan-6-carbonyl)phenyl]acetamid;
2-(Cyclohexylmethylamino)-N-[4-(1,3,3-trimethyl-6-azabicyclo[3.2.1]octan-6-carbonyl)phenyl]acetamid;
2-(2,4-Dimethoxybenzyloxy)-N-[4-(1,3,3-trimethyl-6-azabicyclo[3.2.1]octan-6-carbonyl)phenyl]acetamid;
2-Benzyloxy-N-[4-(1,3,3-trimethyl-6-azabicyclo[3.2.1]octan-6-carbonyl)phenyl]acetamid;
2-(3,4-Dihydro-1H-isochinolin-2-yl)-N-[4-(1,3,3-trimethyl-6-azabicyclo[3.2.1]octan-6-carbonyl)phenyl]acetamid;
2-(6-Azabicyclo[3.2.1]oct-6-yl)-N-[4-(1,3,3-trimethyl-6-azabicyclo[3.2.1]octan-6-carbonyl)phenyl]acetamid;
2-Azepan-1-yl-N-[4-(1,3,3-trimethyl-6-azabicyclo[3.2.1]octan-6-carbonyl)phenyl]acetamid;
2-(6,7-Dimethoxy-3,4-dihydro-1H-isochinolin-2-yl)-N-[4-(1,3,3-trimethyl-6-azabicyclo[3.2.1]octan-6-carbonyl)phenyl]acetamid;
2-Indol-1-yl-N-[4-(1,3,3-trimethyl-6-azabicyclo[3.2.1]octan-6-carbonyl)phenyl]acetamid;
2-Benzoimidazol-1-yl-N-[4-(1,3,3-trimethyl-6-azabicyclo[3.2.1]octan-6-carbonyl)phenyl]acetamid;
2-(2,3-Dihydroindol-1-yl)-N-[4-(1,3,3-trimethyl-6-azabicyclo[3.2.1]octan-6-carbonyl)phenyl]acetamid;
2-Benzotriazol-1-yl-N-[4-(1,3,3-trimethyl-6-azabicyclo[3.2.1]octan-6-carbonyl)phenyl]acetamid;
2-(4-Benzoylpiperidin-1-yl)-N-[4-(1,3,3-trimethyl-6-azabicyclo[3.2.1]octan-6-carbonyl)phenyl]acetamid;
2-(4-*tert*.-Butylpiperidin-1-yl)-N-[4-(1,3,3-trimethyl-6-azabicyclo[3.2.1]octan-6-carbonyl)phenyl]acetamid;
2-(4-Phenylpiperidin-1-yl)-N-[4-(1,3,3-trimethyl-6-azabicyclo[3.2.1]octan-6-carbonyl)phenyl]acetamid;
2-(4-Hydroxy-4-phenylpiperidin-1-yl)-N-[4-(1,3,3-trimethyl-6-azabicyclo[3.2.1]octan-6-carbonyl)phenyl]acetamid;
2-(4-Pyridin-2-ylpiperazin-1-yl)-N-[4-(1,3,3-trimethyl-6-azabicyclo[3.2.1]octan-6-carbonyl)phenyl]acetamid;
2-(4-Phenylpiperazin-1-yl)-N-[4-(1,3,3-trimethyl-6-azabicyclo[3.2.1]octan-6-carbonyl)phenyl]acetamid;
2-(4-Pyrimidin-2-ylpiperazin-1-yl)-N-[4-(1,3,3-trimethyl-6-azabicyclo[3.2.1]octan-6-carbonyl)phenyl]acetamid;
2-(4-Acetyl-4-phenylpiperidin-1-yl)-N-[4-(1,3,3-trimethyl-6-azabicyclo[3.2.1]octan-6-carbonyl)phenyl]acetamid;
2-(4-Cyano-4-phenylpiperidin-1-yl)-N-[4-(1,3,3-trimethyl-6-azabicyclo[3.2.1]octan-6-carbonyl)phenyl]acetamid;
2-(4-Pyridin-4-ylpiperazin-1-yl)-N-[4-(1,3,3-trimethyl-6-azabicyclo[3.2.1]octan-6-carbonyl)phenyl]acetamid;
2-[4-(2-Pyrrolidin-1-ylacetyl)piperazin-1-yl]-N-[4-(1,3,3-trimethyl-6-azabicyclo[3.2.1]octan-6-carbonyl)phenyl]acetamid;
2-(Piperidin-4-yloxy)-N-[4-(1,3,3-trimethyl-6-azabicyclo[3.2.1]octan-6-carbonyl)phenyl]acetamid;
2-(2H-Tetrazol-5-yl)-N-[4-(1,3,3-trimethyl-6-azabicyclo[3.2.1]octan-6-carbonyl)phenyl]acetamid;
3-Piperidin-1-yl-N-[4-(1,3,3-trimethyl-6-azabicyclo[3.2.1]octan-6-carbonyl)phenyl]propionamid;
3-Benzylamino-N-[4-(1,3,3-trimethyl-6-azabicyclo[3.2.1]octan-6-carbonyl)phenyl]propionamid;
2-(3,4-Dihydro-2H-chinolin-1-yl)-N-methyl-N-[4-(1,3,3-trimethyl-6-azabicyclo[3.2.1]octan-6-carbonyl)phenyl]acetamid;
1-Morpholin-4-yl-2-[4-(1,3,3-trimethyl-6-azabicyclo[3.2.1]octan-6-carbonyl)phenylamino]ethanon;
[4-(2-Piperidin-1-ylethylamino)phenyl]-(1,3,3-trimethyl-6-azabicyclo[3.2.1]oct-6-yl)methanon;
N-Methyl-N-phenyl-2-[4-(1,3,3-trimethyl-6-azabicyclo[3.2.1]octan-6-carbonyl)phenylamino]acetamid;
N-(2-Methoxyethyl)-N-[4-(1,3,3-trimethyl-6-azabicyclo[3.2.1]octan-6-carbonyl)phenyl]benzamid;
N-Allyl-N-[4-(1,3,3-trimethyl-6-azabicyclo[3.2.1]octan-6-carbonyl)phenyl]benzamid;
[4-(2-Methoxyethylamino)phenyl]-(1,3,3-trimethyl-6-azabicyclo[3.2.1]oct-6-yl)methanon;
(4-Benzyloxyphenyl)-(1,3,3-trimethyl-6-azabicyclo[3.2.1]oct-6-yl)methanon;
3-[4-(1,3,3-Trimethyl-6-azabicyclo[3.2.1]octan-6-carbonyl)benzoylamino]propionsäuremethylester;
3-[4-(1,3,3-Trimethyl-6-azabicyclo[3.2.1]octan-6-carbonyl)benzoylamino]propionsäure;
(4-Benzyloxyphenyl)-(1,3,3-trimethyl-6-azabicyclo[3.2.1]oct-6-yl)methanon;
oder ein Salz davon mit einer pharmazeutisch unbedenklichen Säure oder Base oder ein beliebiges optisches Isomer oder eine Mischung von optischen Isomeren, einschließlich einer racemischen Mischung, oder beliebige tautomere Formen.

6. Verbindung nach einem der Ansprüche 1 - 5 zur Behandlung, Prävention und/oder Prophylaxe beliebiger Leiden, Erkrankungen und Krankheiten, bei denen eine Modulation oder eine Hemmung der Aktivität von 11βHSD1 förderlich ist.

7. Verbindung nach Anspruch 6, wobei das Leiden, die Erkrankung oder die Krankheit aus Stoffwechselsyndrom, Insulinresistenz, Dyslipidämie, Hypertonie, Fettleibigkeit, Typ-2-Diabetes, pathologischer Glukosetoleranz (IGT), gestörter Fastenglukose (IFG), latentem Autoimmundiabetes im Erwachsenenalter (LADA), Typ-1-Diabetes, diabetischen Spätkomplikationen, einschließlich Herz-Kreislauf-Erkrankungen, Herz-Kreislauf-Erkrankungen, Fettstoffwechselstörungen, neurodegenerativen und psychiatrischen Erkrankungen, Dysregulation des intraokulären Drucks, einschließlich Glaukom, Immunerkrankungen, unzureichenden Immunantworten, Erkrankungen der Skelettmuskulatur, Erkrankungen des Magen-Darm-Trakts, Stein-Leventhal-Syndrom, verringertem Haarwachstum oder anderen Erkrankungen, Erkrankungen oder Leiden, die von intrazellulären Glukokortikoidspiegeln beeinflusst werden, ungünstigen Auswirkungen erhöhter Plasmaspiegel von endogenem aktivem Glukokortikoid, Morbus Cushing, Cushing-Syndrom, ungünstigen Auswirkungen der Behandlung von Autoimmunerkrankungen mit Glukokortikoidrezeptor-Agonist, ungünstigen Auswirkungen der Behandlung von Entzündungserkrankungen mit Glukokortikoidrezeptor-Agonist, ungünstigen Auswirkungen der Behandlung von Erkrankungen mit einer inflammatorischen Komponente mit Glukokortikoidrezeptor-Agonist, ungünstigen Auswirkungen der Behandlung mit Glukokortikoidrezeptor-Agonist als Teil einer Krebschemotherapie, ungünstigen Auswirkungen der Behandlung von operativem/postoperativem oder einem anderen Trauma mit Glukokortikoidrezeptor-Agonist, ungünstigen Auswirkungen der Behandlung mit Glukokortikoidrezeptor-Agonist im Zusammenhang mit einer Organ- oder Gewebetransplantation oder ungünstigen Auswirkungen der Behandlung von anderen Krankheiten, Erkrankungen oder Leiden mit Glukokortikoidrezeptor-Agonist, bei denen Glukokortikoidrezeptor-Agonisten klinisch günstige Wirkungen bereitstellen, ausgewählt ist.

8. Kombination, die eine wie in einem der Ansprüche 1 - 5 definierte Verbindung und eine oder mehrere pharmazeutische Wirkstoffe umfasst.

9. Kombination nach Anspruch 8, wobei die weiteren Wirkstoffe aus Fettleibigkeit bekämpfenden Mitteln, Antidiabetika, Mitteln, die die Fettstoffwechsel verändern, Antihypertonika, Glukokortikoidrezeptor-Agonisten, Mitteln zur Behandlung und/oder Prävention von Komplikationen, die aus Diabetes resultieren oder damit assoziiert werden, und Mitteln zur Behandlung und/oder Prävention von Komplikationen und Erkrankungen, die aus Fettleibigkeit resultieren oder damit assoziiert werden, ausgewählt sind.

10. Pharmazeutische Zusammensetzung, die eine wie in einem der Ansprüche 1 - 5 definierte Verbindung oder eine wie in Anspruch 8 oder 9 definierte Kombination zusammen mit einem oder mehreren pharmazeutisch unbedenklichen Trägerstoffen oder Verdünnungsmitteln umfasst.

11. Kombination nach Anspruch 8 oder 9 oder eine wie in Anspruch 10 definierte Zusammensetzung zur Verwendung als ein Arzneimittel.

12. Verbindung der allgemeinen Formel (I): 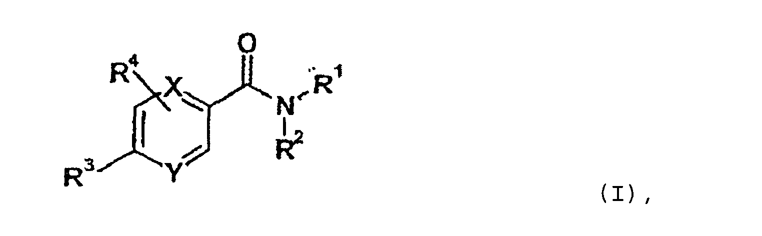 in der
R¹ und R² zusammen mit dem Stickstoff, an den sie angelagert sind, 6-Azabicyclo[3.2.1]octan sind, das gegebenenfalls mit mindestens einem C₁-C₆-Alkyl substituiert ist;
R³ C₁-C₆-Alkyl, -NR⁸R⁹, -C(=O)NR⁸R⁹ oder -OR¹⁰ ist, wobei die Alkylgruppe gegebenenfalls mit einem oder mehreren R¹¹ gegebenenfalls substituiert ist;
R⁴ Wasserstoff, Halogen, Hydroxy, Cyano, Trihalogenmethyl oder C₁-C₆-Alkyl ist;
R⁸ Wasserstoff, C₁-C₆-Alkyl, C₃-C₁₀-Cycloalkyl, C₃-C₁₀-Cycloalkyl-C₁-C₆-alkyl, C₁-C₆-Alkyloxy-C₁-C₆-alkyl, Aryl-C₁-C₆-alkyloxy-C₁-C₆-alkyl oder C₂-C₆-Alkenyl ist;
R⁹ C₁-C₆-Alkyl, C₃-C₁₀-Cycloalkyl, C₃-C₁₀-Cycloalkyl-C₁-C₆-alkyl, C₃-C₁₀-Cycloalkylcarbonyl-, C₃-C₁₀-Hetcycloalkylcarbonyl-, Arylcarbonyl-, Hetarylcarbonyl-, C₁-C₆-Alkyloxy-C₁-C₆-alkyl, NR¹²R¹³-Carbonyl-C₁-C₆-alkyl-, R¹⁴C₁-C₆-Alkylcarbonyl-, -COR¹⁵, C₁-C₆-Alkyl-S(O)ₙ-, Aryl-S(O)ₙ-, Aryl-C₁-C₆-alkyl-S(O)ₙ-, Aryl-C₁-C₆-alkyl oder Hetaryl-C₁-C₆-alkyl ist, wobei die Alkyl-, Cycloalkyl-, Aryl- und Hetarylgruppen unabhängig gegebenenfalls mit einem oder mehreren R¹¹ substituiert sind;
R¹⁰ C₁-C₆-Alkyl, Aryl-C₁-C₆-alkyl oder NR¹²R¹³-Carbonyl-C₁-C₆-alkyl ist, wobei die Alkyl- und Arylgruppen unabhängig gegebenenfalls mit einem oder mehreren R¹¹ substituiert sind;
R¹¹ R⁵, R⁶, Halogen, Hydroxy, Oxo, Cyano, -COR¹⁵, C₁-C₈-Alkyl, C₁-C₈-Alkyloxy, C₃-C₁₀-Cycloalkyl, Trihalogenmethyl, Trihalogenmethyloxy, Aryl, Aryl-C₁-C₆-alkyl, C₁-C₆-Alkyloxy-C₁-C₆-alkyl, Aryloxy-C₁-C₆-alkyl, Aryl-C₁-C₆-alkyloxy-C₁-C₆-alkyl, Hetaryl, Hetaryl-C₁-C₆-alkyl, Hetaryloxy-C₁-C₆-alkyl, Hetaryl-C₁-C₆-alkyloxy-C₁-C₆-alkyl, -NR¹²R¹³, -SO₂NR¹²R¹³, NR¹²R¹³-Carbonyl-C₁-C₆-alkyl, R¹⁶-Carbonyl-N(R)¹²-, Aryl-S(O)ₙ-, Hetaryl-S(O)ₙ- oder R¹⁷S(O)ₙN(R¹²)- ist, wobei die Aryl- und Hetarylgruppen unabhängig gegebenenfalls mit einem oder mehreren R¹⁸ substituiert sind;
R¹² und R¹³ unabhängig Wasserstoff, C₁-C₈-Alkyl, C₃-C₁₀-Cycloalkyl, C₃-C₁₀-Cycloalkyl-C₁-C₆-alkyl, C₃-C₁₀-Hetcycloalkyl, Aryl, Hetaryl, Aryl-C₁-C₆-alkyl oder Hetaryl-C₁-C₆-alkyl ist, wobei die Alkyl-, Aryl- und Hetarylgruppen unabhängig gegebenenfalls mit einem oder mehreren R¹⁸ substituiert sind; oder
R¹² und R¹³ zusammen mit dem Stickstoff, an den sie angelagert sind, ein gesättigtes oder teilgesättigtes cyclisches, bicyclisches oder tricyclisches Ringsystem bilden, das von 4 bis 12 Kohlenstoffatome und von 0 bis 2 zusätzliche Heteroatome, die aus Stickstoff oder Sauerstoff ausgewählt sind, enthält, wobei das Ringsystem gegebenenfalls mit mindestens einem R⁵, R⁵-Oxy-, R⁶, Halogen, Cyano, Hydroxy, Oxo, C₁-C₆-Alkyl, Aryl, Hetaryl, Aryl-C₁-C₆-alkyl, Hetaryl-C₁-C₆-alkyl, C₁-C₆-Alkyloxy-C₁-C₆-alkyl, NR¹²R¹³-Carbonyl-C₁-C₆-alkyl, NR¹²R¹³C₁-C₆-Alkylcarbonyl-, R¹⁴C₁-C₆-Alkylcarbonyl- oder -COR¹⁵ substituiert ist;
R¹⁴ C₁-C₆-Alkyloxy, C₃-C₁₀-Cycloalkyloxy-, C₃-C₁₀-Cycloalkyl-C₁-C₆-alkyloxy-, C₃-C₁₀-Hetcycloalkyloxy-, Aryl, Hetaryl, Aryl-C₁-C₆-alkyloxy, Hetaryl-C₁-C₆-alkyloxy, -NR¹²R^{l3}, -COR¹⁵ ist, wobei die Alkyl-, Cycloalkyl-, Hetcycloalkyl-, Aryl- und Hetarylgruppen unabhängig gegebenenfalls mit einem oder mehreren R²⁰ substituiert sind;
R¹⁵ C₁-C₆-Alkyl, Hydroxy, C₁-C₈-Alkyloxy, -NR¹²R¹³, Aryl, Aryloxy oder Aryl-C₁-C₆-alkyloxy ist;
R¹⁶ R⁶, C₁-C₆-Alkyl, C₂-C₆-Alkenyl, Aryl, Aryl-C₁-C₆-alkyl, Hetaryl, Hetaryl-C₁-C₆-alkyl, C₃-C₁₀-Cycloalkyl, C₃-C₁₀-Hetcycloalkyl, Aryl-C₁-C₆-alkyloxy-C₁-C₆-alkyl-, Hetaryl-C₁-C₆-alkyloxy-C₁-C₆-alkyl- oder R¹²R¹³NC₁-C₆-Alkyl- ist, wobei die Alkyl-, Alkenyl-, Cycloalkyl-, Hetcycloalkyl-, Aryl- und Hetarylgruppen gegebenenfalls mit R¹⁹ substituiert sind;
R¹⁷ C₁-C₆-Alkyl, C₃-C₁₀-Cycloalkyl, C₃-C₁₀-Hetcycloalkyl, Aryl, Aryl-C₁-C₆-alkyl, Hetaryl, Hetaryl-C₁-C₆-alkyl ist;
R¹⁸ R⁶, -NR¹²R¹³, Oxo, C₁-C₆-Alkyl, C₃-C₁₀-Cycloalkyl oder C₃-C₁₀-Hetcycloalkyl ist, wobei die Alkyl-, Cycloalkyl-, Hetcycloalkyl-, Aryl- und Hetarylgruppen unabhängig gegebenenfalls mit einem oder mehreren R⁷ substituiert sind;
R¹⁹ Wasserstoff, Halogen, Hydroxy, Oxo, Nitro, Cyano oder -COR¹⁵ ist;
R²⁰ Wasserstoff, C₁-C₈-Alkyl, -NR¹²R¹³, C₁-C₆-Alkyloxy oder Aryl-C₁-C₆-alkyl ist;
R⁵ C₁-C₆-Alkylcarbonyl-, C₃-C₁₀-Cycloalkylcarbonyl-, C₃-C₁₀-Cycloalkyl-C₁-C₆-alkylcarbonyl-, Arylcarbonyl-, Aryl-C₁-C₆-alkylcarbonyl-, Hetarylcarbonyl- oder Hetaryl-C₁-C₆-alkylcarbonyl- ist;
R⁶ C₁-C₆-Alkyloxy-, Aryloxy-, Aryl-C₁-C₆-alkyloxy-, Hetaryloxy- oder Hetaryl-C₁-C₆-alkyloxy- ist;
R⁷ Wasserstoff, C₁-C₈-Alkyl, C₁-C₆-Alkyloxy oder Aryl-C₁-C₆-alkyl ist;
X und Y unabhängig -CH- oder Stickstoff sind;
n 1 oder 2 ist; oder
ein Salz davon mit einer pharmazeutisch unbedenklichen Säure oder Base oder ein beliebiges optisches Isomer oder eine Mischung von optischen Isomeren, einschließlich einer racemischen Mischung, oder beliebige tautomere Formen;
mit der Maßgabe, dass die Verbindung nicht 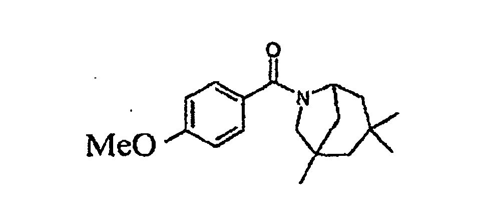 ist.

13. Verbindung nach Anspruch 12, in der X und Y -CH- sind.

14. Verbindung nach Anspruch 12 oder 13, in der R³ -NR⁸R⁹ ist, wobei R⁸ und R⁹ wie in Anspruch 1 definiert sind.

15. Verbindung nach einem der Ansprüche 12 bis 14, in der R⁴ Wasserstoff oder Halogen ist.

16. Verbindung nach einem der Ansprüche 12 bis 15, die aus der Gruppe ausgewählt ist, die aus folgenden Verbindungen besteht: [4-(1,3,3-Trimethyl-6-azabicyclo[3.2.1]octan-6-carbonyl)phenyl]carbaminsäure-*tert*.-butylester;
(4-Aminophenyl)-(1,3,3-trimethyl-6-azabicyclo[3.2.1]oct-6-yl)methanon;
(4-Methylaminophenyl)-(1,3,3-trimethyl-6-azabicyclo[3.2.1]oct-6-yl)methanon;
N-Methyl-N-[4-(1,3,3-trimethyl-6-azabicyclo[3.2.1]octan-6-carbonyl)phenyl]methansulfonamid;
N-Methyl-N-[4-(1,3,3-trimethyl-6-azabicyclo[3.2.1]octan-6-carbonyl)phenyl]benzolsulfonamid;
N-Methyl-C-phenyl-N-[4-(1,3,3-trimethyl-6-azabicyclo[3.2.1]octan-6-carbonyl)phenyl]methansulfonamid;
N-[4-(1,3,3-Trimethyl-6-azabicyclo[3.2.1]octan-6-carbonyl)phenyl]methansulfonamid;
N-Ethyl-N-[4-(1,3,3-trimethyl-6-azabicyclo[3.2.1]octan-6-carbonyl)phenyl]methansulfonamid;
N-Cyclopropylmethyl-N-[4-(1,3,3-trimethyl-6-azabicyclo[3.2.1]octan-6-carbonyl)phenyl]methansulfonamid;
N-[4-(1,3,3-Trimethyl-6-azabicyclo[3.2.1]octan-6-carbonyl)phenyl]isonicotinamid;
2,4-Dichlor-N-[4-(1,3,3-trimethyl-6-azabicyclo[3.2.1]octan-6-carbonyl)phenyl]benzamid;
2,4-Dimethoxy-N-[4-(1,3,3-trimethyl-6-azabicyclo[3.2.1]octan-6-carbonyl)phenyl]benzamid;
4-Trifluormethoxy-N-[4-(1,3,3-trimethyl-6-azabicyclo[3.2.1]octan-6-carbonyl)phenyl]benzamid;
3,4-Dimethoxy-N-[4-(1,3,3-trimethyl-6-azabicyclo[3.2.1]octan-6-carbonyl)phenyl]benzamid;
3,5-Dimethyl-N-[4-(1,3,3-trimethyl-6-azabicyclo[3.2.1]octan-6-carbonyl)phenyl]benzamid;
3-Trifluormethoxy-N-[4-(1,3,3-trimethyl-6-azabicyclo[3.2.1]octan-6-carbonyl)phenyl]benzamid;
3,5-Dimethoxy-N-[4-(1,3,3-trimethyl-6-azabicyclo[3.2.1]octan-6-carbonyl)phenyl]benzamid;
3-Cyano-N-[4-(1,3,3-trimethyl-6-azabicyclo[3.2.1]octan-6-carbonyl)phenyl]benzamid;
N-[4-(1,3,3-Trimethyl-6-azabicyclo[3.2.1]octan-6-carbonyl)phenyl]benzolsulfonamid;
Phenyl-N-[4-(1,3,3-trimethyl-6-azabicyclo[3.2.1]octan-6-carbonyl)phenyl]methansulfonamid;
[4-(1,3,3-Trimethyl-6-azabicyclo[3.2.1]octan-6-carbonyl)phenyl]butan-1-sulfonsäureamid;
3-Trifluormethyl-N-[4-(1,3,3-trimethyl-6-azabicyclo[3.2.1]octan-6-carbonyl)phenyl]benzolsulfonamid;
N-[4-(1,3,3-Trimethyl-6-azabicyclo[3.2.1]octan-6-carbonyl)phenyl]isophthalamidsäuremethylester;
N-[4-(1,3,3-Trimethyl-6-azabicyclo[3.2.1]octan-6-carbonyl)phenyl]isophthalamidsäure;
[4-(1,3,3-Trimethyl-6-azabicyclo[3.2.1]octan-6-carbonyl)phenyl]-4-methoxycyclohexancarbonsäureamid;
6-[4-(1,3,3-Trimethyl-6-azabicyclo[3.2.1]octan-6-carbonyl)phenylcarbamoyl]nicotinsäuremethylester;
6-[4-(1,3,3-Trimethyl-6-azabicyclo[3.2.1]octan-6-carbonyl)phenylcarbamoyl]nicotinsäure;
N-[4-(1,3,3-Trimethyl-6-azabicyclo[3.2.1]octan-6-carbonyl)phenyl]malonamidsäureethylester;
N-[4-(1,3,3-Trimethyl-6-azabicyclo[3.2.1]octan-6-carbonyl)phenyl]malonamidsäure-*tert*.-butylester;
3-[4-(1,3,3-Trimethyl-6-azabicyclo[3.2.1]octan-6-carbonyl)phenylcarbamoyl]cyclohexancarbonsäuremethylester;
3-[4-(1,3,3-Trimethyl-6-azabicyclo[3.2.1]octan-6-carbonyl)phenylcarbamoyl]cyclohexancarbonsäure;
N-[4-(1,3,3-Trimethyl-6-azabicyclo[3.2.1]octan-6-carbonyl)phenyl]benzamid;
N-[4-(1,3,3-Trimethyl-6-azabicyclo[3.2.1]octan-6-carbonyl)phenyl]isonicotinamid;
N-[4-(1,3,3-Trimethyl-6-azabicyclo[3.2.1]octan-6-carbonyl)phenyl]nicotinamid;
[4-(1,3,3-Trimethyl-6-azabicyclo[3.2.1]octan-6-carbonyl)phenyl]pyridin-2-carbonsäureamid;
[4-(1,3,3-Trimethyl-6-azabicyclo[3.2.1]octan-6-carbonyl)phenyl]-1-acetylpiperidin-4-carbonsäureamid;
N-[4-(1,3,3-Trimethyl-6-azabicyclo[3.2.1]octan-6-carbonyl)phenyl]succinamidsäuremethylester;
N-[4-(1,3,3-Trimethyl-6-azabicyclo[3.2.1]octan-6-carbonyl)phenyl]succinamidsäure;
N-[4-(1,3,3-Trimethyl-6-azabicyclo[3.2.1]octan-6-carbonyl)phenyl]malonamidsäure;
2-Amino-N-[4-(1,3,3-trimethyl-6-azabicyclo[3.2.1]octan-6-carbonyl)phenyl]acetamid;
2-Acetylamino-N-[4-(1,3,3-trimethyl-6-azabicyclo[3.2.1]octan-6-carbonyl)phenyl]succinamidsäuremethylester;
2-Acetylamino-N-[4-(1,3,3-trimethyl-6-azabicyclo[3.2.1]octan-6-carbonyl)phenyl]succinamidsäure;
N-Methyl-N'-[4-(1,3,3-trimethyl-6-azabicyclo[3.2.1]octan-6-carbonyl)phenyl]succinamid;
N,N-Dimethyl-N'-[4-(1,3,3-trimethyl-6-azabicyclo[3.2.1]octan-6-carbonyl)phenyl]succinamid;
N-[5-(1,3,3-Trimethyl-6-azabicyclo[3.2.1]octan-6-carbonyl)pyridin-2-yl]benzamid;
N-Methyl-N-[4-(1,3,3-trimethyl-6-azabicyclo[3.2.1]octan-6-carbonyl)phenyl]benzamid;
3,5,N-Trimethyl-N-[4-(1,3,3-trimethyl-6-azabicyclo[3.2.1]octan-6-carbonyl)phenyl]benzamid;
2,4-Dimethoxy-N-methyl-N-[4-(1,3,3-trimethyl-6-azabicyclo[3.2.1]octan-6-carbonyl)phenyl]benzamid;
3,5-Dichlor-N-methyl-N-[4-(1,3,3-trimethyl-6-azabicyclo[3.2.1]octan-6-carbonyl)phenyl]benzamid;
4-Brom-N,N-dimethyl-N-[4-(1,3,3-trimethyl-6-azabicyclo[3.2.1]octan-6-carbonyl)phenyl]benzamid;
N-Methyl-N-[4-(1,3,3-trimethyl-6-azabicyclo[3.2.1]octan-6-carbonyl)phenyl]isonicotinamid;
N-Methyl-N-[4-(1,3,3-trimethyl-6-azabicyclo[3.2.1]octan-6-carbonyl)phenyl]nicotinamid;
2-Piperidin-1-yl-N-[4-(1,3,3-trimethyl-6-azabicyclo[3.2.1]octan-6-carbonyl)phenyl]acetamid;
2-Morpholin-4-yl-N-[4-(1,3,3-trimethyl-6-azabicyclo[3.2.1]octan-6-carbonyl)phenyl]acetamid;
2-(4-Methylpiperazin-1-yl)-N-[4-(1,3,3-trimethyl-6-azabicyclo[3.2.1]octan-6-carbonyl)phenyl]acetamid;
2-Dimethylamino-N-[4-(1,3,3-trimethyl-6-azabicyclo[3.2.1]octan-6-carbonyl)phenyl]acetamid;
2-[(2-Dimethylaminoethyl)methylamino]-N-[4-(1,3,3-trimethyl-6-azabicyclo[3.2.1]octan-6-carbonyl)phenyl]acetamid;
2-(3,4-Dihydro-2H-chinolin-1-yl)-N-[4-(1,3,3-trimethyl-6-azabicyclo[3.2.1]octan-6-carbonyl)phenyl]acetamid;
2-(4-Acetylpiperidin-1-yl)-N-[4-(1,3,3-trimethyl-6-azabicyclo[3.2.1]octan-6-carbonyl)phenyl]acetamid;
2-(4-Dimethylaminopiperidin-1-yl)-N-[4-(1,3,3-trimethyl-6-azabicyclo[3.2.1]octan-6-carbonyl)phenyl]acetamid;
2-(4-Oxopiperidin-1-yl)-N-[4-(1,3,3-trimethyl-6-azabicyclo[3.2.1]octan-6-carbonyl)phenyl]acetamid;
2-[Methyl-(1-methylpiperidin-4-yl)amino]-N-[4-(1,3,3-trimethyl-6-azabicyclo[3.2.1]octan-6-carbonyl)phenyl]acetamid;
2-(3,6-Dihydro-2H-pyridin-1-yl)-N-[4-(1,3,3-trimethyl-6-azabicyclo[3.2.1]octan-6-carbonyl)phenyl]acetamid;
1-{[4-(1,3,3-Trimethyl-6-azabicyclo[3.2.1]octan-6-carbonyl)phenylcarbamoyl]methyl}piperidin-4-carbonsäureethylester;
N-[4-(1,3,3-Trimethyl-6-azabicyclo[3.2,1]octan-6-carbonyl)phenyl]-2-(1,3,3-trimethyl-6-azabicyclo[3.2.1]oct-6-yl)acetamid;
2-(4-Acetylpiperazin-1-yl)-N-[4-(1,3,3-trimethyl-6-azabicyclo[3.2.1]octan-6-carbonyl)phenyl]acetamid;
2-(2,3,5,6-Tetrahydro-[1,2']-bipyrazinyl-4-yl)-N-[4-(1,3,3-trimethyl-6-azabicyclo[3.2.1]octan-6-carbonyl)phenyl]acetamid;
2-(Cyclohexylmethylamino)-N-[4-(1,3,3-trimethyl-6-azabicyclo[3.2.1]octan-6-carbonyl)phenyl]acetamid;
2-(2,4-Dimethoxybenzyloxy)-N-[4-(1,3,3-trimethyl-6-azabicyclo[3.2.1]octan-6-carbonyl)phenyl]acetamid;
2-Benzyloxy-N-[4-(1,3,3-trimethyl-6-azabicyclo[3.2.1]octan-6-carbonyl)phenyl]acetamid;
2-(3,4-Dihydro-1H-isochinolin-2-yl)-N-[4-(1,3,3-trimethyl-6-azabicyclo[3.2.1]octan-6-carbonyl)phenyl]acetamid;
2-(6-Azabicyclo[3.2.1]oct-6-yl)-N-[4-(1,3,3-trimethyl-6-azabicyclo[3.2.1]octan-6-carbonyl)phenyl]acetamid;
2-Azepan-1-yl-N-[4-(1,3,3-trimethyl-6-azabicyclo[3.2.1]octan-6-carbonyl)phenyl]acetamid;
2-(6,7-Dimethoxy-3,4-dihydro-1H-isochinolin-2-yl)-N-[4-(1,3,3-trimethyl-6-azabicyclo[3.2.1]octan-6-carbonyl)phenyl]acetamid;
2-Indol-1-yl-N-[4-(1,3,3-trimethyl-6-azabicyclo[3.2.1]octan-6-carbonyl)phenyl]acetamid;
2-Benzoimidazol-1-yl-N-[4-(1,3,3-trimethyl-6-azabicyclo[3.2.1]octan-6-carbonyl)phenyl]acetamid;
2-(2,3-Dihydroindol-1-yl)-N-[4-(1,3,3-trimethyl-6-azabicyclo[3.2.1]octan-6-carbonyl)phenyl]acetamid;
2-Benzotriazol-1-yl-N-[4-(1,3,3-trimethyl-6-azabicyclo[3.2.1]octan-6-carbonyl)phenyl]acetamid;
2-(4-Benzoylpiperidin-1-yl)-N-[4-(1,3,3-trimethyl-6-azabicyclo[3.2.1]octan-6-carbonyl)phenyl]acetamid;
2-(4-*tert*.-Butylpiperidin-1-yl)-N-[4-(1,3,3-trimethyl-6-azabicyclo[3.2.1]octan-6-carbonyl)phenyl]acetamid;
2-(4-Phenylpiperidin-1-yl)-N-[4-(1,3,3-trimethyl-6-azabicyclo[3.2.1]octan-6-carbonyl)phenyl]acetamid;
2-(4-Hydroxy-4-phenylpiperidin-1-yl)-N-[4-(1,3,3-trimethyl-6-azabicyclo[3.2.1]octan-6-carbonyl)phenyl]acetamid;
2-(4-Pyridin-2-ylpiperazin-1-yl)-N-[4-(1,3,3-trimethyl-6-azabicyclo[3.2.1]octan-6-carbonyl)phenyl]acetamid;
2-(4-Phenylpiperazin-1-yl)-N-[4-(1,3,3-trimethyl-6-azabicyclo[3.2.1]octan-6-carbonyl)phenyl]acetamid;
2-(4-Pyrimidin-2-ylpiperazin-1-yl)-N-[4-(1,3,3-trimethyl-6-azabicyclo[3.2.1]octan-6-carbonyl)phenyl]acetamid;
2-(4-Acetyl-4-phenylpiperidin-1-yl)-N-[4-(1,3,3-trimethyl-6-azabicyclo[3.2.1]octan-6-carbonyl)phenyl]acetamid;
2-(4-Cyano-4-phenylpiperidin-1-yl)-N-[4-(1,3,3-trimethyl-6-azabicyclo[3.2.1]octan-6-carbonyl)phenyl]acetamid;
2-(4-Pyridin-4-ylpiperazin-1-yl)-N-[4-(1,3,3-trimethyl-6-azabicyclo[3.2.1]octan-6-carbonyl)phenyl]acetamid;
2-[4-(2-Pyrrolidin-1-ylacetyl)piperazin-1-yl]-N-[4-(1,3,3-trimethyl-6-azabicyclo[3.2.1]octan-6-carbonyl)phenyl]acetamid;
2-(Piperidin-4-yloxy)-N-[4-(1,3,3-trimethyl-6-azabicyclo[3.2.1]octan-6-carbonyl)phenyl]acetamid;
2-(2H-Tetrazol-5-yl)-N-[4-(1,3,3-trimethyl-6-azabicyclo[3.2.1]octan-6-carbonyl)phenyl]acetamid;
3-Piperidin-1-yl-N-[4-(1,3,3-trimethyl-6-azabicyclo[3.2.1]octan-6-carbonyl)phenyl]propionamid;
3-Benzylamino-N-[4-(1,3,3-trimethyl-6-azabicyclo[3.2.1]octan-6-carbonyl)phenyl]propionamid;
2-(3,4-Dihydro-2H-chinolin-1-yl)-N-methyl-N-[4-(1,3,3-trimethyl-6-azabicyclo[3.2.1]octan-6-carbonyl)phenyl]acetamid;
1-Morpholin-4-yl-2-[4-(1,3,3-trimethyl-6-azabicyclo[3.2.1]octan-6-carbonyl)phenylamino]ethanon;
[4-(2-Piperidin-1-ylethylamino)phenyl]-(1,3,3-trimethyl-6-azabicyclo[3.2.1]oct-6-yl)methanon;
N-Methyl-N-phenyl-2-[4-(1,3,3-trimethyl-6-azabicyclo[3.2.1]octan-6-carbonyl)phenylamino]acetamid;
N-(2-Methoxyethyl)-N-[4-(1,3,3-trimethyl-6-azabicyclo[3.2.1]octan-6-carbonyl)phenyl]benzamid;
N-Allyl-N-[4-(1,3,3-trimethyl-6-azabicyclo[3.2.1]octan-6-carbonyl)phenyl]benzamid;
[4-(2-Methoxyethylamino)phenyl]-(1,3,3-trimethyl-6-azabicyclo[3.2.1]oct-6-yl)methanon;
(4-Benzyloxyphenyl)-(1,3,3-trimethyl-6-azabicyclo[3.2.1]oct-6-yl)methanon;
3-[4-(1,3,3-Trimethyl-6-azabicyclo[3.2.1]octan-6-carbonyl)benzoylamino]propionsäuremethylester;
3-[4-(1,3,3-Trimethyl-6-azabicyclo[3.2.1]octan-6-carbonyl)benzoylamino]propionsäure;
(4-Benzyloxyphenyl)-(1,3,3-trimethyl-6-azabicyclo[3.2.1]oct-6-yl)methanon;
oder ein Salz davon mit einer pharmazeutisch unbedenklichen Säure oder Base oder ein beliebiges optisches Isomer oder eine Mischung von optischen Isomeren, einschließlich einer racemischen Mischung, oder beliebige tautomere Formen.

17. Verfahren zur Herstellung einer wie in einem der Ansprüche 12 bis 16 definierten Verbindung, wobei das Verfahren aus (A) bis (F) im Folgenden ausgewählt ist: 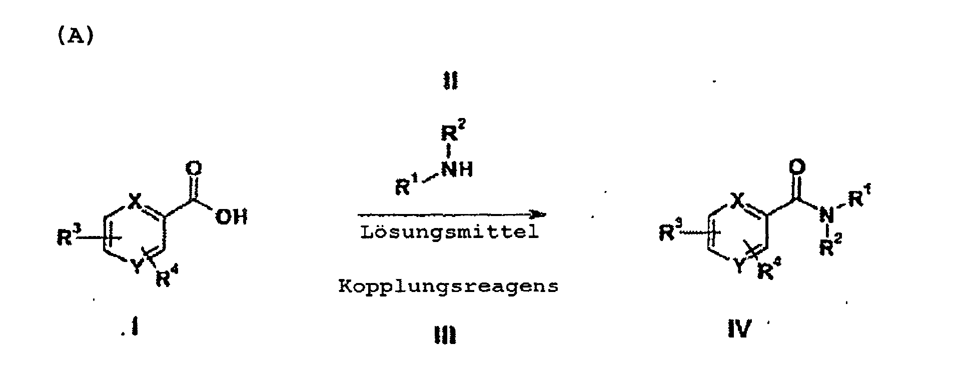 eine Säure (I), wobei R³, R⁴, X und Y in Anspruch 1 definiert sind, wird mit einem Amin (II), wobei R¹ und R² in Anspruch 12 definiert sind, unter
Standardamidbildungsbedingungen unter Verwendung eines Kopplungsreagens (III) gekoppelt, wodurch ein Amid (IV) erhalten wird, wobei R¹, R², R³, R⁴, X und Y wie oben definiert sind; oder 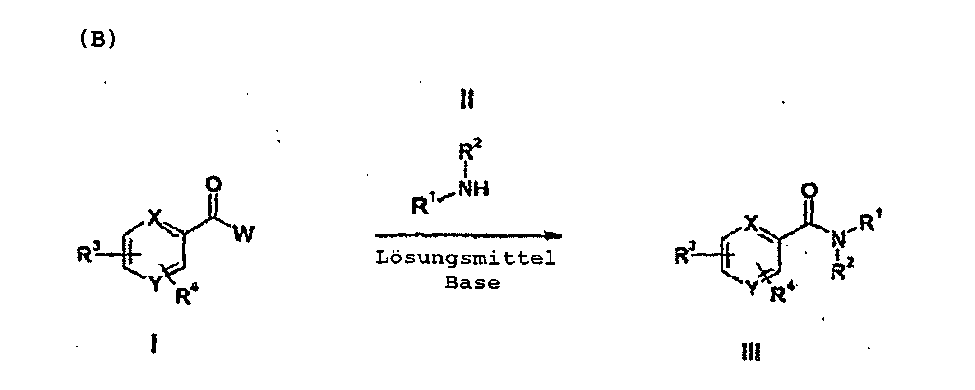 ein Säurederivat (I), wobei W Halogen, R²⁰(C=O)O-, C₁-C₆-Alkyloxy oder Aryl-C₁-C₆-alkyloxy ist, R²⁰ C₁-C₆-Alkyl oder Aryl-C₁-C₆-alkyl ist und R³, R⁴, X und Y in Anspruch 12 definiert sind, wird mit einem Amin (II), wobei R¹ und R² in Anspruch 12 definiert sind, unter basischen Bedingungen in einem Lösungsmittel umgesetzt wird, wodurch ein Amid (III) erhalten wird; wobei R¹, R², R³, R⁴, X und Y wie oben definiert sind; oder 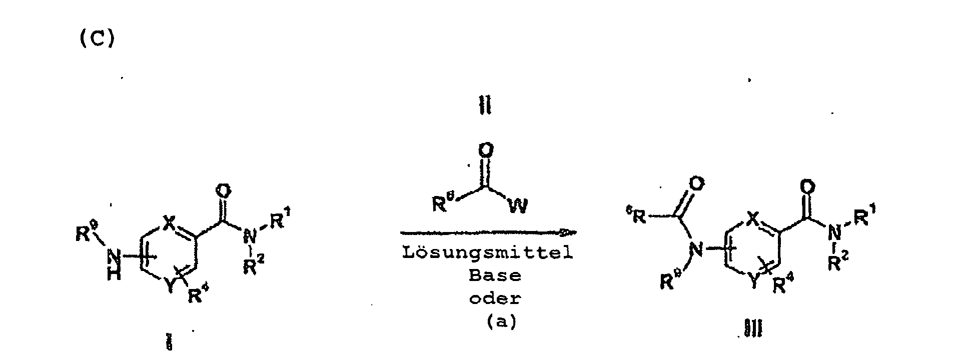
(i) ein Amidderivat (I), wobei R¹, R², R⁴, R⁹, X und Y wie in Anspruch 12 definiert sind, wird mit einem Säurederivat (II), wobei W Halogen, R⁷(C=O)O-, C₁-C₆-Alkyloxy oder Aryl-C₁-C₆-alkyloxy ist, R⁷ C₁-C₆-Alkyl oder Aryl-C₁-C₆-alkyl ist und R⁶ C₃-C₁₀-Cycloalkyl, C₃-C₁₀-Hetcycloalkyl, Aryl, Hetaryl, R¹⁴C₁-C₆-Alkylcarbonyl-, C₁-C₆-Alkyl-S(O)ₙ-, Aryl-S(O)ₙ- oder Aryl-C₁-C₆-alkyl-S(O)ₙ- ist, wobei die Alkyl-, Cycloalkyl-, Hetcycloalkyl-, Arylgruppen gegebenenfalls mit einem oder mehreren wie in Anspruch 12 definierten R¹¹ substituiert sind, unter basischen Bedingungen in einem Lösungsmittel umgesetzt wird, wodurch ein Amid (III) erhalten wird; wobei R¹, R², R⁴, R⁹, X und Y wie in Anspruch 12 definiert sind und R⁶ C₃-C₁₀-Cycloalkyl, C₃-C₁₀-Hetcycloalkyl, Aryl, Hetaryl, R¹⁴C₁-C₆-Alkylcarbonyl-, C₁-C₆-Alkyl-S(O)ₙ-, Aryl-S(O)ₙ- oder Aryl-C₁-C₆-alkyl-S(O)ₙ- ist, wobei die Alkyl-, Cycloalkyl-, Hetcycloalkyl-, Arylgruppen gegebenenfalls mit einem oder mehreren wie oben definierten R¹¹ substituiert sind; oder
(ii) wenn W Hydroxy ist, das Säurederivat (II), wobei R⁶ wie oben definiert ist, wird mit einem Amin (I), wobei R¹, R², R⁴, R⁹, X und Y wie oben definiert sind, unter Standardamidbildungsbedingungen unter Verwendung eines Kopplungsreagens (a) gekoppelt, wodurch ein Amid (III) erhalten wird; wobei R¹, R², R⁴, R⁹, X und Y wie oben definiert sind und R⁶ C₃-C₁₀-Cycloalkyl, C₃-C₁₀-Hetcycloalkyl, Aryl, Hetaryl, R¹⁴C₁-C₆-Alkylcarbonyl-, C₁-C₆-Alkyl-S(O)ₙ-, Aryl-S(O)ₙ- oder Aryl-C₁-C₆-alkyl-S(O)ₙ- ist, wobei die Alkyl-, Cycloalkyl-, Hetcycloalkyl-, Arylgruppen gegebenenfalls mit einem oder mehreren wie oben definierten R¹¹ substituiert sind; oder 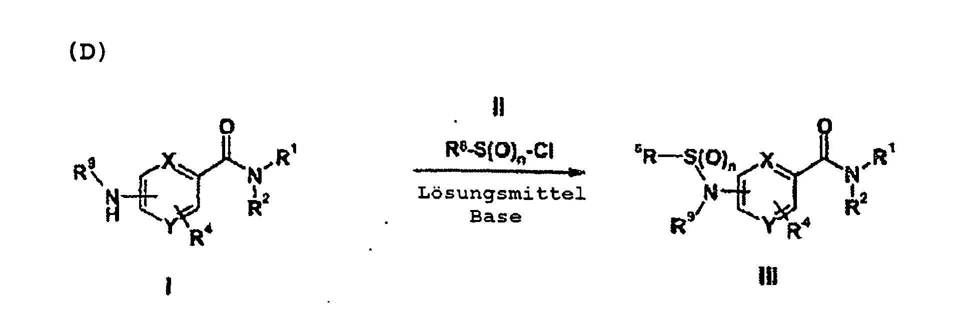 ein Amidderivat (I), wobei R¹, R², R⁴, R⁹, X und Y wie in Anspruch 12 definiert sind, wird mit einem Sulfinylchloridderivat (n = 1) oder Sulfonylchloridderivat (n = 2) (II), wobei R⁶ C₁-C₆-Alkyl, C₃-C₁₀-Cycloalkyl, C₃-C₁₀-Hetcycloalkyl, Aryl, Hetaryl oder Aryl-C₁-C₆-alkyl ist, wobei die Alkyl-, Cycloalkyl-, Hetcycloalkyl-, Aryl- und Hetarylgruppen gegebenenfalls mit einem oder mehreren wie in Anspruch 12 definierten R¹¹ substituiert sind, unter basischen Bedingungen in einem Lösungsmittel umgesetzt, wodurch ein Amid (III) erhalten wird; wobei R¹, R², R⁴, R⁹, n, X und Y wie oben definiert sind und R⁶ C₁-C₆-Alkyl, C₃-C₁₀-Cycloalkyl, C₃-C₁₀-Hetcycloalkyl, Aryl, Hetaryl oder Aryl-C₁-C₆-alkyl ist, wobei die Alkyl-, Cycloalkyl-, Hetcycloalkyl-, Aryl- und Hetarylgruppen gegebenenfalls mit einem oder mehreren wie oben definierten R¹¹ substituiert sind; oder 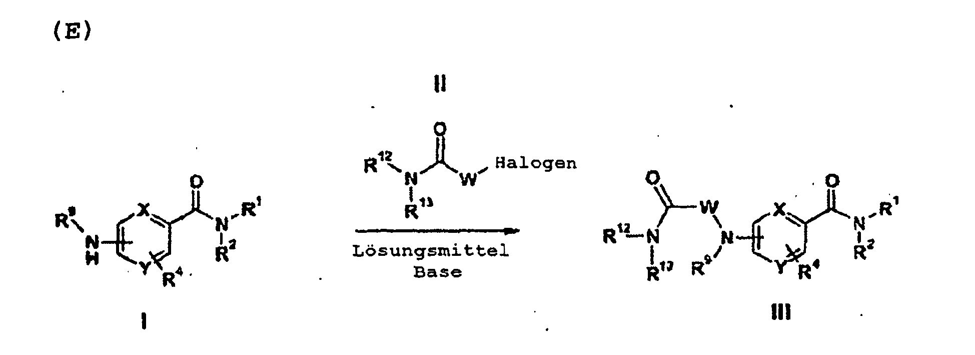 ein Amidderivat (I), wobei R¹, R², R⁴, R⁹, X und Y wie in Anspruch 12 definiert sind, wird mit einem Amidoalkylhalogenidderivat (II), wobei R¹², R¹³ und Halogen wie in Anspruch 12 definiert sind und W C₁-C₆-Alkyl ist, wobei die Alkylgruppe gegebenenfalls mit einem oder mehreren wie in Anspruch 12 definierten R¹¹ substituiert ist, unter basischen Bedingungen in einem Lösungsmittel umgesetzt, wodurch ein Amid (III) erhalten wird; wobei R¹, R², R⁴, R⁹, R¹², R¹³, X und Y wie oben definiert sind und W C₁-C₆-Alkyl ist, wobei die Alkylgruppe gegebenenfalls mit einem oder mehreren wie oben definierten R¹¹ substituiert ist; oder 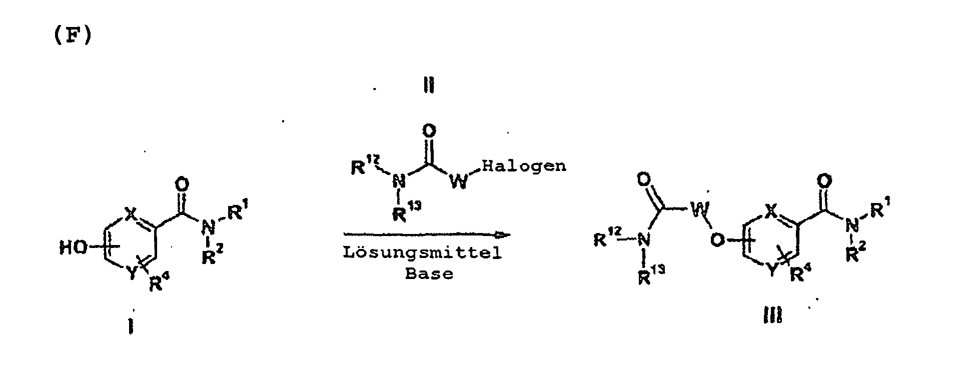 ein Amidderivat (I), wobei R¹, R², R⁴, X und Y wie in Anspruch 12 definiert sind, wird mit einem Amidoalkylhalogenidderivat (II), wobei R¹² und R¹³ wie in Anspruch 12 definiert sind und W C₁-C₆-Alkyl ist, wobei die Alkylgruppe gegebenenfalls mit einem oder mehreren wie in Anspruch 12 definierten R¹¹ substituiert ist, unter basischen Bedingungen in einem Lösungsmittel umgesetzt, wodurch ein Amid (III) erhalten wird; wobei R¹, R², R⁴, R¹², R¹³, X und Y wie oben definiert sind und W C₁-C₆-Alkyl ist, wobei die Alkylgruppe gegebenenfalls mit einem oder mehreren wie oben definierten R¹¹ substituiert ist;
und gegebenenfalls Umwandeln des Produkts aus einem beliebigen der Verfahren (A) bis (F) in ein pharmazeutisch unbedenkliches Salz.

## Revendications

1. Composé de la formule générale (I) : 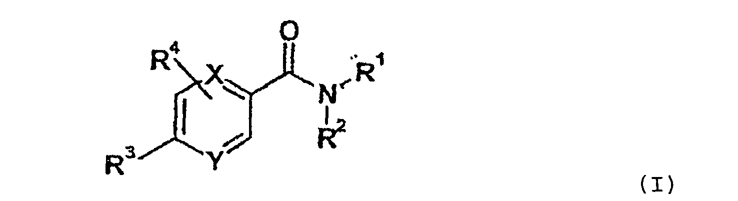 dans laquelle
R¹ et R² avec l'azote auquel ils sont liés sont 6-aza-bicyclo[3.2.1]octane substitué en option par au moins un de C₁-C₆-alkyle ;
R³ est C₁-C₆-alkyle, -NR⁸R⁹, -C(=O)NR⁸R⁹ ou -OR¹⁰, dans lequel le groupe alkyle est substitué en option par un ou plusieurs de R¹¹ ;
R⁴ est hydrogène, halogéno, hydroxy, cyano, trihalogénométhyle ou C₁-C₆-alkyle ;
R⁸ est hydrogène, C₁-C₆-alkyle, C₃-C₁₀-cycloalkyle, C₃-C₁₀-cycloalkyl-C₁-C₆-alkyle, C₁-C₆-alkyloxy-C₁-C₆-alkyle, aryl-C₁-C₆-alkyloxy-C₁-C₆-alkyle ou C₂-C₈-alkényle ;
R⁹ est C₁-C₆-alkyle, C₃-C₁₀-cycloalkyle, C₃-C₁₀-cycloalkyl-C₁-C₆-alkyle, C₃-C₁₀-cycloalkylcarbonyl-, C₃-C₁₀-hetcycloalkylcarbonyl-, arylcarbonyl-, hétarylcarbonyl-, C₁-C₆-alkyloxy-C₁-C₆-alkyle, NR¹²R¹³⁻ carbonyl-C₁-C₆-alkyl-, R¹⁴-C₁-C₆-alkylcarbonyl-, -COR¹⁵, C₁-C₆-alkyl-S(O)ₙ-, aryl-S(O)ₙ-, aryl-C₁-C₆-alkyl-S(O)ₙ-, aryl-C₁-C₆-alkyle ou hétaryl-C₁-C₆-alkyle, dans lequel les groupes alkyle, cycloalkyle, aryle et hétaryle sont indépendamment substitués en option par un ou plusieurs R¹¹ ;
R¹⁰ est C₁-C₆-alkyle, aryle-C₁-C₆-alkyle ou NR¹²R¹³-carbonyl-C₁-C₆-alkyle, dans lequel les groupes alkyle et aryle sont indépendamment substitués en option par un ou plusieurs R¹¹ ;
R¹¹ est R⁵, R⁶, halogéno, hydroxy, oxo, cyano, -COR¹⁵, C₁-C₈-alkyle, C₁-C₈-alkyloxy, C₃-C₁₀-cycloalkyle, trihalogénométhyle, trihalogénométhyloxy, aryle, aryl-C₁-C₆-alkyle, C₁-C₆-alkyloxy-C₁-C₆-alkyle, aryloxy-C₁-C₆-alkyle, aryl-C₁-C₆-alkyloxy-C₁-C₆-alkyle, hétaryle, hétaryl-C₁-C₆-alkyle, hétaryloxy-C₁-C₆-alkyle, hétaryl-C₁-C₆-alkyloxy-C₁-C₆-alkyle, -NR¹²R¹³, -SO₂NR¹²R¹³, NR¹²R¹³-carbonyl-C₁-C₆-alkyle, R¹⁶-carbonyl-N(R¹²)-, aryl-S(O)ₙ-, hétaryl-S(O)ₙ- ou R¹⁷S(O)ₙN(R¹²)- ; dans lequel les groupes aryle et hétaryle sont indépendamment substitués en option par un ou plusieurs R¹⁸ ;
R¹² et R¹³ sont indépendamment hydrogène, C₁-C₈-alkyle, C₃-C₁₀-cycloalkyle, C₃-C₁₀-cycloalkyl-C₁-C₆-alkyle, C₃-C₁₀-hetcycloalkyle, aryle, hétaryle, aryl-C₁-C₆-alkyle ou hétaryl-C₁-C₆-alkyle, dans lequel les groupes alkyle, aryle et hétaryle sont indépendamment substitués en option par un ou plusieurs de R¹⁸ ; ou R¹² et R¹³ avec l'azote auquel ils sont liés forment un système annulaire cyclique, bicyclique ou tricyclique, saturé ou partiellement saturé, contenant 4 à 12 atomes de carbone et 0 à 2 hétéroatomes supplémentaires sélectionnés parmi l'azote ou l'oxygène, le système annulaire étant substitué en option par au moins un R⁵, R⁵-oxy-, R⁶, halogéno, cyano, hydroxy, oxo, C₁-C₈-alkyle, aryle, hétaryle, aryl-C₁-C₆-alkyle, hétaryl-C₁-C₆-alkyle, C₁-C₆-alkyloxy-C₁-C₆-alkyle, NR¹²R¹³-carbonyl-C₁-C₆-alkyle, NR¹²R¹³-C₁-C₆-alkylcarbonyl-, R¹⁴-C₁-C₆-alkylcarbonyl- ou COR¹⁵ ;
R¹⁴ est C₁-C₆-alkyloxy, C₃-C₁₀-cycloalkyloxy-, C₃-C₁₀-cycloalkyl-C₁-C₆-alkyloxy-, C₃-C₁₀-hetcycloalkyloxy-, aryle, hétaryle, aryl-C₁-C₆-alkyloxy, hétaryl-C₁-C₆-alkyloxy, -NR¹²R¹³, -COR¹⁵, dans lequel les groupes alkyle, cycloalkyle, hetcycloalkyle, aryle et hétaryle sont indépendamment substitués en option par un ou plusieurs de R²⁰ ;
R¹⁵ est C₁-C₆-alkyle, hydroxy, C₁-C₈-alkyloxy, -NR¹²R¹³, aryle, aryloxy ou aryl-C₁-C₆-alkyloxy ;
R¹⁶ est R⁶, C₁-C₆-alkyle, C₂-C₆-alkényle, aryle, aryl-C₁-C₆-alkyle, hétaryle, hétaryl-C₁-C₆-alkyle, C₃-C₁₀-cycloalkyle, C₃-C₁₀-hetcycloalkyle, aryl-C₁-C₆-alkyloxy-C₁-C₆-alkyl-, hétaryl-C₁-C₆-alkyloxy-C₁-C₆-alkyl- ou R¹²R¹³-NC₁-C₆-alkyl-, dans lequel les groupes alkyle, alkényle, cycloalkyle, hetcycloalkyle, aryle et hétaryle sont substitués en option par R¹⁹ ;
R¹⁷ est C₁-C₆-alkyle, C₃-C₁₀-cycloalkyle, C₃-C₁₀-hetcycloalkyle, aryle, aryl-C₁-C₆-alkyle, hétaryle, hétaryl-C₁-C₆-alkyle ;
R¹⁸ est R⁶, -NR¹²R¹³, oxo, C₁-C₆-alkyle, C₃-C₁₀-cycloalkyle ou C₃-C₁₀-hetcycloalkyle, dans lequel les groupes alkyle, cycloalkyle, hetcycloalkyle, aryle et hétaryle sont indépendamment substitués en option par un ou plusieurs de R⁷ ;
R¹⁹ est hydrogène, halogéno, hydroxy, oxo, nitro, cyano ou -COR¹⁵ ;
R²⁰ est hydrogène, C₁-C₈-alkyle, -NR¹²R¹³, C₁-C₆-alkyloxy ou aryl-C₁-C₆-alkyle ;
R⁵ est C₁-C₆-alkylcarbonyl-, C₃-C₁₀-cycloalkylcarbonyl-, C₃-C₁₀-cycloalkyl-C₁-C₆-alkylcarbonyl-, arylcarbonyl-, aryl-C₁-C₆-alkylcarbonyl-, hétarylcarbonyl- ou hétaryl-C₁-C₆-alkylcarbonyl- ;
R⁶ est C₁-C₆-alkyloxy-, aryloxy-, aryl-C₁-C₆-alkyloxy-, hétaryloxy- ou hétaryl-C₁-C₆-alkyloxy- ;
R⁷ est hydrogène, C₁-C₈-alkyle, C₁-C₆-alkyloxy ou aryl-C₁-C₆-alkyle ;
X et Y sont indépendamment -CH- ou azote ;
n est 1 ou 2 ; ou
un sel de celui-ci avec un acide ou une base pharmaceutiquement acceptable, ou tout isomère optique
ou mélange d'isomères optiques, y compris un mélange racémique, ou toute forme tautomère, destiné à être utilisé en tant que médicament.

2. Composé destiné à être utilisé en tant que médicament selon la revendication 1, dans lequel X et Y sont -CH-.

3. Composé destiné à être utilisé en tant que médicament selon la revendication 1 ou 2, dans lequel R³ est -NR⁸R⁹, dans lequel R⁸ et R⁹ sont tels que définis à la revendication 1.

4. Composé destiné à être utilisé en tant que médicament selon l'une quelconque des revendications 1 à 3, dans lequel R⁴ est hydrogène ou halogéno.

5. Composé destiné à être utilisé en tant que médicament selon l'une quelconque des revendications 1 à 4 sélectionné dans le groupe constitué de : ester tert-butylique d'acide [4-(1,3,3-triméthyl-6-aza-bicyclo[3.2.1]octane-6-carbonyl)-phényl]carbamique ;
(4-amino-phényl)-(1,3,3-triméthyl-6-aza-bicyclo[3.2.1]oct-6-yl)-méthanone ;
(4-méthylamino-phényl)-(1,3,3-triméthyl-6-aza-bicyclo[3.2.1]oct-6-yl)-méthanone ;
N-méthyl-N-[4-(1,3,3-triméthyl-6-aza-bicyclo[3.2.1]octane-6-carbonyl)-phényl]-méthanesulfonamide ;
N-méthyl-N-[4-(1,3,3-triméthyl-6-aza-bicyclo[3.2.1]octane-6-carbonyl)-phényl]-benzènesulfonamide ;
N-méthyl-C-phényl-N-[4-(1,3,3-triméthyl-6-aza-bicyclo[3.2.1]octane-6-carbonyl)-phényl]-méthanesulfonamide ;
N-[4-(1,3,3-triméthyl-6-aza-bicyclo[3.2.1]octane-6-carbonyl)-phényl]-méthanesulfonamide ;
N-éthyl-N-[4-(1,3,3-triméthyl-6-aza-bicyclo[3.2.1]octane-6-carbonyl)-phényl]-méthanesulfonamide ;
N-cyclopropylméthyl-N-[4-(1,3,3-triméthyl-6-aza-bicyclo[3.2.1]octane-6-carbonyl)-phényl]-méthanesulfonamide ;
N-[4-(1,3,3-triméthyl-6-aza-bicyclo[3.2.1]octane-6-carbonyl)-phényl]-isonicotinamide ;
2,4-dichloro-N-[4-(1,3,3-triméthyl-6-aza-bicyclo[3.2.1]octane-6-carbonyl)-phényl]-benzamide ;
2,4-diméthoxy-N-[4-(1,3,3-triméthyl-6-aza-bicyclo[3.2.1]octane-6-carbonyl)-phényl]-benzamide ;
4-trifluorométhoxy-N-[4-(1,3,3-triméthyl-6-aza-bicyclo[3.2.1]octane-6-carbonyl)-phényl]-benzamide ;
3,4-diméthoxy-N-[4-(1,3,3-triméthyl-6-aza-bicyclo[3.2.1]octane-6-carbonyl)-phényl]-benzamide ;
3,5-diméthyl-N-[4-(1,3,3-triméthyl-6-aza-bicyclo[3.2.1]octane-6-carbonyl)-phényl]-benzamide ; 3-trifluorométhoxy-N-[4-(1,3,3-triméthyl-6-aza-bicyclo[3.2.1]octane-6-carbonyl)-phényl]-benzamide ;
3,5-diméthoxy-N-[4-(1,3,3-triméthyl-6-aza-bicyclo[3.2.1]octane-6-carbonyl)-phényl]-benzamide ;
3-cyano-N-[4-(1,3,3-triméthyl-6-aza-bicyclo[3.2.1]octane-6-carbonyl)-phényl]-benzènesulfonamide ;
N-[4-(1,3,3-triméthyl-6-aza-bicyclo[3.2.1]octane-6-carbonyl)-phényl]-benzènesulfonamide ;
phényl-N-[4-(1,3,3-triméthyl-6-aza-bicyclo[3.2.1]octane-6-carbonyl)-phényl]-méthanesulfonamide ;
[4-(1,3,3-triméthyl-6-aza-bicyclo[3.2.1]octane-6-carbonyl)-phényl]-amide d'acide butane-1-sulfonique ;
3-trifluorométhyl-N-[4-(1,3,3-triméthyl-6-aza-bicyclo[3.2.1]octane-6-carbonyl)-phényl]-benzènesulfonamide ;
ester méthylique d'acide N-[4-(1,3,3-triméthyl-6-aza-bicyclo[3.2.1]octane-6-carbonyl)-phényl]-isophtalamique ;
acide N-[4-(1,3,3-triméthyl-6-aza-bicyclo[3.2.1]octane-6-carbonyl)-phényl]-isophtalamique ;
[4-(1,3,3-triméthyl-6-aza-bicyclo[3.2.1]octane-6-carbonyl)-phényl]-amide d'acide 4-méthoxy-cyclohexanecarboxylique ;
ester méthylique d'acide 6-[4-(1,3,3-triméthyl-6-aza-bicyclo[3.2.1]octane-6-carbonyl)-phénylcarbamoyl]-nicotinique ;
acide 6-[4-(1,3,3-triméthyl-6-aza-bicyclo[3.2.1]octane-6-carbonyl)-phénylcarbamoyl]-nicotinique ;
ester éthylique d'acide N-[4-(1,3,3-triméthyl-6-aza-bicyclo[3.2.1]octane-6-carbonyl)-phényl]-malonamique ;
ester tert-butylique d'acide N-[4-(1,3,3-triméthyl-6-aza-bicyclo[3.2.1]octane-6-carbonyl)-phényl]-malonamique ;
ester méthylique d'acide 3-[4-(1,3,3-triméthyl-6-aza-bicyclo[3.2.1]octane-6-carbonyl)-phénylcarbamoyl]-cyclohexane-carboxylique ;
acide 3-[4-(1,3,3-triméthyl-6-aza-bicyclo[3.2.1]octane-6-carbonyl)-phénylcarbamoyl]-cyclohexane-carboxylique ;
N-[4-(1,3,3-triméthyl-6-aza-bicyclo[3.2.1]octane-6-carbonyl)-phényl]-benzamide ;
N-[4-(1,3,3-triméthyl-6-aza-bicyclo[3.2.1]octane-6-carbonyl)-phényl]-isonicotinamide ;
N-[4-(1,3,3-triméthyl-6-aza-bicyclo[3.2.1]octane-6-carbonyl)-phényl]-nicotinamide ;
[4-(1,3,3-triméthyl-6-aza-bicyclo[3.2.1]octane-6-carbonyl)-phényl]-amide d'acide pyridine-2-carboxylique ;
[4-(1,3,3-triméthyl-6-aza-bicyclo[3.2.1]octane-6-carbonyl)-phényl]-amide d'acide 1-acétyl-pipéridine-4-carboxylique ;
ester méthylique d'acide N-[4-(1,3,3-triméthyl-6-aza-bicyclo[3.2.1]octane-6-carbonyl)-phényl]-succinamique ;
acide N-[4-(1,3,3-triméthyl-6-aza-bicyclo[3.2.1]octane-6-carbonyl)-phényl]-succinamique ;
acide N-[4-(1,3,3-triméthyl-6-aza-bicyclo[3.2.1]octane-6-carbonyl)-phényl]-malonamique ; 2-amino-N-[4-(1,3,3-triméthyl-6-aza-bicyclo[3.2.1]octane-6-carbonyl)-phényl]-acétamide ;
ester méthylique d'acide 2-acétylamino-N-[4-(1,3,3-triméthyl-6-aza-bicyclo[3.2.1]octane-6-carbonyl)-phényl]-succinamique ;
acide 2-acétylamino-N-[4-(1,3,3-triméthyl-6-aza-bicyclo[3.2.1]octane-6-carbonyl)-phényl]-succinamique ;
N-méthyl-N'-[4-(1,3,3-triméthyl-6-aza-bicyclo[3.2.1]octane-6-carbonyl)-phényl]-succinamide ;
N,N-diméthyl-N'-[4-(1,3,3-triméthyl-6-aza-bicyclo[3.2.1]octane-6-carbonyl)-phényl]-succinamide ;
N-[5-(1,3,3-triméthyl-6-aza-bicyclo[3.2.1]octane-6-carbonyl)-pyridin-2-yl]-benzamide ;
N-méthyl-N-[4-(1,3,3-triméthyl-6-aza-bicyclo[3.2.1]octane-6-carbonyl)-phényl]-benzamide ;
3,5,N-triméthyl-N-[4-(1,3,3-triméthyl-6-aza-bicyclo[3.2.1]octane-6-carbonyl)-phényl]-benzamide ;
2,4-diméthoxy-N-méthyl-N-[4-(1,3,3-triméthyl-6-aza-bicyclo[3.2.1]octane-6-carbonyl)-phényl]-benzamide ;
3,5-dichloro-N-méthyl-N-[4-(1,3,3-triméthyl-6-aza-bicyclo[3.2.1]octane-6-carbonyl)-phényl]-benzamide ;
4-bromo-N,N-diméthyl-N-[4-(1,3,3-triméthyl-6-aza-bicyclo[3.2.1]octane-6-carbonyl)-phényl]-benzamide ;
N-méthyl-N-[4-(1,3,3-triméthyl-6-aza-bicyclo[3.2.1]octane-6-carbonyl)-phényl]-isonicotinamide ;
N-méthyl-N-[4-(1,3,3-triméthyl-6-aza-bicyclo[3.2.1]octane-6-carbonyl)-phényl]-nicotinamide ;
2-pipéridin-1-yl-N-[4-(1,3,3-triméthyl-6-aza-bicyclo[3.2.1]octane-6-carbonyl)-phényl]-acétamide ; 2-morpholin-4-yl-N-[4-(1,3,3-triméthyl-6-aza-bicyclo[3.2.1]octane-6-carbonyl)-phényl]-acétamide;
2-(4-méthyl-pipérazin-1-yl)-N-[4-(1,3,3-triméthyl-6-aza-bicyclo[3.2.1]octane-6-carbonyl)-phényl]-acétamide ;
2-diméthylamino-N-[4-(1,3,3-triméthyl-6-aza-bicyclo[3.2.1]octane-6-carbonyl)-phényl]-acétamide ;
2-[(2-diméthylamino-éthyl)-méthyl-amino]-N-[4-(1,3,3-triméthyl-6-aza-bicyclo[3.2.1]octane-6-carbonyl)-phényl]-acétamide ;
2-(3,4-dihydro)-2H-quinolin-1-yl)-N-[4-(1,3,3-triméthyl-6-aza-bicyclo[3.2.1]octane-6-carbonyl)-phényl]-acétamide ;
2-(4-acétyl-pipéridin-1-yl)-N-[4-(1,3,3-triméthyl-6-aza-bicyclo[3.2.1]octane-6-carbonyl)-phényl]-acétamide ;
2-(4-diméthylamino-pipéridin-1-yl)-N-[4-(1,3,3-triméthyl-6-aza-bicyclo[3.2.1]octane-6-carbonyl)-phényl]-acétamide ;
2-(4-oxo-pipéridin-1-yl)-N-[4-(1,3,3-triméthyl-6-aza-bicyclo[3.2.1]octane-6-carbonyl)-phényl]-acétamide ;
2-[méthyl-(1-méthyl-pipéridin-4-yl)-amino]-N-[4-(1,3,3-triméthyl-6-aza-bicyclo[3.2.1]octane-6-carbonyl)-phényl]-acétamide ;
2-(3,6-dihydro-2H-pyridin-1-yl)-N-[4-(1,3,3-triméthyl-6-aza-bicyclo[3.2.1]octane-6-carbonyl)-phényl]-acétamide ;
ester éthylique d'acide 1-{[4-(1,3,3-triméthyl-6-aza-bicyclo[3.2.1]octane-6-carbonyl)-phénylcarbamoyl]-méthyl}-pipéridine-4-carboxylique ;
N-[4-(1,3,3-triméthyl-6-aza-bicyclo[3.2.1]octane-6-carbonyl)-phényl]-2-(1,3,3-triméthyl-6-aza-bicyclo[3.2.1]oct-6-yl)-acétamide ;
2-(4-acétyl-pipérazin-1-yl)-N-[4-(1,3,3-triméthyl-6-aza-bicyclo[3.2.1]octane-6-carbonyl)-phényl]-acétamide ;
2-(2,3,5,6-tétrahydro-[1,2']bipyrazinyl-4-yl)-N-[4-(1,3,3-triméthyl-6-aza-bicyclo[3.2.1]octane-6-carbonyl)-phényl]-acétamide ;
2-(cyclohexyl-méthyl-amino)-N-[4-(1,3,3-triméthyl-6-aza-bicyclo[3.2.1]octane-6-carbonyl)-phényl]-acétamide ;
2-(2,4-diméthoxy-benzyloxy)-N-[4-(1,3,3-triméthyl-6-aza-bicyclo[3.2.1]octane-6-carbonyl)-phényl]-acétamide ;
2-benzyloxy-N-[4-(1,3,3-triméthyl-6-aza-bicyclo[3.2.1]octane-6-carbonyl)-phényl]-acétamide
2-(3,4-dihydro-1H-isoquinol-2-yl)-N-[4-(1,3,3-triméthyl-6-aza-bicyclo[3.2.1]octane-6-carbonyl)-phényl]-acétamide ;
2-(6-aza-bicyclo[3.2.1]oct-6-yl)-N-[4-(1,3,3-triméthyl-6-aza-bicyclo[3.2.1]octane-6-carbonyl)-phényl]-acétamide ;
2-azépan-1-yl-N-[4-(1,3,3-triméthyl-6-aza-bicyclo[3.2.1]octane-6-carbonyl)-phényl]-acétamide ;
2-(6,7-diméthoxy-3,4-dihydro-1H-isoquinolin-2-yl)-N-[4-(1,3,3-triméthyl-6-aza-bicyclo[3.2.1]octane-6-carbonyl)-phényl]-acétamide ;
2-indol-1-yl-N-[4-(1,3,3-triméthyl-6-aza-bicyclo[3.2.1]octane-6-carbonyl)-phényl]-acétamide ;
2-benzoimidazol-1-yl-N-[4-(1,3,3-triméthyl-6-aza-bicyclo[3.2.1]octane-6-carbonyl)-phényl]-acétamide ; 2-(2,3-dihydro-indol-1-yl)-N-[4-(1,3,3-triméthyl-6-aza-bicyclo[3.2.1]octane-6-carbonyl)-phényl]-acétamide ;
2-benzotriazol-1-yl-N-[4-(1,3,3-triméthyl-6-aza-bicyclo[3.2.1]octane-6-carbonyl)-phényl]-acétamide ;
2-(4-benzoyl-pipéridin-1-yl)-N-[4-(1,3,3-triméthyl-6-aza-bicyclo[3.2.1]octane-6-carbonyl)-phényl]-acétamide ;
2-(4-tert-butyl-pipéridin-1-yl)-N-[4-(1,3,3-triméthyl-6-aza-bicyclo[3.2.1]octane-6-carbonyl)-phényl]-acétamide ;
2-(4-phényl-pipéridin-1-yl)-N-[4-(1,3,3-triméthyl-6-aza-bicyclo[3.2.1]octane-6-carbonyl)-phényl]-acétamide ;
2-(4-hydroxy-4-phényl-pipéridin-1-yl)-N-[4-(1,3,3-triméthyl-6-aza-bicyclo[3.2.1]octane-6-carbonyl)-phényl]-acétamide ;
2-(4-pyridin-2-yl-pipérazin-1-yl)-N-[4-(1,3,3-triméthyl-6-aza-bicyclo[3.2.1]octane-6-carbonyl)-phényl]-acétamide ;
2-(4-phényl-pipérazin-1-yl)-N-[4-(1,3,3-triméthyl-6-aza-bicyclo[3.2.1]octane-6-carbonyl)-phényl]-acétamide ;
2-(4-pyrimidin-2-yl-pipérazin-1-yl)-N-[4-(1,3,3-triméthyl-6-aza-bicyclo[3.2.1]octane-6-carbonyl)-phényl]-acétamide ;
2-(4-acétyl-4-phényl-pipéridin-1-yl)-N-[4-(1,3,3-triméthyl-6-aza-bicyclo[3.2.1]octane-6-carbonyl)-phényl]-acétamide ;
2-(4-cyano-4-phényl-pipéridin-1-yl)-N-[4-(1,3,3-triméthyl-6-aza-bicyclo[3.2.1]octane-6-carbonyl)-phényl]-acétamide ;
2-(4-pyridin-4-yl-pipérazin-1-yl)-N-[4-(1,3,3-triméthyl-6-aza-bicyclo[3.2.1]octane-6-carbonyl)-phényl]-acétamide ;
2-[4-(2-pyrrolidin-1-yl-acétyl)-pipérazin-1-yl]-N-[4-(1,3,3-triméthyl-6-aza-bicyclo[3.2.1]octane-6-carbonyl)-phényl]-acétamide ;
2-(pipéridin-4-yloxy)-N-[4-(1,3,3-triméthyl-6-aza-bicyclo[3.2.1]octane-6-carbonyl)-phényl]-acétamide ;
2-(2H-tétrazol-5-yl)-N-[4-(1,3,3-triméthyl-6-aza-bicyclo[3.2.1]octane-6-carbonyl)-phényl]-acétamide ;
3-pipéridin-1-yl-N-[4-(1,3,3-triméthyl-6-aza-bicyclo[3.2.1]octane-6-carbonyl)-phényl]-propionamide ;
3-benzylamino-N-[4-(1,3,3-triméthyl-6-aza-bicyclo[3.2.1]octane-6-carbonyl)-phényl]-propionamide ;
2-(3,4-dihydro-2H-quinolin-1-yl)-N-méthyl-N-[4-(1,3,3-triméthyl-6-aza-bicyclo[3.2.1]octane-6-carbonyl)-phényl]-acétamide ;
1-morpholin-4-yl-2-[4-(1,3,3-triméthyl-6-aza-bicyclo[3.2.1]octane-6-carbonyl)-phénylamino]-éthanone ;
[4-(2-pipéridin-1-yl-éthylamino)-phényl]-(1,3,3-triméthyl-6-aza-bicyclo[3.2.1]oct-6-yl)-méthanone
N-méthyl-N-phényl-2-[4-(1,3,3-triméthyl-6-aza-bicyclo[3.2.1]octane-6-carbonyl)-phénylamino]-acétamide ;
N-(2-méthoxy-éthyl)-N-[4-(1,3,3-triméthyl-6-aza-bicyclo[3.2.1]octane-6-carbonyl)-phényl]-benzamide ;
N-allyl-N-[4-(1,3,3-triméthyl-6-aza-bicyclo[3.2.1]octane-6-carbonyl)-phényl]-benzamide ;
[4-(2-méthoxy-éthylamino)-phényl]-(1,3,3-triméthyl-6-aza-bicyclo[3.2.1]oct-6-yl)-méthanone ;
(4-benzyloxy-phényl)-(1,3,3-triméthyl-6-aza-bicyclo[3.2.1]oct-6-yl)-méthanone ;
ester méthylique d'acide 3-[4-(1,3,3-triméthyl-6-aza-bicyclo[3.2.1]octane-6-carbonyl)-benzoylamino]-propionique ;
acide 3-[4-(1,3,3-triméthyl-6-aza-bicyclo[3.2.1]octane-6-carbonyl)-benzoylamino]-propionique ;
(4-benzyloxy-phényl)-(1,3,3-triméthyl-6-aza-bicyclo[3.2.1]oct-6-yl)-méthanone ;
ou un sel de celui-ci avec un acide ou une base pharmaceutiquement acceptable, ou tout isomère optique
ou mélange d'isomères optiques, y compris un mélange racémique, ou toute forme tautomère.

6. Composé selon l'une quelconque des revendications 1 à 5, pour le traitement, la prévention et/ou la prophylaxie de toute affection, tout trouble et toute maladie dans lequel une modulation ou une inhibition de l'activité de β11HSD1 est bénéfique.

7. Composé selon la revendication 6, dans lequel l'affection, le trouble ou la maladie est sélectionné parmi le syndrome métabolique, la résistance à l'insuline, la dyslipidémie, l'hypertension, l'obésité, le diabète de type 2, l'intolérance au glucose (IGT), l'hyperglycémie modérée à jeun (IFG), le diabète auto-immun latent de l'adulte (LADA), le diabète de type 1, les complications tardives diabétiques y compris les maladies cardiovasculaires, les troubles cardiovasculaires, les troubles du métabolisme lipidique, les troubles neurodégénératifs et psychiatriques, la dysrégulation de la pression intraoculaire y compris le glaucome, les troubles immunitaires, les réponses immunitaires inappropriées, les troubles musculo-squelettiques, les troubles gastro-intestinaux, le syndrome des ovaires polykystiques (PCOS), la croissance capillaire réduite
ou d'autres maladies, troubles ou affections qui sont influencés par les niveaux de glucocorticoïdes intracellulaires, les effets néfastes de niveaux sanguins accrus de glucocorticoïde endogène ou exogène, et toute combinaison de ceux-ci, les effets néfastes de niveaux plasmatiques accrus de glucocorticoïde actif endogène, la maladie de Cushing, le syndrome de Cushing, les effets néfastes d'un traitement avec agonistes de récepteurs de glucocorticoïdes de maladies inflammatoires, les effets néfastes d'un traitement avec agonistes de récepteurs de glucocorticoïdes de maladies avec un composant inflammatoire, les effets néfastes d'un traitement avec agonistes de récepteurs de glucocorticoïdes en tant que partie de chimiothérapie cancéreuse, les effets néfastes d'un traitement avec agonistes de récepteurs de glucocorticoïdes pour le trauma chirurgical/post-chirurgical ou autre, les effets néfastes d'un traitement avec agonistes de récepteurs de glucocorticoïdes dans le contexte de la transplantation d'organe ou de tissu ou les effets néfastes d'un traitement avec agonistes de récepteurs de glucocorticoïdes dans d'autres maladies, troubles
ou affections où les agonistes de récepteurs de glucocorticoïdes fournissent des effets bénéfiques du point de vue clinique.

8. Combinaison comprenant un composé tel que défini dans l'une quelconque des revendications 1 à 5 et une
ou plusieurs autres substances pharmaceutiquement actives.

9. Combinaison selon la revendication 8, dans laquelle lesdites autres substances actives sont sélectionnées parmi des agents anti-obésité, des agents anti-diabétiques, des agents modifiant le métabolisme lipidique, des agents antihypertenseurs, des agonistes de récepteurs de glucocorticoïdes, des agents pour le traitement et/ou la prévention de complications résultant de ou associées au diabète et des agents pour le traitement et/ou la prévention de complications et de troubles résultant de ou associés à l'obésité.

10. Composition pharmaceutique comprenant un composé tel que défini dans l'une quelconque des revendications 1 à 5, ou une combinaison telle que définie à la revendication 8 ou la revendication 9, avec un ou plusieurs supports ou diluants pharmaceutiquement acceptables.

11. Combinaison telle que définie à la revendication 8 ou 9 ou composition telle que définie à la revendication 10 destinée à être utilisée en tant que médicament.

12. Composé de la formule générale (I) : 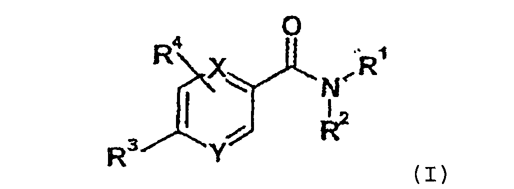 dans laquelle
R¹ et R² avec l'azote auquel ils sont liés sont 6-aza-bicyclo[3.2.1]octane substitué en option par au moins un de C₁-C₆-alkyle ;
R³ est C₁-C₆-alkyle, -NR⁸R⁹, -C(=O)NR⁸R⁹ ou -OR¹⁰, dans lequel le groupe alkyle est substitué en option par un ou plusieurs de R¹¹ ;
R⁴ est hydrogène, halogéno, hydroxy, cyano, trihalogénométhyle ou C₁-C₆-alkyle ;
R⁸ est hydrogène, C₁-C₆-alkyle, C₃-C₁₀-cycloalkyle, C₃-C₁₀-cycloalkyl-C₁-C₆-alkyle, C₁-C₆-alkyloxy-C₁-C₆-alkyle, aryl-C₁-C₆-alkyloxy-C₁-C₆-alkyle ou C₂-C₈-alkényle ;
R⁹ est C₁-C₆-alkyle, C₃-C₁₀-cycloalkyle, C₃-C₁₀-cycloalkyl-C₁-C₆-alkyle, C₃-C₁₀-cycloalkylcarbonyl-, C₃-C₁₀-hetcycloalkylcarbonyl-, arylcarbonyl-, hétarylcarbonyl-, C₁-C₆-alkyloxy-C₁-C₆-alkyle, NR¹²R¹³⁻ carbonyl-C₁-C₆-alkyl-, R¹⁴-C₁-C₆-alkylcarbonyl-, -COR¹⁵, C₁-C₆-alkyl-S(O)ₙ-, aryl-S(O)ₙ-, aryl-C₁-C₆-alkyl-S(O)ₙ-, aryl-C₁-C₆-alkyle ou hétaryl-C₁-C₆-alkyle, dans lequel les groupes alkyle, cycloalkyle, aryle et hétaryle sont indépendamment substitués en option par un ou plusieurs R¹¹ ;
R¹⁰ est C₁-C₆-alkyle, aryle-C₁-C₆-alkyle ou NR¹²R¹³-carbonyl-C₁-C₆-alkyle, dans lequel les groupes alkyle et aryle sont indépendamment substitués en option par un ou plusieurs R¹¹ ;
R¹¹ est R⁵, R⁶, halogéno, hydroxy, oxo, cyano, -COR¹⁵, C₁-C₈-alkyle, C₁-C₈-alkyloxy, C₃-C₁₀-cycloalkyle, trihalogénométhyle, trihalogénométhyloxy, aryle, aryl-C₁-C₆-alkyle, C₁-C₆-alkyloxy-C₁-C₆-alkyle, aryloxy-C₁-C₆-alkyle, aryl-C₁-C₆-alkyloxy-C₁-C₆-alkyle, hétaryle, hétaryl-C₁-C₆-alkyle, hétaryloxy-C₁-C₆-alkyle, hétaryl-C₁-C₆-alkyloxy-C₁-C₆-alkyle, -NR¹²R¹³, -SO₂NR¹²R¹³, NR¹²R¹³-carbonyl-C₁-C₆-alkyle, R¹⁶-carbonyl-N(R¹²)-, aryl-S(O)ₙ-, hétaryl-S(O)ₙ- ou R¹⁷S(O)ₙN(R¹²)- ; dans lequel les groupes aryle et hétaryle sont indépendamment substitués en option par un ou plusieurs R¹⁸ ;
R¹² et R¹³ sont indépendamment hydrogène, C₁-C₈-alkyle, C₃-C₁₀-cycloalkyle, C₃-C₁₀-cycloalkyl-C₁-C₆-alkyle, C₃-C₁₀-hetcycloalkyle, aryle, hétaryle, aryl-C₁-C₆-alkyle ou hétaryl-C₁-C₆-alkyle, dans lequel les groupes alkyle, aryle et hétaryle sont indépendamment substitués en option par un ou plusieurs de R¹⁸ ; ou R¹² et R¹³ avec l'azote auquel ils sont liés forment un système annulaire cyclique, bicyclique ou tricyclique, saturé ou partiellement saturé, contenant 4 à 12 atomes de carbone et 0 à 2 hétéroatomes supplémentaires sélectionnés parmi l'azote ou l'oxygène, le système annulaire étant substitué en option par au moins un R⁵, R⁵-oxy-, R⁶, halogéno, cyano, hydroxy, oxo, C₁-C₈-alkyle, aryle, hétaryle, aryl-C₁-C₆-alkyle, hétaryl-C₁-C₆-alkyle, C₁-C₆-alkyloxy-C₁-C₆-alkyle, NR¹²R¹³-carbonyl-C₁-C₆-alkyle, NR¹²R¹³-C₁-C₆-alkylcarbonyl-, R¹⁴-C₁-C₆-alkylcarbonyl- ou COR¹⁵ ;
R¹⁴ est C₁-C₆-alkyloxy, C₃-C₁₀-cycloalkyloxy-, C₃-C₁₀-cycloalkyl-C₁-C₆-alkyloxy-, C₃-C₁₀-hetcycloalkyloxy-, aryle, hétaryle, aryl-C₁-C₆-alkyloxy, hétaryl-C₁-C₆-alkyloxy, -NR¹²R¹³, -COR¹⁵, dans lequel les groupes alkyle, cycloalkyle, hetcycloalkyle, aryle et hétaryle sont indépendamment substitués en option par un ou plusieurs de R²⁰ ;
R¹⁵ est C₁-C₆-alkyle, hydroxy, C₁-C₈-alkyloxy, -NR¹²R¹³, aryle, aryloxy ou aryl-C₁-C₆-alkyloxy ;
R¹⁶ est R⁶, C₁-C₆-alkyle, C₂-C₆-alkényle, aryle, aryl-C₁-C₆-alkyle, hétaryle, hétaryl-C₁-C₆-alkyle, C₃-C₁₀-cycloalkyle, C₃-C₁₀-hetcycloalkyle, aryl-C₁-C₆-alkyloxy-C₁-C₆-alkyl-, hétaryl-C₁-C₆-alkyloxy-C₁-C₆-alkyl- ou R¹²R¹³-NC₁-C₆-alkyl-, dans lequel les groupes alkyle, alkényle, cycloalkyle, hetcycloalkyle, aryle et hétaryle sont substitués en option par R¹⁹ ; R¹⁷ est C₁-C₆-alkyle, C₃-C₁₀-cycloalkyle, C₃-C₁₀-hetcycloalkyle, aryle, aryl-C₁-C₆-alkyle, hétaryle, hétaryl-C₁-C₆-alkyle ;
R¹⁸ est R⁶, -NR¹²R¹³, oxo, C₁-C₆-alkyle, C₃-C₁₀-cycloalkyle ou C₃-C₁₀-hetcycloalkyle, dans lequel les groupes alkyle, cycloalkyle, hetcycloalkyle, aryle et hétaryle sont indépendamment substitués en option par un ou plusieurs de R⁷ ;
R¹⁹ est hydrogène, halogéno, hydroxy, oxo, nitro, cyano ou -COR¹⁵ ;
R²⁰ est hydrogène, C₁-C₈-alkyle, -NR¹²R¹³, C₁-C₆-alkyloxy ou aryl-C₁-C₆-alkyle ;
R⁵ est C₁-C₆-alkylcarbonyl-, C₃-C₁₀-cycloalkylcarbonyl-, C₃-C₁₀-cycloalkyl-C₁-C₆-alkylcarbonyl-, arylcarbonyl-, aryl-C₁-C₆-alkylcarbonyl-, hétarylcarbonyl- ou hétaryl-C₁-C₆-alkylcarbonyl- ;
R⁶ est C₁-C₆-alkyloxy-, aryloxy-, aryl-C₁-C₆-alkyloxy-, hétaryloxy- ou hétaryl-C₁-C₆-alkyloxy- ;
R⁷ est hydrogène, C₁-C₈-alkyle, C₁-C₆-alkyloxy ou aryl-C₁-C₆-alkyle ;
X et Y sont indépendamment -CH- ou azote ;
n est 1 ou 2 ; ou
un sel de celui-ci avec un acide ou une base pharmaceutiquement acceptable, ou tout isomère optique
ou mélange d'isomères optiques, y compris un mélange racémique, ou toute forme tautomère ;
à condition que le composé ne soit pas

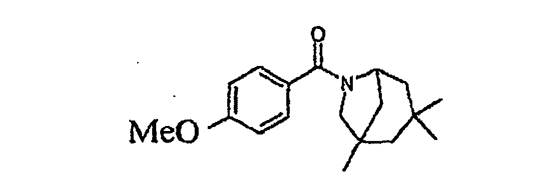

13. Composé selon la revendication 12, dans lequel X et Y sont -CH-.

14. Composé selon la revendication 12 ou 13, dans lequel R³ est -NR⁸R⁹, dans lequel R⁸ et R⁹ sont tels que définis à la revendication 1.

15. Composé selon l'une quelconque des revendications 12 à 14, dans lequel R⁴ est hydrogène ou halogéno.

16. Composé selon l'une quelconque des revendications 12 à 15 sélectionné dans le groupe constitué de : ester tert-butylique d'acide [4-(1,3,3-triméthyl-6-aza-bicyclo[3.2.1]octane-6-carbonyl)-phényl]carbamique ;
(4-amino-phényl)-(1,3,3-triméthyl-6-aza-bicyclo[3.2.1]oct-6-yl)-méthanone ;
(4-méthylamino-phényl)-(1,3,3-triméthyl-6-aza-bicyclo[3.2.1]oct-6-yl)-méthanone ;
N-méthyl-N-[4-(1,3,3-triméthyl-6-aza-bicyclo[3.2.1]octane-6-carbonyl)-phényl]-méthanesulfonamide ;
N-méthyl-N-[4-(1,3,3-triméthyl-6-aza-bicyclo[3.2.1]octane-6-carbonyl)-phényl]-benzènesulfonamide ;
N-méthyl-C-phényl-N-[4-(1,3,3-triméthyl-6-aza-bicyclo[3.2.1]octane-6-carbonyl)-phényl]-méthanesulfonamide ;
N-[4-(1,3,3-triméthyl-6-aza-bicyclo[3.2.1]octane-6-carbonyl)-phényl]-méthanesulfonamide;
N-éthyl-N-[4-(1,3,3-triméthyl-6-aza-bicyclo[3.2.1]octane-6-carbonyl)-phényl]-méthanesulfonamide ;
N-cyclopropylméthyl-N-[4-(1,3,3-triméthyl-6-aza-bicyclo[3.2.1]octane-6-carbonyl)-phényl]-méthanesulfonamide ;
N-[4-(1,3,3-triméthyl-6-aza-bicyclo[3.2.1]octane-6-carbonyl)-phényl]-isonicotinamide ;
2,4-dichloro-N-[4-(1,3,3-triméthyl-6-aza-bicyclo[3.2.1]octane-6-carbonyl)-phényl]-benzamide ;
2,4-diméthoxy-N-[4-(1,3,3-triméthyl-6-aza-bicyclo[3.2.1]octane-6-carbonyl)-phényl]-benzamide ;
4-trifluorométhoxy-N-[4-(1,3,3-triméthyl-6-aza-bicyclo[3.2.1]octane-6-carbonyl)-phényl]-benzamide ;
3,4-diméthoxy-N-[4-(1,3,3-triméthyl-6-aza-bicyclo[3.2.1]octane-6-carbonyl)-phényl]-benzamide ;
3,5-diméthyl-N-[4-(1,3,3-triméthyl-6-aza-bicyclo[3.2.1]octane-6-carbonyl)-phényl]-benzamide ; 3-trifluorométhoxy-N-[4-(1,3,3-triméthyl-6-aza-bicyclo[3.2.1]octane-6-carbonyl)-phényl]-benzamide;
3,5-diméthoxy-N-[4-(1,3,3-triméthyl-6-aza-bicyclo[3.2.1]octane-6-carbonyl)-phényl]-benzamide ;
3-cyano-N-[4-(1,3,3-triméthyl-6-aza-bicyclo[3.2.1]octane-6-carbonyl)-phényl]-benzènesulfonamide ;
N-[4-(1,3,3-triméthyl-6-aza-bicyclo[3.2.1]octane-6-carbonyl)-phényl]-benzènesulfonamide ;
phényl-N-[4-(1,3,3-triméthyl-6-aza-bicyclo[3.2.1]octane-6-carbonyl)-phényl]-méthanesulfonamide ;
[4-(1,3,3-triméthyl-6-aza-bicyclo[3.2.1]octane-6-carbonyl)-phényl]-amide d'acide butane-1-sulfonique ;
3-trifluorométhyl-N-[4-(1,3,3-triméthyl-6-aza-bicyclo[3.2.1]octane-6-carbonyl)-phényl]-benzènesulfonamide ;
ester méthylique d'acide N-[4-(1,3,3-triméthyl-6-aza-bicyclo[3.2.1]octane-6-carbonyl)-phényl]-isophtalamique ;
acide N-[4-(1,3,3-triméthyl-6-aza-bicyclo[3.2.1]octane-6-carbonyl)-phényl]-isophtalamique ;
[4-(1,3,3-triméthyl-6-aza-bicyclo[3.2.1]octane-6-carbonyl)-phényl]-amide d'acide 4-méthoxy-cyclo-hexanecarboxylique ;
ester méthylique d'acide 6-[4-(1,3,3-triméthyl-6-aza-bicyclo[3.2.1]octane-6-carbonyl)-phénylcarbamoyl]-nicotinique ;
acide 6-[4-(1,3,3-triméthyl-6-aza-bicyclo[3.2.1]octane-6-carbonyl)-phénylcarbamoyl]-nicotinique ;
ester éthylique d'acide N-[4-(1,3,3-triméthyl-6-aza-bicyclo[3.2.1]octane-6-carbonyl)-phényl]-malonamique ;
ester tert-butylique d'acide N-[4-(1,3,3-triméthyl-6-aza-bicyclo[3.2.1]octane-6-carbonyl)-phényl]-malonamique ;
ester méthylique d'acide 3-[4-(1,3,3-triméthyl-6-aza-bicyclo[3.2.1]octane-6-carbonyl)-phénylcarbamoyl]-cyclohexane-carboxylique ;
acide 3-[4-(1,3,3-triméthyl-6-aza-bicyclo[3.2.1]octane-6-carbonyl)-phénylcarbamoyl]-cyclohexane-carboxylique ;
N-[4-(1,3,3-triméthyl-6-aza-bicyclo[3.2.1]octane-6-carbonyl)-phényl]-benzamide ;
N-[4-(1,3,3-triméthyl-6-aza-bicyclo[3.2.1]octane-6-carbonyl)-phényl]-isonicotinamide ;
N-[4-(1,3,3-triméthyl-6-aza-bicyclo[3.2.1]octane-6-carbonyl)-phényl]-nicotinamide ;
[4-(1,3,3-triméthyl-6-aza-bicyclo[3.2.1]octane-6-carbonyl)-phényl]-amide d'acide pyridine-2-carboxylique ;
[4-(1,3,3-triméthyl-6-aza-bicyclo[3.2.1]octane-6-carbonyl)-phényl]-amide d'acide 1-acétyl-pipéridine-4-carboxylique ;
ester méthylique d'acide N-[4-(1,3,3-triméthyl-6-aza-bicyclo[3.2.1]octane-6-carbonyl)-phényl]-succinamique ;
acide N-[4-(1,3,3-triméthyl-6-aza-bicyclo[3.2.1]octane-6-carbonyl)-phényl]-succinamique ;
acide N-[4-(1,3,3-triméthyl-6-aza-bicyclo[3.2.1]octane-6-carbonyl)-phényl]-malonamique ; 2-amino-N-[4-(1,3,3-triméthyl-6-aza-bicyclo[3.2.1]octane-6-carbonyl)-phényl]-acétamide ;
ester méthylique d'acide 2-acétylamino-N-[4-(1,3,3-triméthyl-6-aza-bicyclo[3.2.1]octane-6-carbonyl)-phényl]-succinamique ;
acide 2-acétylamino-N-[4-(1,3,3-triméthyl-6-aza-bicyclo[3.2.1]octane-6-carbonyl)-phényl]-succinamique ;
N-méthyl-N'-[4-(1,3,3-triméthyl-6-aza-bicyclo[3.2.1]octane-6-carbonyl)-phényl]-succinamide ;
N,N-diméthyl-N'-[4-(1,3,3-triméthyl-6-aza-bicyclo[3.2.1]octane-6-carbonyl)-phényl]-succinamide ;
N-[5-(1,3,3-triméthyl-6-aza-bicyclo[3.2.1]octane-6-carbonyl)-pyridin-2-yl]-benzamide ;
N-méthyl-N-[4-(1,3,3-triméthyl-6-aza-bicyclo[3.2.1]octane-6-carbonyl)-phényl]-benzamide ;
3,5,N-triméthyl-N-[4-(1,3,3-triméthyl-6-aza-bicyclo[3.2.1]octane-6-carbonyl)-phényl]-benzamide ;
2,4-diméthoxy-N-méthyl-N-[4-(1,3,3-triméthyl-6-aza-bicyclo[3.2.1]octane-6-carbonyl)-phényl]-benzamide ;
3,5-dichloro-N-méthyl-N-[4-(1,3,3-triméthyl-6-aza-bicyclo[3.2.1]octane-6-carbonyl)-phényl]-benzamide ;
4-bromo-N,N-diméthyl-N-[4-(1,3,3-triméthyl-6-aza-bicyclo[3.2.1]octane-6-carbonyl)-phényl]-benzamide ;
N-méthyl-N-[4-(1,3,3-triméthyl-6-aza-bicyclo[3.2.1]octane-6-carbonyl)-phényl]-isonicotinamide ;
N-méthyl-N-[4-(1,3,3-triméthyl-6-aza-bicyclo[3.2.1]octane-6-carbonyl)-phényl]-nicotinamide ;
2-pipéridin-1-yl-N-[4-(1,3,3-triméthyl-6-aza-bicyclo[3.2.1]octane-6-carbonyl)-phényl]-acétamide ; 2-morpholin-4-yl-N-[4-(1,3,3-triméthyl-6-aza-bicyclo[3.2.1]octane-6-carbonyl)-phényl]-acétamide ;
2-(4-méthyl-pipérazin-1-yl)-N-[4-(1,3,3-triméthyl-6-aza-bicyclo[3.2.1]octane-6-carbonyl)-phényl]-acétamide ;
2-diméthylamino-N-[4-(1,3,3-triméthyl-6-aza-bicyclo[3.2.1]octane-6-carbonyl)-phényl]-acétamide ;
2-[(2-diméthylamino-éthyl)-méthyl-amino]-N-[4-(1,3,3-triméthyl-6-aza-bicyclo[3.2.1]octane-6-carbonyl)-phényl]-acétamide ;
2-(3,4-dihydro)-2H-quinolin-1-yl)-N-[4-(1,3,3-triméthyl-6-aza-bicyclo[3.2.1]octane-6-carbonyl)-phényl]-acétamide ;
2-(4-acétyl-pipéridin-1-yl)-N-[4-(1,3,3-triméthyl-6-aza-bicyclo[3.2.1]octane-6-carbonyl)-phényl]-acétamide ;
2-(4-diméthylamino-pipéridin-1-yl)-N-[4-(1,3,3-triméthyl-6-aza-bicyclo[3.2.1]octane-6-carbonyl)-phényl]-acétamide ;
2-(4-oxo-pipéridin-1-yl)-N-[4-(1,3,3-triméthyl-6-aza-bicyclo[3.2.1]octane-6-carbonyl)-phényl]-acétamide ;
2-[méthyl-(1-méthyl-pipéridin-4-yl)-amino]-N-[4-(1,3,3-triméthyl-6-aza-bicyclo[3.2.1]octane-6-carbonyl)-phényl]-acétamide ;
2-(3,6-dihydro-2H-pyridin-1-yl)-N-[4-(1,3,3-triméthyl-6-aza-bicyclo[3.2.1]octane-6-carbonyl)-phényl]-acétamide ;
ester éthylique d'acide 1-{[4-(1,3,3-triméthyl-6-aza-bicyclo[3.2.1]octane-6-carbonyl)-phénylcarbamoyl]-méthyl}-pipéridine-4-carboxylique ;
N-[4-(1,3,3-triméthyl-6-aza-bicyclo[3.2.1]octane-6-carbonyl)-phényl]-2-(1,3,3-triméthyl-6-aza-bicyclo[3.2.1]oct-6-yl)-acétamide ;
2-(4-acétyl-pipérazin-1-yl)-N-[4-(1,3,3-triméthyl-6-aza-bicyclo[3.2.1]octane-6-carbonyl)-phényl]-acétamide ;
2-(2,3,5,6-tétrahydro-[1,2']bipyrazinyl-4-yl)-N-[4-(1,3,3-triméthyl-6-aza-bicyclo[3.2.1]octane-6-carbonyl)-phényl]-acétamide ;
2-(cyclohexyl-méthyl-amino)-N-[4-(1,3,3-triméthyl-6-aza-bicyclo[3.2.1]octane-6-carbonyl)-phényl]-acétamide ;
2-(2,4-diméthoxy-benzyloxy)-N-[4-(1,3,3-triméthyl-6-aza-bicyclo[3.2.1]octane-6-carbonyl)-phényl]-acétamide ;
2-benzyloxy-N-[4-(1,3,3-triméthyl-6-aza-bicyclo[3.2.1]octane-6-carbonyl)-phényl]-acétamide ;
2-(3,4-dihydro-1H-isoquinol-2-yl)-N-[4-(1,3,3-triméthyl-6-aza-bicyclo[3.2.1]octane-6-carbonyl)-phényl]-acétamide ;
2-(6-aza-bicyclo[3.2.1]oct-6-yl)-N-[4-(1,3,3-triméthyl-6-aza-bicyclo[3.2.1]octane-6-carbonyl)-phényl]-acétamide ;
2-azépan-1-yl-N-[4-(1,3,3-triméthyl-6-aza-bicyclo[3.2.1]octane-6-carbonyl)-phényl]-acétamide ;
2-(6,7-diméthoxy-3,4-dihydro-1H-isoquinolin-2-yl)-N-[4-(1,3,3-triméthyl-6-aza-bicyclo[3.2.1]octane-6-carbonyl)-phényl]-acétamide ;
2-indol-1-yl-N-[4-(1,3,3-triméthyl-6-aza-bicyclo[3.2.1]octane-6-carbonyl)-phényl]-acétamide ;
2-benzoimidazol-1-yl-N-[4-(1,3,3-triméthyl-6-aza-bicyclo[3.2.1]octane-6-carbonyl)-phényl]-acétamide ; 2-(2,3-dihydro-indol-1-yl)-N-[4-(1,3,3-triméthyl-6-aza-bicyclo[3.2.1]octane-6-carbonyl)-phényl]-acétamide ;
2-benzotriazol-1-yl-N-[4-(1,3,3-triméthyl-6-aza-bicyclo[3.2.1]octane-6-carbonyl)-phényl]-acétamide ;
2-(4-benzoyl-pipéridin-1-yl)-N-[4-(1,3,3-triméthyl-6-aza-bicyclo[3.2.1]octane-6-carbonyl)-phényl]-acétamide ;
2-(4-tert-butyl-pipéridin-1-yl)-N-[4-(1,3,3-triméthyl-6-aza-bicyclo[3.2.1]octane-6-carbonyl)-phényl]-acétamide ;
2-(4-phényl-pipéridin-1-yl)-N-[4-(1,3,3-triméthyl-6-aza-bicyclo[3.2.1]octane-6-carbonyl)-phényl]-acétamide ;
2-(4-hydroxy-4-phényl-pipéridin-1-yl)-N-[4-(1,3,3-triméthyl-6-aza-bicyclo[3.2.1]octane-6-carbonyl)-phényl]-acétamide ;
2-(4-pyridin-2-yl-pipérazin-1-yl)-N-[4-(1,3,3-triméthyl-6-aza-bicyclo[3.2.1]octane-6-carbonyl)-phényl]-acétamide ;
2-(4-phényl-pipérazin-1-yl)-N-[4-(1,3,3-triméthyl-6-aza-bicyclo[3.2.1]octane-6-carbonyl)-phényl]-acétamide ;
2-(4-pyrimidin-2-yl-pipérazin-1-yl)-N-[4-(1,3,3-triméthyl-6-aza-bicyclo[3.2.1]octane-6-carbonyl)-phényl]-acétamide ;
2-(4-acétyl-4-phényl-pipéridin-1-yl)-N-[4-(1,3,3-triméthyl-6-aza-bicyclo[3.2.1]octane-6-carbonyl)-phényl]-acétamide ;
2-(4-cyano-4-phényl-pipéridin-1-yl)-N-[4-(1,3,3-triméthyl-6-aza-bicyclo[3.2.1]octane-6-carbonyl)-phényl]-acétamide ;
2-(4-pyridin-4-yl-pipérazin-1-yl)-N-[4-(1,3,3-triméthyl-6-aza-bicyclo[3.2.1]octane-6-carbonyl)-phényl]-acétamide ;
2-[4-(2-pyrrolidin-1-yl-acétyl)-pipérazin-1-yl]-N-[4-(1,3,3-triméthyl-6-aza-bicyclo[3.2.1]octane-6-carbonyl)-phényl]-acétamide ;
2-(pipéridin-4-yloxy)-N-[4-(1,3,3-triméthyl-6-aza-bicyclo[3.2.1]octane-6-carbonyl)-phényl]-acétamide ;
2-(2H-tétrazol-5-yl)-N-[4-(1,3,3-triméthyl-6-aza-bicyclo[3.2.1]octane-6-carbonyl)-phényl]-acétamide ;
3-pipéridin-1-yl-N-[4-(1,3,3-triméthyl-6-aza-bicyclo[3.2.1]octane-6-carbonyl)-phényl]-propionamide ;
3-benzylamino-N-[4-(1,3,3-triméthyl-6-aza-bicyclo[3.2.1]octane-6-carbonyl)-phényl]-propionamide ;
2-(3,4-dihydro-2H-quinolin-1-yl)-N-méthyl-N-[4-(1,3,3-triméthyl-6-aza-bicyclo[3.2.1]octane-6-carbonyl)-phényl]-acétamide ;
1-morpholin-4-yl-2-[4-(1,3,3-triméthyl-6-aza-bicyclo[3.2.1]octane-6-carbonyl)-phénylamino]-éthanone ;
[4-(2-pipéridin-1-yl-éthylamino)-phényl]-(1,3,3-triméthyl-6-aza-bicyclo[3.2.1]oct-6-yl)-méthanone ;
N-méthyl-N-phényl-2-[4-(1,3,3-triméthyl-6-aza-bicyclo[3.2.1]octane-6-carbonyl)-phénylamino]-acétamide ;
N-(2-méthoxy-éthyl)-N-[4-(1,3,3-triméthyl-6-aza-bicyclo[3.2.1]octane-6-carbonyl)-phényl]-benzamide ;
N-allyl-N-[4-(1,3,3-triméthyl-6-aza-bicyclo[3.2.1]octane-6-carbonyl)-phényl]-benzamide ;
[4-(2-méthoxy-éthylamino)-phényl]-(1,3,3-triméthyl-6-aza-bicyclo[3.2.1]oct-6-yl)-méthanone ;
(4-benzyloxy-phényl)-(1,3,3-triméthyl-6-aza-bicyclo[3.2.1]oct-6-yl)-méthanone ;
ester méthylique d'acide 3-[4-(1,3,3-triméthyl-6-aza-bicyclo[3.2.1]octane-6-carbonyl)-benzoylamino]-propionique ;
acide 3-[4-(1,3,3-triméthyl-6-aza-bicyclo[3.2.1]octane-6-carbonyl)-benzoylamino]-propionique ;
(4-benzyloxy-phényl)-(1,3,3-triméthyl-6-aza-bicyclo[3.2.1]oct-6-yl)-méthanone ;
ou un sel de celui-ci avec un acide ou une base pharmaceutiquement acceptable, ou tout isomère optique
ou mélange d'isomères optiques, y compris un mélange racémique, ou toute forme tautomère.

17. Procédé de préparation d'un composé tel que défini dans l'une quelconque des revendications 12 à 16, ledit procédé sélectionné parmi (A) à (F) ci-dessous : 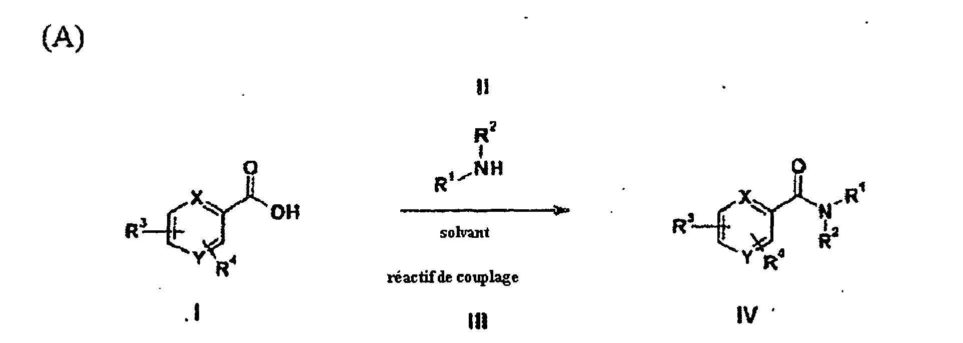 un acide (I) dans lequel R³, R⁴, X et Y sont définis à la revendication 12 est couplé à une amine (II) dans laquelle R¹ et R² sont définis à la revendication 12 dans des conditions de formation d'amide standard en utilisant un réactif de couplage (III) donnant un amide (IV) dans lequel R¹, R², R³, R⁴, X et Y sont définis comme ci-dessus ; ou 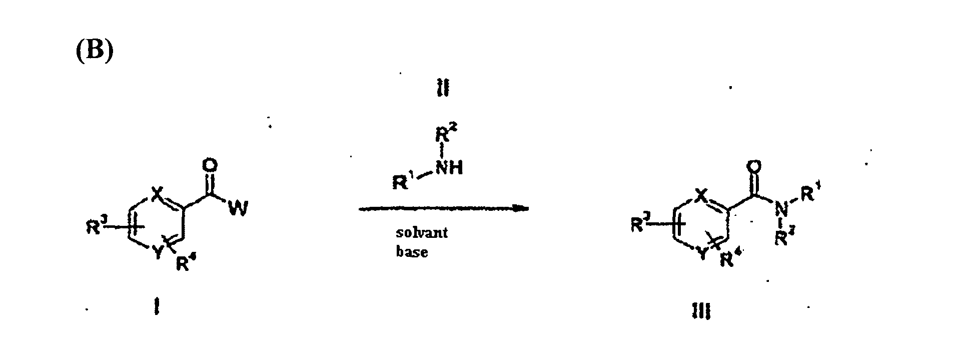 un dérivé d'acide (I) dans lequel W est halogéno, R²⁰(C=O)O-, C₁-C₆-alkyloxy ou aryl-C₁-C₆-alkyloxy, R²⁰ est C₁-C₆-alkyle ou aryl-C₁-C₆-alkyle et R³, R⁴, X et Y sont définis à la revendication 12 est mis à réagir avec une amine (II) dans laquelle R¹ et R² sont définis à la revendication 12 dans des conditions basiques dans un solvant donnant un amide (III) dans lequel R¹, R², R³, R⁴, X et Y sont définis comme ci-dessus ; ou 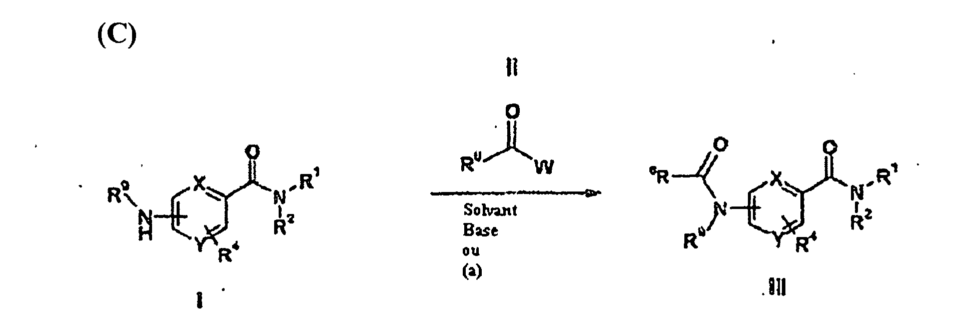
(i) un dérivé d'amide (I) dans lequel R¹, R², R⁴, R⁹, X et Y sont définis comme à la revendication 12 est mis à réagir avec un dérivé d'acide (II) dans lequel W est halogéno, R⁷(C=O)O-, C₁-C₆-alkyloxy ou aryl-C₁-C₆-alkyloxy, R⁷ est C₁-C₆-alkyle ou aryl-C₁-C₆-alkyle et R⁶ est C₃-C₁₀-cycloalkyle, C₃-C₁₀-hetcycloalkyle, aryle, hétaryle, R¹⁴C₁-C₆-alkylcarbonyl-, C₁-C₆-alkyl-S(O)ₙ-, aryl-S(O)ₙ- ou aryl-C₁-C₆-alkyl-S(O)ₙ-, dans lequel les groupes alkyle, cycloalkyle, hetcycloalkyle, aryle sont substitués en option par un ou plusieurs R¹¹ tels que définis à la revendication 12 dans des conditions basiques dans un solvant donnant un amide (III) ; dans lequel R¹, R², R⁴, R⁹, X et Y sont définis comme à la revendication 12 et R⁶ est C₃-C₁₀-cycloalkyle, C₃-C₁₀-hetcycloalkyle, aryle, hétaryle, R¹⁴C₁-C₆-alkylcarbonyl-, C₁-C₆-alkyl-S(O)ₙ-, aryl-S(O)ₙ- ou aryl-C₁-C₆-alkyl-S(O)ₙ-, dans lequel les groupes alkyle, cycloalkyle, hetcycloalkyle, aryle sont substitués en option par un ou plusieurs R¹¹ tels que définis ci-dessus ; ou
(ii) lorsque W est hydroxy, le dérivé d'acide (II) dans lequel R⁶ est tel que défini ci-dessus est couplé à une amine (I) dans laquelle R¹, R², R⁴, R⁹, X et Y sont définis comme ci-dessus dans des conditions de formation d'amide standard en utilisant un réactif de couplage (a) donnant un amide (III) dans lequel R¹, R², R⁴, R⁹, X et Y sont définis comme ci-dessus et R⁶ est C₃-C₁₀-cycloalkyle, C₃-C₁₀-hetcycloalkyle, aryle, hétaryle, R¹⁴C₁-C₆-alkylcarbonyl-, C₁-C₆-alkyl-S(O)ₙ-, aryl-S(O)ₙ- ou aryl-C₁-C₆-alkyl-S(O)ₙ-, dans lequel les groupes alkyle, cycloalkyle, hetcycloalkyle, aryle sont substitués en option par un ou plusieurs R¹¹ tels que définis ci-dessus ; ou 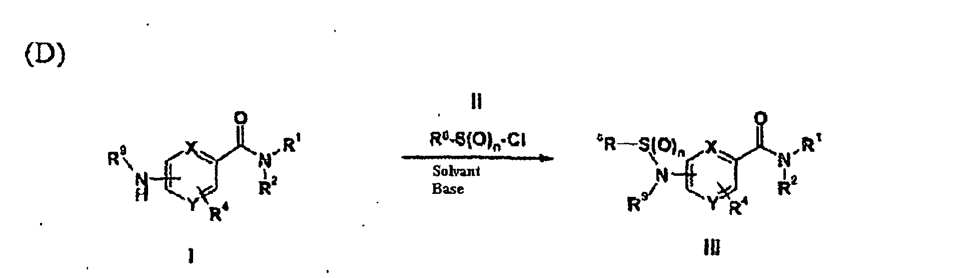 un dérivé d'amide (I) dans lequel R¹, R², R⁴, R⁹, X et Y sont définis comme à la revendication 12 est mis à réagir avec un dérivé de chlorure de sulfinyle (n = 1) ou sulfonyle (n = 2) (II) dans lequel R⁶ est C₁-C₆-alkyle, C₃-C₁₀-cycloalkyle, C₃-C₁₀-hetcycloalkyle, aryle, hétaryle ou aryl-C₁-C₆-alkyle, dans lequel les groupes alkyle, cycloalkyle, hetcycloalkyle, aryle et hétaryle sont substitués en option par un ou plusieurs R¹¹ tels que définis à la revendication 12 dans des conditions basiques dans un solvant pour donner un amide (III) ; dans lequel R¹, R², R⁴, R⁹, n, X et Y sont définis comme ci-dessus et R⁶ est C₁-C₆-alkyle, C₃-C₁₀-cycloalkyle, C₃-C₁₀-hetcycloalkyle, aryle, hétaryle
ou aryl-C₁-C₆-alkyle, dans lequel les groupes alkyle, cycloalkyle, hetcycloalkyle, aryle et hétaryle sont substitués en option par un ou plusieurs R¹¹ tels que définis ci-dessus ; ou 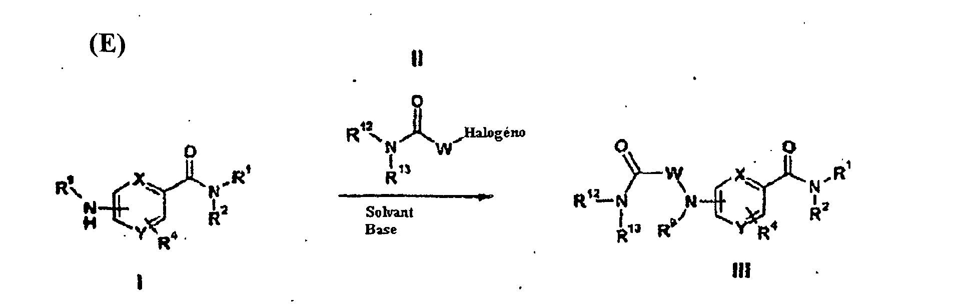 un dérivé d'amide (I) dans lequel R¹, R², R⁴, R⁹, X et Y sont définis comme à la revendication 12 est mis à réagir avec un dérivé d'halogénure amidoalkylique (II) dans lequel R¹² , R¹³ et halogéno sont définis à la revendication 12 et W est C₁-C₆-alkyle, dans lequel le groupe alkyle est substitué en option par un ou plusieurs R¹¹ tels que définis à la revendication 12 dans des conditions basiques dans un solvant pour donner un amide (III) ; dans lequel R¹, R², R⁴, R⁹, R¹², R¹³, X et Y sont définis comme ci-dessus et W est C₁-C₆-alkyle, dans lequel le groupe alkyle est substitué en option par un ou plusieurs R¹¹ tels que définis ci-dessus ; ou 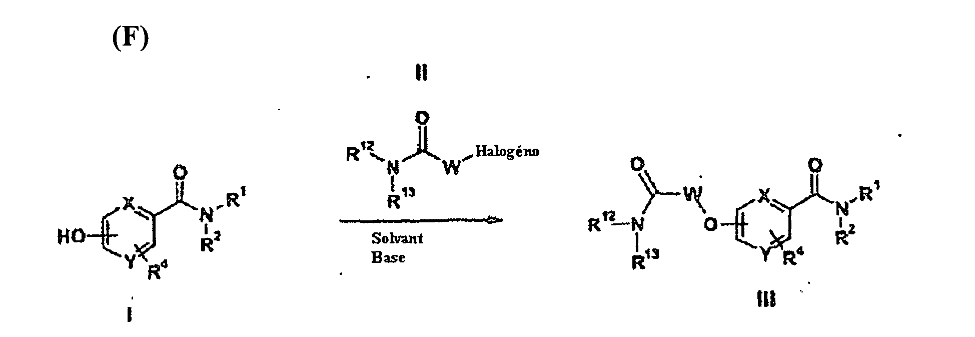 un dérivé d'amide (I) dans lequel R¹, R², R⁴, X et Y sont définis comme à la revendication 12 est mis à réagir avec un dérivé d'halogénure amidoalkylique (II) dans lequel R¹² et R¹³ sont définis à la revendication 12 et W est C₁-C₆-alkyle, dans lequel le groupe alkyle est substitué en option par un ou plusieurs R¹¹ tels que définis à la revendication 12 dans des conditions basiques dans un solvant pour donner un amide (III); dans lequel R¹, R², R⁴, R¹², R¹³, X et Y sont définis comme ci-dessus et W est C₁-C₆-alkyle, dans lequel le groupe alkyle est substitué en option par un ou plusieurs R¹¹ tels que définis ci-dessus ; et la conversion optionnelle du produit de l'un quelconque de (A) à (F) en un sel pharmaceutiquement acceptable.
